# EUROPEAN PATENT APPLICATION

(11) **EP 2 868 666 A1**
(43) Date of publication of application: **06.05.2015**
(21) Application number: 14191851.6
(22) Date of filing: 05.11.2014
(51) Int. Cl.: C07K 14/34, C12P 7/42, C12N 1/21, C12R 1/15

(54) **Microorganism with increased iron-regulated ABC transporter activity and method of producing hydroxycarboxylic acid by using the microorganism**

(30) Priority: 05.11.2013 KR 20130133829; 28.10.2014 KR 20140147637
(71) Applicant: Samsung Electronics Co., Ltd, Gyeonggi-do 443-742 (KR)
(72) Inventor: Cho, Hwayoung, Suwon-si Gyeonggi-do 443-803 (KR); Park, Jinhwan, Suwon-si Gyeonggi-do 443-803 (KR); Rhee, Hongsoon, Suwon-si Gyeonggi-do 443-803 (KR); Cho, Kwangmyung, Suwon-si Gyeonggi-do 443-803 (KR)
(74) Representative: Grünecker Patent- und Rechtsanwälte PartG mbB

(57) **Abstract**

A recombinant microorganism having increased iron-regulated ABC transporter activity and increased hydroxycarboxylic acid production, as well as a method of producing a hydroxycarboxylic acid using the recombinant microorganism, and a method of producing the recombinant microorganism.

<110>    Samsung Electronics Co., Ltd.

<120>    Microorganism with Increased Iron-Regulated ABC Transporter
         Activity and Method of Producing Hydroxycarboxylic Acid by
         using the Microorganism

<130>    EP97399FZTRpau

<140>    not yet assigned
<140>    herewith

<150>    10-2014-0147637
<151>    2014-10-28

<150>    10-2013-0133829
<151>    2013-11-05

<160>    90

<170>    KopatentIn 2.0

<210>    1
<211>    392
<212>    PRT
<213>    Corynebacterium glutamicum 13032

<400>    1
Met Thr Glu Val Ala Thr Val Lys Ala Ala Thr Pro His Asn Thr Lys
  1               5                  10                  15

Gly Asp Leu Phe Ser Ser Phe Asn Val Glu Asp Phe Glu Ile Pro Arg
             20                  25                  30

Gly Arg Asp Glu Glu Trp Arg Phe Ile Ser Leu Arg Arg Leu Arg Gly
         35                  40                  45

Leu His Asn Gly Glu Phe Ala Pro Ala Thr Thr Ser Asp Val Thr Val
     50                  55                  60

Glu Ile Pro Ala Asp Ala Glu Gly Ala Ser His Glu Val Val Ala Lys
 65                  70                  75                  80

Asp Asp Ala Arg Leu Gly Arg Ala Gly Ala Pro Val Asp Arg Val Ala
                 85                  90                  95

Ala Gln Ala Trp Thr Ser Lys Glu Gln Gly Asn Val Val Thr Phe Ala
            100                 105                 110

Lys Asn Ser His Asn Pro Thr Pro Val Thr Ile Thr Val Thr Gly Lys
        115                 120                 125

Gly Glu Gly Val Thr Ser Phe Gly Ala Ile Ser Ile Glu Val Glu Ala
    130                 135                 140

Gly Ala Asp Ala Ile Val Ala Leu Gln Tyr Val Gly Ser Gly Thr His
145                 150                 155                 160

Ala Asp Asn Val Glu Phe Ile Val Gly Asp Asn Ala Arg Leu Thr Val
                165                 170                 175

Ile Thr Asp Thr His Trp Asn Ala Asp Ala Val His Leu Ser Asn Gln
            180                 185                 190

Leu Ala Gln Leu Gly Arg Asp Ala Thr Leu Arg His Thr Val Ala Thr
        195                 200                 205

Phe Gly Gly Glu Val Val Arg Ile Val Pro Arg Val Arg Phe Thr Ala
    210                 215                 220

Pro Gly Gly Asp Ala Glu Met Leu Gly Val Tyr Phe Ala Asp Asp Gly
225                 230                 235                 240

Gln Tyr Phe Glu Gln Arg Leu Leu Val Asp His Ala Val Pro Asn Cys
                245                 250                 255

Arg Ser Asn Val Leu Tyr Lys Gly Ala Leu Gln Gly Asp Lys Asn Ser
            260                 265                 270

Asp Lys Pro Asp Ala Arg Thr Cys Trp Val Gly Asp Val Leu Ile Arg
        275                 280                 285

Ser Asn Ala His Gly Thr Asp Thr Tyr Glu Ala Asn Arg Ser Leu Val
    290                 295                 300

Leu Thr Glu Gly Ala Arg Ala Asp Ala Ile Pro Asn Leu Glu Ile Glu
305                 310                 315                 320

Thr Gly Gln Ile Val Gly Ala Gly His Ala Ala Thr Val Gly Arg Phe
                325                 330                 335

Asp Asp Glu His Val Phe Tyr Leu Gln Ala Arg Gly Ile Pro Ala Glu
            340                 345                 350

Glu Ala Arg Arg Leu Ile Val Arg Gly Phe Phe Asn Glu Val Ile Asn
        355                 360                 365

Lys Val Pro Val Glu Ser Ile Arg Gly Glu Leu Asp Asn Arg Val Ser
    370                 375                 380

Ser Glu Leu Ala Val Leu Gly Met
385                 390


<210>    2
<211>    1179
<212>    DNA
<213>    Corynebacterium glutamicum 13032

<400>    2
atgactgaag tagcaaccgt aaaggccgca accccgcaca acaccaaggg cgacctcttc      60

tcctcattta atgttgagga ctttgagatc ccacgcggac gcgacgaaga atggcgtttc     120

atttccctac gccgtcttcg cggactccac aacggcgagt tcgcacctgc aaccacctct     180

gacgtcaccg tcgagatccc tgcagatgca gaaggcgcaa gccacgaagt tgtcgcaaag     240

gacgatgctc gactgggtcg cgcaggcgca ccagtagacc gcgtcgcagc tcaggcctgg     300

acctccaagg aacagggcaa tgttgtcacc tttgccaaaa actcccacaa cccaacccca     360

gtaaccatca cggttactgg caagggtgaa ggcgttacct ctttcggcgc aatctccatc     420

gaggttgaag cgggcgcaga cgctatcgtc gcactgcagt acgtcggatc cggcacccac     480

gctgacaacg tcgaattcat cgttggcgac aacgcacgcc tgaccgtcat cacggacacc     540

cactggaacg ctgacgcagt tcacctgagc aaccagcttg cacagctggg acgcgacgca 600

actctacgcc acaccgtggc aaccttcggt ggagaagtag tccgcatcgt cccacgcgtg 660

cgtttcaccg caccaggtgg cgacgcagaa atgctcggcg tctacttcgc agatgatgga 720

cagtacttcg agcagcgcct gctggttgac cacgctgtac caaactgtcg ctccaacgtc 780

ttgtacaagg gcgcacttca gggtgacaag aactctgaca agccagatgc ccgtacctgc 840

tgggttggcg atgtgctcat ccgctcaaac gcccacggca ctgacaccta cgaagctaac 900

cgctcactcg tcctcaccga gggtgcacgc gcagacgcta ttccaaacct cgagattgaa 960

accggccaga tcgttggcgc aggacacgca gcaaccgtcg gtcgtttcga cgacgagcac 1020

gtgttctacc tccaggcccg tggtattcct gcagaggaag cacgccgcct catcgtccgc 1080

ggtttcttca acgaagtgat caacaaggtc ccagttgaat ccatccgcgg ggaattggac 1140

aaccgagtca gctcggaact cgcagttctt ggcatgtaa 1179

<210> 3
<211> 481
<212> PRT
<213> Corynebacterium glutamicum 13032

<400> 3
Met Thr Ser Ala Thr Thr Asn Pro Gly Val Asn Glu Pro Leu Thr Asp
1               5                   10                  15

Asp Gln Ile Ile Glu Ser Ile Gly Pro Tyr Asn Tyr Gly Trp His Asp
            20                  25                  30

Ser Asp Asp Ala Gly Ala Ser Ala Gln Arg Gly Leu Ser Glu Asp Val
        35                  40                  45

Val Arg Asp Ile Ser Ala Lys Lys Ser Glu Pro Glu Trp Met Leu Gln
    50                  55                  60

Gln Arg Leu Lys Ala Leu Ser Ile Phe Asp Lys Lys Pro Val Pro Thr
65                  70                  75                  80

Trp Gly Ala Asp Leu Ser Gly Ile Asp Phe Asp Asn Ile Lys Tyr Phe
                85                  90                  95

Val Arg Ser Thr Glu Lys Gln Ala Gln Ser Trp Glu Asp Leu Pro Glu
            100                 105                 110

Asp Ile Lys Asn Thr Tyr Asp Lys Leu Gly Ile Pro Glu Ala Glu Lys
        115                 120                 125

Gln Arg Leu Val Ala Gly Val Ala Ala Gln Tyr Glu Ser Glu Val Val
    130                 135                 140

Tyr His Gln Ile Arg Glu Asp Leu Glu Glu Lys Gly Val Ile Phe Leu
145                 150                 155                 160

Asp Thr Asp Thr Ala Leu Lys Glu His Pro Glu Ile Phe Gln Glu Tyr
                165                 170                 175

Phe Gly Thr Val Ile Pro Ala Gly Asp Asn Lys Phe Ser Ala Leu Asn
            180                 185                 190

Ser Ala Val Trp Ser Gly Gly Ser Phe Ile Tyr Val Pro Lys Gly Val
        195                 200                 205

His Val Asp Ile Pro Leu Gln Ala Tyr Phe Arg Ile Asn Thr Glu Asn
    210                 215                 220

Met Gly Gln Phe Glu Arg Thr Leu Ile Ile Val Asp Glu Asp Ala Tyr
225                 230                 235                 240

Val His Tyr Val Glu Gly Cys Thr Ala Pro Ile Tyr Lys Ser Asp Ser
                245                 250                 255

Leu His Ser Ala Val Val Glu Ile Ile Val Lys Lys Gly Gly Arg Cys
            260                 265                 270

Arg Tyr Thr Thr Ile Gln Asn Trp Ser Asn Asn Val Tyr Asn Leu Val
        275                 280                 285

Thr Lys Arg Thr Lys Val Glu Glu Gly Gly Thr Met Glu Trp Val Asp
    290                 295                 300

Gly Asn Ile Gly Ser Lys Val Thr Met Lys Tyr Pro Ala Val Trp Met
305                 310                 315                 320

Thr Gly Pro His Ala Lys Gly Glu Val Leu Ser Val Ala Phe Ala Gly
                325                 330                 335

Glu Gly Gln Phe Gln Asp Thr Gly Ala Lys Met Thr His Met Ala Pro
            340                 345                 350

Tyr Thr Ser Ser Asn Ile Val Ser Lys Ser Val Ala Arg Gly Gly Gly
        355                 360                 365

Arg Ala Ala Tyr Arg Gly Leu Val Gln Ile Asn Ala Asn Ala His His
    370                 375                 380

Ser Thr Ser Asn Val Glu Cys Asp Ala Leu Leu Val Asp Asp Ile Ser
385                 390                 395                 400

Arg Ser Asp Thr Tyr Pro Tyr Asn Asp Ile Arg Asn Asp His Val Ser
                405                 410                 415

Leu Gly His Glu Ala Thr Val Ser Gln Val Ser Glu Glu Gln Leu Phe
            420                 425                 430

Tyr Leu Met Ser Arg Gly Leu Ala Glu Glu Glu Ala Met Ala Met Ile
        435                 440                 445

Val Arg Gly Phe Val Glu Pro Ile Ala Lys Glu Leu Pro Met Glu Tyr
    450                 455                 460

Ala Leu Glu Leu Asn Arg Leu Ile Glu Leu Gln Met Glu Gly Ser Val
465                 470                 475                 480

Gly

<210>   4
<211>   1446

<212>     DNA
<213>     Corynebacterium glutamicum 13032

<400>     4
atgacttcgg caacgacgaa cccaggggtt aacgagccct tgaccgatga ccagatcatt          60

gaatccatcg gtccgtacaa ctatggttgg cacgactccg acgacgctgg tgcatccgca         120

cagcgtggtc tcagcgagga tgtcgtacgc gacatctctg cgaagaagag cgagccagaa         180

tggatgcttc agcagcgcct caaggccctg agcatttttg ataagaagcc agttccaacc          240

tggggtgcag acctttcagg cattgacttc gacaacatta aatacttcgt ccgctccact         300

gagaagcagg cacagtcctg ggaggatctc ccagaagaca tcaagaatac ctacgacaag         360

ctgggtattc ctgaggccga gaagcagcgc ctcgttgcag gtgttgcagc tcagtacgag         420

tctgaggttg tctaccacca gatccgcgag gacctggagg aaaagggagt tatcttcctt         480

gacaccgata ccgcactgaa agagcaccct gagatcttcc aggagtactt cggcaccgtc         540

attccagcag gcgacaacaa gttctccgca ctgaactccg ctgtctggtc cggtggatct         600

ttcatctacg tgccaaaggg tgtccacgtg gacattcctc tgcaggctta cttccgcatc         660

aacaccgaga acatgggtca gttcgaacgc accctgatca tcgttgatga ggatgcctac         720

gttcactacg ttgagggctg taccgcacct atttacaagt ccgactccct gcactccgca         780

gtcgttgaga tcatcgtgaa gaaggtggga cgctgccgct acaccaccat tcagaactgg         840

tccaacaacg tctacaacct ggtgaccaag cgcaccaagg ttgaagaggg cggcaccatg         900

gaatgggtcg atggcaacat cggctccaag gtcaccatga agtacccagc tgtctggatg         960

actggcccac acgcaaaggg cgaagttctc tccgtcgctt tcgcaggtga gggacagttc        1020

caggacaccg gcgccaagat gacccacatg gctccttaca cttcctccaa catcgtgtcc        1080

aagtctgtgg cacgtggcgg tggacgtgcg gcttaccgtg gtctggttca gatcaacgca        1140

aacgctcacc actcaacctc caacgttgag tgtgacgcac tgctggtcga tgacatctcc        1200

cgttctgaca cctacccata caacgacatc cgtaacgatc acgtgtcact cggccacgag        1260

gcaactgttt cccaggtttc tgaagagcag ctgttctacc tcatgagccg cggacttgcg        1320

gaagaagaag caatggcaat gatcgttcgt ggcttcgttg agccaatcgc taaggaactc        1380

ccaatggagt acgcccttga gctcaaccga ctgatcgaac tgcagatgga aggatcggtg        1440

ggctaa                                                                   1446


<210>     5
<211>     338
<212>     PRT
<213>     Corynebacterium glutamicum 13032

<400>     5
Met Val Lys Asn Arg Phe Lys Leu Val Ser Ile Ala Thr Val Ala Ala
1               5                   10                  15

Leu Ala Leu Val Gly Cys Ser Ser Thr Asp Ser Thr Ser Ser Glu Ser
                20                  25                  30

Ser Ser Ala Ala Glu Ser Thr Ala Ala Ala Ser Thr Leu Thr Ile Glu
            35                  40                  45

Asp Asn His Gly Thr Glu Gly Ile Ser Leu Pro Ile Glu Gly Val Ala
    50                  55                  60

Ala Thr Asp Asn Arg Ala Phe Glu Leu Leu Asp Arg Trp Gly Val Glu
65                  70                  75                  80

Leu Val Ala Ala Pro Leu Gln Leu Val Pro Phe Thr Val Thr Gly Tyr
                85                  90                  95

Thr Glu Glu Gly Gly Val Ala Asn Leu Gly Ser His Arg Glu Pro Asp
            100                 105                 110

Leu Glu Ala Leu Ala Ala Ala Gln Pro Ser Leu Ile Ile Asn Gly Gln
        115                 120                 125

Arg Phe Ala Gln Tyr Tyr Asp Asp Ile Ile Ala Leu Asn Pro Asp Ala
    130                 135                 140

Thr Val Val Glu Leu Asp Pro Arg Asp Gly Glu Pro Leu Asp Gln Glu
145                 150                 155                 160

Leu Ile Arg Gln Ala Glu Thr Leu Gly Glu Ile Phe Gly Glu Glu Glu
                165                 170                 175

Asp Ala Ala Lys Ile Val Ala Asp Phe Glu Ser Ala Leu Glu Arg Ala
            180                 185                 190

Lys Thr Ala Tyr Ala Ala Ile Ser Asp Gln Thr Val Met Ala Val Asn
        195                 200                 205

Val Ser Gly Gly Asn Ile Gly Tyr Ile Ala Pro Ser Val Gly Arg Thr
    210                 215                 220

Tyr Gly Pro Ile Phe Asp Leu Val Gly Leu Thr Pro Ala Leu Glu Val
225                 230                 235                 240

Gly Asn Ala Ser Ser Asp His Glu Gly Asp Asp Ile Asn Val Glu Ala
                245                 250                 255

Ile Ala Ala Ala Asn Pro Asp Leu Ile Leu Val Met Asp Arg Asp Gly
            260                 265                 270

Gly Thr Ser Thr Arg Asn Glu Ala Asp Tyr Val Pro Ala Glu Gln Ile
        275                 280                 285

Val Ser Asp Asn Glu Ala Leu Ala Asn Val Lys Ala Val Thr Asp Gly
    290                 295                 300

Tyr Val Tyr Tyr Ala Pro Ala Asp Thr Tyr Thr Asn Glu Asn Ile Ile
305                 310                 315                 320

Thr Tyr Thr Glu Ile Leu Asn Gly Met Ala Asp Met Phe Glu Lys Ala
                325                 330                 335

Ala Gln

<210>    6
<211>    1017
<212>    DNA
<213>    Corynebacterium glutamicum 13032

<400>    6
atggtgaaaa accgattcaa gctagtttca atcgcaactg ttgctgccct ggcgctcgtt      60

ggctgctctt ccaccgacag cacctcttcc gagtcttctt ccgctgcaga gtcaaccgct     120

gcagctagca ccctgactat cgaagacaac cacggcaccg aagggatctc cctgccaatc     180

gagggcgtcg ctgcgaccga caaccgcgca ttcgaactgc ttgatcgctg gggtgtagag     240

ctcgttgcag ctccacttca gctggttcca tttaccgtta cgggctacac cgaagagggc     300

ggcgtcgcta accttggctc ccaccgcgag ccagacctgg aagcacttgc tgctgcacag     360

ccttccctga tcatcaacgg ccagcgcttc gctcagtact acgatgacat cattgccctg     420

aaccctgacg caaccgttgt tgagctagac ccacgcgatg gcgagccact tgaccaggag     480

cttatccgcc aggctgaaac cctcggtgag atcttcggcg aagaagaaga tgctgcaaag     540

atcgttgctg atttcgagtc cgcacttgag cgcgctaaga ccgcatacgc agcaatctcc     600

gaccagaccg tcatggcagt taacgtttcc ggcggaaaca ttggctacat cgctccttcc     660

gttggacgca cctacggccc aatcttcgac ctggttggac tcaccccagc actcgaggtt     720

ggcaacgcgt cctccgacca cgagggcgac gacattaacg tcgaagcaat cgcagctgca     780

aacccagacc tgatcctggt catggaccgc gatggtggca ccagcacccg caacgaagct     840

gattacgttc cagcagagca gatcgtctcc gacaatgaag cactggcaaa cgtcaaggct     900

gtcaccgacg gatacgttta ctacgcacct gcagatacct acaccaacga aaacatcatc     960

acctacaccg agatcctcaa cggcatggca gatatgttcg agaaggcagc tcagtag       1017

<210>    7
<211>    538
<212>    PRT
<213>    Clostridium kluyveri

<400>    7
Met Ser Lys Gly Ile Lys Asn Ser Gln Leu Lys Lys Lys Asn Val Lys
1               5                   10                  15

Ala Ser Asn Val Ala Glu Lys Ile Glu Glu Lys Val Glu Lys Thr Asp
            20                  25                  30

Lys Val Val Glu Lys Ala Ala Glu Val Thr Glu Lys Arg Ile Arg Asn
        35                  40                  45

Leu Lys Leu Gln Glu Lys Val Val Thr Ala Asp Val Ala Ala Asp Met
    50                  55                  60

Ile Glu Asn Gly Met Ile Val Ala Ile Ser Gly Phe Thr Pro Ser Gly
65                  70                  75                  80

Tyr Pro Lys Glu Val Pro Lys Ala Leu Thr Lys Lys Val Asn Ala Leu
                85                  90                  95

Glu Glu Glu Phe Lys Val Thr Leu Tyr Thr Gly Ser Ser Thr Gly Ala
            100                 105                 110

Asp Ile Asp Gly Glu Trp Ala Lys Ala Gly Ile Ile Glu Arg Arg Ile
        115                 120                 125

Pro Tyr Gln Thr Asn Ser Asp Met Arg Lys Lys Ile Asn Asp Gly Ser
    130                 135                 140

Ile Lys Tyr Ala Asp Met His Leu Ser His Met Ala Gln Tyr Ile Asn
145                 150                 155                 160

Tyr Ser Val Ile Pro Lys Val Asp Ile Ala Ile Ile Glu Ala Val Ala
                165                 170                 175

Ile Thr Glu Glu Gly Asp Ile Ile Pro Ser Thr Gly Ile Gly Asn Thr
            180                 185                 190

Ala Thr Phe Val Glu Asn Ala Asp Lys Val Ile Val Glu Ile Asn Glu
        195                 200                 205

Ala Gln Pro Leu Glu Leu Glu Gly Met Ala Asp Ile Tyr Thr Leu Lys
    210                 215                 220

Asn Pro Pro Arg Arg Glu Pro Ile Pro Ile Val Asn Ala Gly Asn Arg
225                 230                 235                 240

Ile Gly Thr Thr Tyr Val Thr Cys Gly Ser Glu Lys Ile Cys Ala Ile
                245                 250                 255

Val Met Thr Asn Thr Gln Asp Lys Thr Arg Pro Leu Thr Glu Val Ser
            260                 265                 270

Pro Val Ser Gln Ala Ile Ser Asp Asn Leu Ile Gly Phe Leu Asn Lys
        275                 280                 285

Glu Val Glu Glu Gly Lys Leu Pro Lys Asn Leu Leu Pro Ile Gln Ser
    290                 295                 300

Gly Val Gly Ser Val Ala Asn Ala Val Leu Ala Gly Leu Cys Glu Ser
305                 310                 315                 320

Asn Phe Lys Asn Leu Ser Cys Tyr Thr Glu Val Ile Gln Asp Ser Met
                325                 330                 335

Leu Lys Leu Ile Lys Cys Gly Lys Ala Asp Val Val Ser Gly Thr Ser
            340                 345                 350

Ile Ser Pro Ser Pro Glu Met Leu Pro Glu Phe Ile Lys Asp Ile Asn
        355                 360                 365

Phe Phe Arg Glu Lys Ile Val Leu Arg Pro Gln Glu Ile Ser Asn Asn
    370                 375                 380

Pro Glu Ile Ala Arg Arg Ile Gly Val Ile Ser Ile Asn Thr Ala Leu
385                 390                 395                 400

Glu Val Asp Ile Tyr Gly Asn Val Asn Ser Thr His Val Met Gly Ser
                405                 410                 415

Lys Met Met Asn Gly Ile Gly Gly Ser Gly Asp Phe Ala Arg Asn Ala
            420                 425                 430

Tyr Leu Thr Ile Phe Thr Thr Glu Ser Ile Ala Lys Lys Gly Asp Ile
        435                 440                 445

Ser Ser Ile Val Pro Met Val Ser His Val Asp His Thr Glu His Asp
    450                 455                 460

Val Met Val Ile Val Thr Glu Gln Gly Val Ala Asp Leu Arg Gly Leu
465                 470                 475                 480

Ser Pro Arg Glu Lys Ala Val Ala Ile Ile Glu Asn Cys Val His Pro
                485                 490                 495

Asp Tyr Lys Asp Met Leu Met Glu Tyr Phe Glu Glu Ala Cys Lys Ser
            500                 505                 510

Ser Gly Gly Asn Thr Pro His Asn Leu Glu Lys Ala Leu Ser Trp His
        515                 520                 525

Thr Lys Phe Ile Lys Thr Gly Ser Met Lys
    530                 535

<210>    8
<211>    1617
<212>    DNA
<213>    Clostridium kluyveri

<400>    8
atgtctaaag gaatcaagaa tagccaattg aaaaaaaaga acgtcaaggc cagtaacgtt      60

gctgagaaga tcgaagagaa ggtggaaaag accgacaagg tcgttgagaa ggctgctgag     120

gtgaccgaaa agcgcattcg aaacttaaag ctccaggaaa aagttgtgac cgcagatgtc     180

gcagctgaca tgatcgagaa tggcatgatc gtcgcaatta gcggcttcac gccatccggg     240

tatccaaagg aggttccaaa agcccttact aagaaggtta atgcgctgga ggaggagttc     300

aaggtgacgc tgtataccgg ttctagcaca ggcgctgata ttgacggaga atgggcgaag     360

gcaggaataa tcgaacggcg tatcccatac cagaccaact ctgacatgag gaaaaaaata     420

aacgatggtt caatcaagta cgcagatatg cacctgagcc acatggctca atacattaac     480

tattctgtga ttcctaaggt tgacattgcc atcatcgagg cggtggccat taccgaggaa     540

ggggatatta ttcctagtac tggaatcggc aacacagcta cgtttgtcga gaatgcggat     600

aaggtaattg tggaaataaa cgaggctcag ccgcttgagt tggaaggcat ggcagatatc     660

tataccctga agaaccctcc acgtcgcgag cccatcccga tagtcaacgc aggcaaccgc     720

atagggacca cttacgtcac ctgtggctct gaaaaaatct gcgcgatcgt catgaccaac     780

acccaagaca aaacccgccc actcaccgaa gtttctcctg tcagtcaggc aatctccgat     840

aacctgattg gcttcctgaa caaagaagta gaggagggta aactcccaaa aaacctgctc     900

cccatacagt caggtgtcgg ttcggttgct aacgccgttc tagccggact ctgcgaatca     960

aacttcaaaa atttgagctg ctacacagaa gtgatccagg attcgatgtt gaagctcatc 1020

aaatgtggaa aggcagatgt ggtgtccggc acctcgatct cgccatcacc ggaaatgctg 1080

cccgagttca taaaggacat aaattttttt cgcgagaaga tagtactgcg cccccaggaa 1140

atatctaata atccggaaat agctcgtcgt ataggagtga tctccataaa cactgctttg 1200

gaagtagaca tctacggtaa tgtgaactcc acgcatgtca tgggctccaa gatgatgaac 1260

ggcatcggcg gcagcggcga ctttgcccgc aacgcatacc tcaccatatt cactacggag 1320

tccatcgcga agaagggcga catttcctct atcgttccta tggtttccca cgtggaccac 1380

accgagcatg acgtaatggt catcgttacc gaacaggggg ttgcggatct gcgcggtctt 1440

tcccctcggg aaaaggccgt ggcgataatt gagaattgcg tccacccgga ttacaaggat 1500

atgctcatgg agtacttcga ggaggcttgt aagtcctcag gtggcaacac cccacacaac 1560

cttgaaaaag ccctatcctg gcacactaag ttcataaaaa ctggctcgat gaagtaa 1617

<210> 9
<211> 502
<212> PRT
<213> Corynebacterium glutamicum

<400> 9
Met Ser Asp Arg Ile Ala Ser Glu Lys Leu Arg Ser Lys Leu Met Ser
1               5                   10                  15

Ala Asp Glu Ala Ala Gln Phe Val Asn His Gly Asp Lys Val Gly Phe
            20                  25                  30

Ser Gly Phe Thr Gly Ala Gly Tyr Pro Lys Ala Leu Pro Thr Ala Ile
        35                  40                  45

Ala Asn Arg Ala Lys Glu Ala His Gly Ala Gly Asn Asp Tyr Ala Ile
    50                  55                  60

Asp Leu Phe Thr Gly Ala Ser Thr Ala Pro Asp Cys Asp Gly Val Leu
65                  70                  75                  80

Ala Glu Ala Asp Ala Ile Arg Trp Arg Met Pro Tyr Ala Ser Asp Pro
                85                  90                  95

Ile Met Arg Asn Lys Ile Asn Ser Gly Ser Met Gly Tyr Ser Asp Ile
            100                 105                 110

His Leu Ser His Ser Gly Gln Gln Val Glu Glu Gly Phe Phe Gly Gln
        115                 120                 125

Leu Asn Val Ala Val Ile Glu Ile Thr Arg Ile Thr Glu Glu Gly Tyr
    130                 135                 140

Ile Ile Pro Ser Ser Ser Val Gly Asn Asn Val Glu Trp Leu Asn Ala
145                 150                 155                 160

Ala Glu Lys Val Ile Leu Glu Val Asn Ser Trp Gln Ser Glu Asp Leu
                165                 170                 175

Glu Gly Met His Asp Ile Trp Ser Val Pro Ala Leu Pro Asn Arg Ile
            180                 185                 190

Ala Val Pro Ile Asn Lys Pro Gly Asp Arg Ile Gly Lys Thr Tyr Ile
        195                 200                 205

Glu Phe Asp Thr Asp Lys Val Val Ala Val Val Glu Thr Asn Thr Ala
    210                 215                 220

Asp Arg Asn Ala Pro Phe Lys Pro Val Asp Asp Ile Ser Lys Lys Ile
225                 230                 235                 240

Ala Gly Asn Phe Leu Asp Phe Leu Glu Ser Glu Val Ala Ala Gly Arg
                245                 250                 255

Leu Ser Tyr Asp Gly Tyr Ile Met Gln Ser Gly Val Gly Asn Val Pro
            260                 265                 270

Asn Ala Val Met Ala Gly Leu Leu Glu Ser Lys Phe Glu Asn Ile Gln
        275                 280                 285

Ala Tyr Thr Glu Val Ile Gln Asp Gly Met Val Asp Leu Ile Asp Ala
    290                 295                 300

Gly Lys Met Thr Val Ala Ser Ala Thr Ser Phe Ser Leu Ser Pro Glu
305                 310                 315                 320

Tyr Ala Glu Lys Met Asn Asn Glu Ala Lys Arg Tyr Arg Glu Ser Ile
                325                 330                 335

Ile Leu Arg Pro Gln Gln Ile Ser Asn His Pro Glu Val Ile Arg Arg
            340                 345                 350

Val Gly Leu Ile Ala Thr Asn Gly Leu Ile Glu Ala Asp Ile Tyr Gly
        355                 360                 365

Asn Val Asn Ser Thr Asn Val Ser Gly Ser Arg Val Met Asn Gly Ile
    370                 375                 380

Gly Gly Ser Gly Asp Phe Thr Arg Asn Gly Tyr Ile Ser Ser Phe Ile
385                 390                 395                 400

Thr Pro Ser Glu Ala Lys Gly Gly Ala Ile Ser Ala Ile Val Pro Phe
                405                 410                 415

Ala Ser His Ile Asp His Thr Glu His Asp Val Met Val Val Ile Ser
            420                 425                 430

Glu Tyr Gly Tyr Ala Asp Leu Arg Gly Leu Ala Pro Arg Glu Arg Val
        435                 440                 445

Ala Lys Met Ile Gly Leu Ala His Pro Asp Tyr Arg Pro Leu Leu Glu
    450                 455                 460

Glu Tyr Tyr Ala Arg Ala Thr Ser Gly Asp Asn Lys Tyr Met Gln Thr
465                 470                 475                 480

Pro His Asp Leu Ala Thr Ala Phe Asp Phe His Ile Asn Leu Ala Lys
                485                 490                 495

Asn Gly Ser Met Lys Ala
            500

<210>	10
<211>	1509
<212>	DNA
<213>	Corynebacterium glutamicum

<400>	10
atgtctgatc gcattgcttc agaaaagctg cgctccaagc tcatgtccgc cgacgaggcg	60

gcacagtttg ttaaccacgg tgacaaggtt ggtttctccg gcttcaccgg cgctggctac	120

ccaaaggcac tgcctacggc aatcgctaac cgggctaaag aagcacacgg tgcaggcaac	180

gactacgcaa tcgacctgtt cactggcgca tcgaccgccc ctgactgcga tggcgtactt	240

gcagaagctg acgctatccg ctggcgcatg ccatacgcat ctgatccaat catgcgtaac	300

aagatcaact ccggctccat gggatactcc gatatccacc tgtcccactc cggccagcag	360

gttgaagagg gcttcttcgg ccagctcaac gtagctgtca ttgaaatcac ccgcatcact	420

gaagagggct acatcatccc ttcttcctcc gtgggtaaca acgttgagtg gctcaacgct	480

gcagagaagg tcatcctcga ggttaactct tggcagtctg aagacctcga aggtatgcac	540

gacatctggt ctgttcctgc cctgccaaac cgcattgccg tgccaatcaa caagccaggc	600

gaccgcatcg gtaagaccta catcgagttc gacaccgaca aggttgttgc tgttgttgag	660

accaacaccg cagaccgcaa cgcaccattc aagcctgtcg acgacatctc taagaagatc	720

gctggcaact tcctcgactt cctggaaagc gaagttgctg caggtcgcct gtcctacgac	780

ggctacatca tgcagtccgg cgtgggcaac gtgccaaacg cggtgatggc aggcctgctg	840

gaatccaagt ttgagaacat ccaggcctac accgaagtta tccaggacgg catggtggac	900

ctcatcgacg ccggcaagat gaccgttgca tccgcaactt ccttctccct gtctcctgag	960

tacgcagaga agatgaacaa cgaggctaag cgttaccgcg agtccattat cctgcgccca	1020

cagcagatct ctaaccaccc agaggtcatc cgccgcgttg gcctgatcgc caccaacggt	1080

ctcatcgagg ctgacattta cggcaacgtc aactccacca acgtttctgg ctcccgcgtc	1140

atgaacggca tcggcggctc cggcgacttc acccgtaacg gctacatctc cagcttcatc	1200

accccttcag aggcaaaggg cggcgcaatc tctgcgatcg ttcctttcgc atcccacatc	1260

gaccacaccg agcacgatgt catggttgtt atctctgagt acggttacgc agaccttcgt	1320

ggtctggctc cacgtgagcg cgttgccaag atgatcggcc tggctcaccc tgattaccgc	1380

ccactgctcg aggagtacta cgctcgcgca acctccggtg acaacaagta catgcagacc	1440

cctcatgatc ttgcaaccgc gtttgatttc cacatcaacc tggctaagaa cggctccatg	1500

aaggcataa	1509

<210>	11
<211>	125
<212>	PRT

<213>    Corynebacterium glutamicum

<400>    11
Met Asn Gly Ile Gly Gly Ser Gly Asp Phe Thr Arg Asn Ala Phe Ala
1               5                   10                  15

Ser Thr Phe Ile Ser Pro Ser Ala Ala Lys Val Asp Ala Ile Ser Ala
            20                  25                  30

Ile Val Pro Phe Ala Ser His Ile Asp His Thr Glu His Asp Ala Met
        35                  40                  45

Val Val Ile Thr Glu Tyr Gly Tyr Ala Asp Leu Arg Gly Leu Ser Pro
    50                  55                  60

Lys Gln Arg Val Pro Lys Met Ile Ala Ile Ala His Pro Asp Tyr Arg
65                  70                  75                  80

Pro Leu Leu Glu Ala Tyr Phe Asp Arg Ala Leu Asn Ser Ala Asp Ser
                85                  90                  95

Tyr Gln His Thr Leu His Asp Leu Arg Thr Ala Phe Asp Phe His Asn
            100                 105                 110

Arg Leu Asn Ser Gln Gly Thr Met Lys Ile Glu Lys Ala
        115                 120                 125


<210>    12
<211>    378
<212>    DNA
<213>    Corynebacterium glutamicum

<400>    12
atgaatggta tcggcggctc gggcgatttc acgcgtaacg cctttgcttc cacatttatc      60

tctccctcgg cagccaaagt tgatgcgatt tccgcgattg tgcctttcgc gtcccatatc     120

gatcacacgg aacatgatgc gatggttgtc attactgaat atggctacgc agacctgcgc     180

gggctatcgc caaaacaacg agtccccaaa atgattgcca tcgcccaccc ggactatcga     240

ccactgctgg aagcatactt tgaccgggcg ctgaacagtg ctgattccta tcagcacacc     300

ctgcatgatc tgcgcaccgc cttcgatttc cataatcgct tgaactcaca aggaaccatg     360

aaaatcgaaa aagcatag                                                   378


<210>    13
<211>    451
<212>    PRT
<213>    Porphyromonas gingivalis

<400>    13
Met Glu Ile Lys Glu Met Val Ser Leu Ala Arg Lys Ala Gln Lys Glu
1               5                   10                  15

Tyr Gln Ala Thr His Asn Gln Glu Ala Val Asp Asn Ile Cys Arg Ala
            20                  25                  30

Ala Ala Lys Val Ile Tyr Glu Asn Ala Ala Ile Leu Ala Arg Glu Ala
        35                  40                  45

Val Asp Glu Thr Gly Met Gly Val Tyr Glu His Lys Val Ala Lys Asn
    50                  55                  60
Gln Gly Lys Ser Lys Gly Val Trp Tyr Asn Leu His Asn Lys Lys Ser
65                  70                  75                  80
Ile Gly Ile Leu Asn Ile Asp Glu Arg Thr Gly Met Ile Glu Ile Ala
                85                  90                  95
Lys Pro Ile Gly Val Val Gly Ala Val Thr Pro Thr Thr Asn Pro Ile
            100                 105                 110
Val Thr Pro Met Ser Asn Ile Ile Phe Ala Leu Lys Thr Cys Asn Ala
        115                 120                 125
Ile Ile Ile Ala Pro His Pro Arg Ser Lys Lys Cys Ser Ala His Ala
    130                 135                 140
Val Arg Leu Ile Lys Glu Ala Ile Ala Pro Phe Asn Val Pro Glu Gly
145                 150                 155                 160
Met Val Gln Ile Ile Glu Glu Pro Ser Ile Glu Lys Thr Gln Glu Leu
                165                 170                 175
Met Gly Ala Val Asp Val Val Val Ala Thr Gly Gly Met Gly Met Val
            180                 185                 190
Lys Ser Ala Tyr Ser Ser Gly Lys Pro Ser Phe Gly Val Gly Ala Gly
        195                 200                 205
Asn Val Gln Val Ile Val Asp Ser Asn Ile Asp Phe Glu Ala Ala Ala
    210                 215                 220
Glu Lys Ile Ile Thr Gly Arg Ala Phe Asp Asn Gly Ile Ile Cys Ser
225                 230                 235                 240
Gly Glu Gln Ser Ile Ile Tyr Asn Glu Ala Asp Lys Glu Ala Val Phe
                245                 250                 255
Thr Ala Phe Arg Asn His Gly Ala Tyr Phe Cys Asp Glu Ala Glu Gly
            260                 265                 270
Asp Arg Ala Arg Ala Ala Ile Phe Glu Asn Gly Ala Ile Ala Lys Asp
        275                 280                 285
Val Val Gly Gln Ser Val Ala Phe Ile Ala Lys Lys Ala Asn Ile Asn
    290                 295                 300
Ile Pro Glu Gly Thr Arg Ile Leu Val Val Glu Ala Arg Gly Val Gly
305                 310                 315                 320
Ala Glu Asp Val Ile Cys Lys Glu Lys Met Cys Pro Val Met Cys Ala
                325                 330                 335
Leu Ser Tyr Lys His Phe Glu Glu Gly Val Glu Ile Ala Arg Thr Asn
            340                 345                 350
Leu Ala Asn Glu Gly Asn Gly His Thr Cys Ala Ile His Ser Asn Asn
        355                 360                 365
Gln Ala His Ile Ile Leu Ala Gly Ser Glu Leu Thr Val Ser Arg Ile
    370                 375                 380

Val Val Asn Ala Pro Ser Ala Thr Thr Ala Gly Gly His Ile Gln Asn
385                 390                 395                 400

Gly Leu Ala Val Thr Asn Thr Leu Gly Cys Gly Ser Trp Gly Asn Asn
                405                 410                 415

Ser Ile Ser Glu Asn Phe Thr Tyr Lys His Leu Leu Asn Ile Ser Arg
            420                 425                 430

Ile Ala Pro Leu Asn Ser Ser Ile His Ile Pro Asp Asp Lys Glu Ile
        435                 440                 445

Trp Glu Leu
    450

<210>    14
<211>    1356
<212>    DNA
<213>    Porphyromonas gingivalis

<400>    14
atggagatta aagagatggt cagtcttgcg cgcaaagctc agaaggagta tcaggccacc      60

cataaccaag aagctgtgga caacatctgc cgagcagcag cgaaggttat ttacgaaaat     120

gcagcaattc tggcacgcga ggcagtggac gaaaccggca tgggtgttta cgagcacaag     180

gtggccaaga atcaaggcaa gtccaaaggt gtttggtaca acctgcataa caagaagtcg     240

attggcatcc tcaatatcga cgagcgtacc ggcatgatcg agatcgcaaa acctatcggg     300

gttgtaggcg ccgttacgcc aaccaccaac cctatcgtta ctccgatgag caacatcatc     360

tttgctctta agacctgcaa cgccatcatt atcgccccac acccgcgctc caaaaagtgc     420

tctgcccacg cagttcggct gatcaaagag gctatcgctc cgttcaacgt gcccgaaggt     480

atggttcaga tcatcgagga gcctagcatc gagaagacgc aggaattgat gggcgccgta     540

gacgtggtcg ttgctaccgg gggcatgggc atggtcaagt ctgcctactc ctcagggaag     600

ccttctttcg gtgtcggagc cggcaatgtt caggtgatag tggacagcaa catcgacttc     660

gaagcggcag cagaaaagat catcaccgga cgtgccttcg acaacggtat catctgctca     720

ggcgaacagt ccatcatcta caacgaggct gacaaggaag cagttttcac agcattccgc     780

aaccacggtg cgtacttttg cgacgaggcc gagggagatc gggctcgtgc agcgatcttc     840

gaaaatggag ccatcgcgaa agatgttgtg ggccagtccg ttgcctttat tgcaaagaag     900

gcgaacatta atatccccga gggtactcgt attctcgtgg tcgaagctcg cggagtaggc     960

gccgaagatg tcatctgtaa agaaaagatg tgtccagtca tgtgcgccct ctcctacaag    1020

cacttcgaag agggggtaga gatcgcaagg acgaacctcg caaacgaagg caatggccat    1080

acctgtgcta tccactccaa caaccaagca cacatcatct tggcaggctc ggagctgacc    1140

gtgtctcgca tcgtggtcaa cgcgccaagt gctaccacag caggcggtca catccagaac    1200

ggtcttgccg tcaccaatac tctaggctgc ggctcttggg gtaacaactc gatctccgaa 1260

aacttcactt ataaacacct gctcaacatt tcacgcatcg ccccgttgaa ctccagcatt 1320

catatcccag atgataagga aatctgggaa ctctaa 1356

<210> 15
<211> 371
<212> PRT
<213> Porphyromonas gingivalis

<400> 15
Met Gln Leu Phe Lys Leu Lys Ser Val Thr His His Phe Asp Thr Phe
1               5                   10                  15

Ala Glu Phe Ala Lys Glu Phe Cys Leu Gly Glu Arg Asp Leu Val Ile
            20                  25                  30

Thr Asn Glu Phe Ile Tyr Glu Pro Tyr Met Lys Ala Cys Gln Leu Pro
        35                  40                  45

Cys His Phe Val Met Gln Glu Lys Tyr Gly Gln Gly Glu Pro Ser Asp
    50                  55                  60

Glu Met Met Asn Asn Ile Leu Ala Asp Ile Arg Asn Ile Gln Phe Asp
65                  70                  75                  80

Arg Val Ile Gly Ile Gly Gly Gly Thr Val Ile Asp Ile Ser Lys Leu
                85                  90                  95

Phe Val Leu Lys Gly Leu Asn Asp Val Leu Asp Ala Phe Asp Arg Lys
            100                 105                 110

Ile Pro Leu Ile Lys Glu Lys Glu Leu Ile Ile Val Pro Thr Thr Cys
        115                 120                 125

Gly Thr Gly Ser Glu Val Thr Asn Ile Ser Ile Ala Glu Ile Lys Ser
    130                 135                 140

Arg His Thr Lys Met Gly Leu Ala Asp Asp Ala Ile Val Ala Asp His
145                 150                 155                 160

Ala Ile Ile Ile Pro Glu Leu Leu Lys Ser Leu Pro Phe His Phe Tyr
                165                 170                 175

Ala Cys Ser Ala Ile Asp Ala Leu Ile His Ala Ile Glu Ser Tyr Val
            180                 185                 190

Ser Pro Lys Ala Ser Pro Tyr Ser Arg Leu Phe Ser Glu Ala Ala Trp
        195                 200                 205

Asp Ile Ile Leu Glu Val Phe Lys Lys Ile Ala Glu His Gly Pro Glu
    210                 215                 220

Tyr Arg Phe Glu Lys Leu Gly Glu Met Ile Met Ala Ser Asn Tyr Ala
225                 230                 235                 240

Gly Ile Ala Phe Gly Asn Ala Gly Val Gly Ala Val His Ala Leu Ser
                245                 250                 255

Tyr Pro Leu Gly Gly Asn Tyr His Val Pro His Gly Glu Ala Asn Tyr
            260                 265                 270

Gln Phe Phe Thr Glu Val Phe Lys Val Tyr Gln Lys Lys Asn Pro Phe
        275                 280                 285

Gly Tyr Ile Val Glu Leu Asn Trp Lys Leu Ser Lys Ile Leu Asn Cys
    290                 295                 300

Gln Pro Glu Tyr Val Tyr Pro Lys Leu Asp Glu Leu Leu Gly Cys Leu
305                 310                 315                 320

Leu Thr Lys Lys Pro Leu His Glu Tyr Gly Met Lys Asp Glu Glu Val
                325                 330                 335

Arg Gly Phe Ala Glu Ser Val Leu Lys Thr Gln Gln Arg Leu Leu Ala
            340                 345                 350

Asn Asn Tyr Val Glu Leu Thr Val Asp Glu Ile Glu Gly Ile Tyr Arg
        355                 360                 365

Arg Leu Tyr
    370

<210>    16
<211>    1116
<212>    DNA
<213>    Porphyromonas gingivalis

<400>    16
atgcagcttt tcaagctcaa gagcgtcaca catcactttg atacttttgc agagtttgcc      60

aaggagttct gtctcggtga acgcgacttg gtaattacca acgagttcat ctacgaaccg     120

tatatgaagg catgccagct gccttgtcat tttgtgatgc aggagaaata cggccaaggc     180

gagccttctg acgagatgat gaacaacatc ctagcagata tccgtaatat ccagttcgac     240

cgcgtgatcg ggatcggagg tggtacggtt attgacatct caaaactctt tgttctgaag     300

ggattaaatg atgttctcga cgcgttcgat cgcaagattc cccttatcaa agagaaagaa     360

ctgatcattg tgcccaccac ctgcggaacc ggctcggagg tgacgaacat ttccatcgcc     420

gagatcaagt cccggcacac caagatgggt ttggctgacg atgcaattgt tgctgaccac     480

gccataatca tccctgaact tctgaagagc ttgcccttcc acttctatgc atgctccgca     540

atcgacgctc ttattcatgc catcgagtca tacgtttctc caaaagcgtc tccatactcc     600

cgtctgttca gtgaggcggc gtgggacatt atcctggaag ttttcaagaa aatcgccgaa     660

cacggcccag agtaccgctt cgagaagctg ggggaaatga tcatggccag caactatgcc     720

ggtatcgctt tcggcaacgc aggcgttggc gccgtccacg ctctatccta cccgttgggc     780

ggcaactatc acgtgccgca tggagaagca aactatcagt tcttcaccga ggtctttaaa     840

gtataccaaa agaagaatcc gttcggctat attgtcgaac tcaactggaa gctctccaag     900

attctgaact gccagccaga gtacgtgtac ccgaagctgg atgaactgct cggttgcctt     960

cttaccaaga aacctttgca cgaatacggc atgaaggacg aagaggttcg tggcttcgcg    1020

gaatcggtcc tgaagaccca gcaacgcttg ctcgccaaca actacgtcga acttactgtc     1080

gatgagatcg aaggtatcta ccgacgtctc tactag                              1116

<210>    17
<211>    543
<212>    PRT
<213>    Corynebacterium glutamicum

<400>    17
Met Ser Ser Thr Pro Ala Gln Asp Leu Ala Arg Ala Val Ile Asp Ser
1               5                   10                  15

Leu Ala Pro His Val Thr Asp Val Val Leu Cys Pro Gly Ser Arg Asn
            20                  25                  30

Ser Pro Leu Ser Leu Glu Leu Leu Ala Arg Gln Asp Leu Arg Val His
        35                  40                  45

Val Arg Ile Asp Glu Arg Ser Ala Ser Phe Leu Ala Leu Ser Leu Ala
    50                  55                  60

Arg Thr Gln Ala Arg Pro Val Ala Val Val Met Thr Ser Gly Thr Ala
65                  70                  75                  80

Val Ala Asn Cys Leu Pro Ala Val Ala Glu Ala Ala His Ala His Ile
                85                  90                  95

Pro Leu Ile Val Leu Ser Ala Asp Arg Pro Ala His Leu Val Gly Thr
            100                 105                 110

Gly Ala Ser Gln Thr Ile Asn Gln Thr Gly Ile Phe Gly Asp Leu Ala
        115                 120                 125

Pro Thr Val Gly Ile Thr Glu Leu Asp Gln Val Ala Gln Ile Ala Glu
    130                 135                 140

Ser Leu Ala Gln Gly Ala Ser Gln Ile Pro Arg His Phe Asn Leu Ala
145                 150                 155                 160

Leu Asp Val Pro Leu Val Ala Pro Glu Leu Pro Glu Leu His Gly Glu
                165                 170                 175

Ala Val Gly Ala Ser Trp Thr His Arg Trp Ile Asn His Gly Glu Val
            180                 185                 190

Thr Val Asp Leu Gly Glu His Thr Leu Val Ile Ala Gly Asp Glu Ala
        195                 200                 205

Trp Glu Val Glu Gly Leu Glu Asp Val Pro Thr Ile Ala Glu Pro Thr
    210                 215                 220

Ala Pro Lys Pro Tyr Asn Pro Val His Pro Leu Ala Ala Glu Ile Leu
225                 230                 235                 240

Leu Lys Glu Gln Val Ser Ala Glu Gly Tyr Val Val Asn Thr Arg Pro
                245                 250                 255

Asp His Val Ile Val Val Gly His Pro Thr Leu His Arg Gly Val Leu
            260                 265                 270

Lys Leu Met Ser Asp Pro Gly Ile Lys Leu Thr Val Leu Ser Arg Thr

275                 280                 285

Asp Ile Ile Thr Asp Pro Gly Arg His Ala Asp Gln Val Gly Ser Thr
    290                 295                 300

Val Lys Val Thr Gly Thr Gln Glu Lys Gln Trp Leu Lys Ile Cys Ser
305                 310                 315                 320

Ala Ala Ser Glu Leu Ala Ala Asp Gly Val Arg Asp Val Leu Asp Asn
                325                 330                 335

Gln Glu Phe Gly Phe Thr Gly Leu His Val Ala Ala Ala Val Ala Asp
            340                 345                 350

Thr Leu Gly Thr Gly Asp Thr Leu Phe Ala Ala Ala Ser Asn Ser Ile
        355                 360                 365

Arg Asp Leu Ser Leu Val Gly Met Pro Phe Asp Gly Val Asp Thr Phe
    370                 375                 380

Ser Pro Arg Gly Val Ala Gly Ile Asp Gly Ser Val Ala Gln Ala Ile
385                 390                 395                 400

Gly Thr Ser Leu Ala Val Gln Ser Arg His Pro Asp Glu Ile Arg Ala
                405                 410                 415

Pro Arg Thr Val Ala Leu Leu Gly Asp Leu Ser Phe Leu His Asp Ile
            420                 425                 430

Gly Gly Leu Leu Ile Gly Pro Asp Glu Pro Arg Pro Glu Asn Leu Thr
        435                 440                 445

Ile Val Val Ser Asn Asp Asn Gly Gly Gly Ile Phe Glu Leu Leu Glu
    450                 455                 460

Thr Gly Ala Asp Gly Leu Arg Pro Asn Phe Glu Arg Ala Phe Gly Thr
465                 470                 475                 480

Pro His Asp Ala Ser Ile Ala Asp Leu Cys Ala Gly Tyr Gly Ile Glu
                485                 490                 495

His Gln Val Val Asp Asn Leu Gln Asp Leu Ile Ile Ala Leu Val Asp
            500                 505                 510

Thr Thr Glu Val Ser Gly Phe Thr Ile Ile Glu Ala Ser Thr Val Arg
        515                 520                 525

Asp Thr Arg Arg Ala Gln Gln Gln Ala Leu Met Asp Thr Val His
    530                 535                 540

<210>    18
<211>    933
<212>    PRT
<213>    Escherichia coli

<400>    18
Met Gln Asn Ser Ala Leu Lys Ala Trp Leu Asp Ser Ser Tyr Leu Ser
1               5                   10                  15

Gly Ala Asn Gln Ser Trp Ile Glu Gln Leu Tyr Glu Asp Phe Leu Thr

20                  25                  30

Asp Pro Asp Ser Val Asp Ala Asn Trp Arg Ser Thr Phe Gln Gln Leu
        35                  40                  45

Pro Gly Thr Gly Val Lys Pro Asp Gln Phe His Ser Gln Thr Arg Glu
    50                  55                  60

Tyr Phe Arg Arg Leu Ala Lys Asp Ala Ser Arg Tyr Ser Ser Thr Ile
65                  70                  75                  80

Ser Asp Pro Asp Thr Asn Val Lys Gln Val Lys Val Leu Gln Leu Ile
                85                  90                  95

Asn Ala Tyr Arg Phe Arg Gly His Gln His Ala Asn Leu Asp Pro Leu
            100                 105                 110

Gly Leu Trp Gln Gln Asp Lys Val Ala Asp Leu Asp Pro Ser Phe His
        115                 120                 125

Asp Leu Thr Glu Ala Asp Phe Gln Glu Thr Phe Asn Val Gly Ser Phe
    130                 135                 140

Ala Ser Gly Lys Glu Thr Met Lys Leu Gly Glu Leu Leu Glu Ala Leu
145                 150                 155                 160

Lys Gln Thr Tyr Cys Gly Pro Ile Gly Ala Glu Tyr Met His Ile Thr
                165                 170                 175

Ser Thr Glu Glu Lys Arg Trp Ile Gln Gln Arg Ile Glu Ser Gly Arg
            180                 185                 190

Ala Thr Phe Asn Ser Glu Glu Lys Lys Arg Phe Leu Ser Glu Leu Thr
        195                 200                 205

Ala Ala Glu Gly Leu Glu Arg Tyr Leu Gly Ala Lys Phe Pro Gly Ala
    210                 215                 220

Lys Arg Phe Ser Leu Glu Gly Gly Asp Ala Leu Ile Pro Met Leu Lys
225                 230                 235                 240

Glu Met Ile Arg His Ala Gly Asn Ser Gly Thr Arg Glu Val Val Leu
                245                 250                 255

Gly Met Ala His Arg Gly Arg Leu Asn Val Leu Val Asn Val Leu Gly
            260                 265                 270

Lys Lys Pro Gln Asp Leu Phe Asp Glu Phe Ala Gly Lys His Lys Glu
        275                 280                 285

His Leu Gly Thr Gly Asp Val Lys Tyr His Met Gly Phe Ser Ser Asp
    290                 295                 300

Phe Gln Thr Asp Gly Gly Leu Val His Leu Ala Leu Ala Phe Asn Pro
305                 310                 315                 320

Ser His Leu Glu Ile Val Ser Pro Val Val Ile Gly Ser Val Arg Ala
                325                 330                 335

Arg Leu Asp Arg Leu Asp Glu Pro Ser Ser Asn Lys Val Leu Pro Ile
            340                 345                 350

Thr Ile His Gly Asp Ala Ala Val Thr Gly Gln Gly Val Val Gln Glu

355                 360                 365

Thr Leu Asn Met Ser Lys Ala Arg Gly Tyr Glu Val Gly Gly Thr Val
    370                 375                 380

Arg Ile Val Ile Asn Asn Gln Val Gly Phe Thr Thr Ser Asn Pro Leu
385                 390                 395                 400

Asp Ala Arg Ser Thr Pro Tyr Cys Thr Asp Ile Gly Lys Met Val Gln
                405                 410                 415

Ala Pro Ile Phe His Val Asn Ala Asp Asp Pro Glu Ala Val Ala Phe
            420                 425                 430

Val Thr Arg Leu Ala Leu Asp Phe Arg Asn Thr Phe Lys Arg Asp Val
        435                 440                 445

Phe Ile Asp Leu Val Cys Tyr Arg Arg His Gly His Asn Glu Ala Asp
    450                 455                 460

Glu Pro Ser Ala Thr Gln Pro Leu Met Tyr Gln Lys Ile Lys Lys His
465                 470                 475                 480

Pro Thr Pro Arg Lys Ile Tyr Ala Asp Lys Leu Glu Gln Glu Lys Val
                485                 490                 495

Ala Thr Leu Glu Asp Ala Thr Glu Met Val Asn Leu Tyr Arg Asp Ala
            500                 505                 510

Leu Asp Ala Gly Asp Cys Val Val Ala Glu Trp Arg Pro Met Asn Met
        515                 520                 525

His Ser Phe Thr Trp Ser Pro Tyr Leu Asn His Glu Trp Asp Glu Glu
    530                 535                 540

Tyr Pro Asn Lys Val Glu Met Lys Arg Leu Gln Glu Leu Ala Lys Arg
545                 550                 555                 560

Ile Ser Thr Val Pro Glu Ala Val Glu Met Gln Ser Arg Val Ala Lys
                565                 570                 575

Ile Tyr Gly Asp Arg Gln Ala Met Ala Ala Gly Glu Lys Leu Phe Asp
            580                 585                 590

Trp Gly Gly Ala Glu Asn Leu Ala Tyr Ala Thr Leu Val Asp Glu Gly
        595                 600                 605

Ile Pro Val Arg Leu Ser Gly Glu Asp Ser Gly Arg Gly Thr Phe Phe
    610                 615                 620

His Arg His Ala Val Ile His Asn Gln Ser Asn Gly Ser Thr Tyr Thr
625                 630                 635                 640

Pro Leu Gln His Ile His Asn Gly Gln Gly Ala Phe Arg Val Trp Asp
                645                 650                 655

Ser Val Leu Ser Glu Glu Ala Val Leu Ala Phe Glu Tyr Gly Tyr Ala
            660                 665                 670

Thr Ala Glu Pro Arg Thr Leu Thr Ile Trp Glu Ala Gln Phe Gly Asp
        675                 680                 685

Phe Ala Asn Gly Ala Gln Val Val Ile Asp Gln Phe Ile Ser Ser Gly

690                 695                 700

Glu Gln Lys Trp Gly Arg Met Cys Gly Leu Val Met Leu Leu Pro His
705                 710                 715                 720

Gly Tyr Glu Gly Gln Gly Pro Glu His Ser Ser Ala Arg Leu Glu Arg
                725                 730                 735

Tyr Leu Gln Leu Cys Ala Glu Gln Asn Met Gln Val Cys Val Pro Ser
            740                 745                 750

Thr Pro Ala Gln Val Tyr His Met Leu Arg Arg Gln Ala Leu Arg Gly
        755                 760                 765

Met Arg Arg Pro Leu Val Val Met Ser Pro Lys Ser Leu Leu Arg His
    770                 775                 780

Pro Leu Ala Val Ser Ser Leu Glu Glu Leu Ala Asn Gly Thr Phe Leu
785                 790                 795                 800

Pro Ala Ile Gly Glu Ile Asp Glu Leu Asp Pro Lys Gly Val Lys Arg
                805                 810                 815

Val Val Met Cys Ser Gly Lys Val Tyr Tyr Asp Leu Leu Glu Gln Arg
            820                 825                 830

Arg Lys Asn Asn Gln His Asp Val Ala Ile Val Arg Ile Glu Gln Leu
        835                 840                 845

Tyr Pro Phe Pro His Lys Ala Met Gln Glu Val Leu Gln Gln Phe Ala
    850                 855                 860

His Val Lys Asp Phe Val Trp Cys Gln Glu Glu Pro Leu Asn Gln Gly
865                 870                 875                 880

Ala Trp Tyr Cys Ser Gln His His Phe Arg Glu Val Ile Pro Phe Gly
                885                 890                 895

Ala Ser Leu Arg Tyr Ala Gly Arg Pro Ala Ser Ala Ser Pro Ala Val
            900                 905                 910

Gly Tyr Met Ser Val His Gln Lys Gln Gln Gln Asp Leu Val Asn Asp
        915                 920                 925

Ala Leu Asn Val Glu
    930

<210>    19
<211>    1632
<212>    DNA
<213>    Corynebacterium glutamicum

<400>    19
atgtccagca cgccagctca agatcttgcc cgcgccgtta ttgattccct cgcaccacac        60

gtcactgacg tggtgttatg cccaggatcc aggaactcac cgttgtcgct tgagttgctg       120

gcgcggcagg atctgcgtgt ccatgtgcgt atcgacgagc gcagcgcctc atttttggcg        180

ctgtccctag cgcgtaccca ggcccggccg gtggctgtgg tgatgacctc cggcacggct       240

gtagctaact gcctgcctgc tgttgctgaa gctgcgcatg cccatatccc gttgattgtg       300

ctctctgctg accgtcctgc acatttggtg ggaacggggg cgagccaaac gattaaccag 360

accggtattt ttggtgatct tgcaccgacg gtcggtatca ctgagctgga tcaggtagcg 420

cagattgctg aaagccttgc tcagggggct tcccagattc cgcgtcattt caatcttgca 480

cttgatgttc ctttggttgc tcctgaactg ccagagcttc atggtgaggc agttggagca 540

tcatggacgc atcgctggat caaccacggt gaggtgaccg tggacctggg ggagcacacc 600

ctcgtgattg ccggtgatga agcatgggaa gtggaagggc tggaagatgt gcccaccatc 660

gctgaaccta ctgcaccaaa gccttataat ccggtgcacc cactggctgc tgaaatcttg 720

ctgaaggagc aggtctccgc ggaaggctat gtggtaaaca ccaggcctga tcatgtgatc 780

gtggtgggac accccacgct gcaccgcgga gtgttgaagt tgatgtcaga tcctggcatt 840

aaattaactg tgctttcacg caccgatatc atcactgatc ccggccgcca tgccgatcag 900

gtgggcagca cagtgaaagt caccggcacc caggaaaagc agtggctaaa gatctgttcg 960

gcagcatcag aacttgcggc cgatggtgtg cgtgacgtcc tggacaacca agaattcggt 1020

ttcaccggcc tccatgttgc cgcagccgtg gcggatacct taggcaccgg cgatactctc 1080

tttgctgcag catccaactc aatccgtgac ctctccctgg tgggtatgcc ttttgatggc 1140

gtggatacct tctccccacg aggtgtcgca ggcattgatg gttctgttgc tcaagcaatc 1200

ggcacttcac ttgctgtgca gtcccgccac cccgatgaaa tccgcgcgcc acgcactgtg 1260

gcccttctgg gcgatctgtc gttccttcac gatattggcg gactgctcat cggccctgat 1320

gaaccacgcc cagaaaacct caccatcgtg gtctccaacg acaacggtgg cggaatcttc 1380

gaactcctag aaaccggcgc agatggtctc cgccccaact tcgagcgtgc tttcggtacc 1440

ccacacgacg cgtccatcgc ggatctctgc gcaggctacg gcattgaaca ccaagtggta 1500

gacaacctcc aagacctcat catcgcgcta gttgatacca ccgaagtatc cggattcacc 1560

attattgaag cttcgaccgt ccgagatacc cgccgtgcac aacagcaagc tctcatggac 1620

acggtgcact aa 1632

<210> 20
<211> 2802
<212> DNA
<213> Escherichia coli

<400> 20
atgcagaaca gcgctttgaa agcctggttg gactcttctt acctctctgg cgcaaaccag 60

agctggatag aacagctcta tgaagacttc ttaaccgatc ctgactcggt tgacgctaac 120

tggcgttcga cgttccagca gttacctggt acgggagtca aaccggatca attccactct 180

caaacgcgtg aatatttccg ccgcctggcg aaagacgctt cacgttactc ttcaacgatc 240

tccgaccctg acaccaatgt gaagcaggtt aaagtcctgc agctcattaa cgcataccgc 300

ttccgtggtc accagcatgc gaatctcgat ccgctgggac tgtggcagca agataaagtg 360

gccgatctgg atccgtcttt ccacgatctg accgaagcag acttccagga gaccttcaac 420

gtcggttcat ttgccagcgg caaagaaacc atgaaactcg gcgaactgct ggaagcactc 480

aagcaaacct actgcggccc gattggtgcc gagtatatgc acattaccag cactgaagaa 540

aaacgctgga tccaacagcg tattgagtct ggtcgcgcga ctttcaatag cgaagagaaa 600

aaacgcttct taagcgaact gaccgccgct gaaggccttg aacgttacct cggcgcaaaa 660

ttccctggcg caaaacgctt ctcgctggaa ggcggtgacg cgttaatccc gatgcttaaa 720

gagatgatcc gccacgctgg caacagcggc acccgcgaag tggttctcgg aatggcgcac 780

cgtggtcgtc tgaacgtgct ggtgaacgtg ctgggtaaaa aaccgcaaga cttgttcgac 840

gagtttgccg gtaaacataa agaacacctc ggcacgggcg acgtgaaata ccacatgggc 900

ttctcgtctg acttccagac cgatggcggc ctggttcacc tggcgctggc gtttaacccg 960

tctcaccttg agattgtaag cccggtcgtt atcggttctg ttcgtgcccg tctggacaga 1020

cttgatgagc cgagcagcaa caaagtgctg ccaatcacca ttcatggtga cgccgcagtg 1080

accgggcagg gcgtggttca ggaaaccctg aacatgtcga aagcgcgtgg ttatgaagtt 1140

ggcggtacgg tacgtatcgt tatcaacaac caggttggct tcaccacctc taacccgctg 1200

gatgcccgtt cgacgccgta ctgtactgat atcggtaaga tggttcaggc accgattttc 1260

cacgttaacg cggatgatcc ggaagccgtt gcctttgtta cccgtctggc gctcgatttc 1320

cgtaacacct ttaaacgtga tgtcttcatc gacctggtat gctaccgccg tcacggccac 1380

aacgaagccg acgagccgag cgcaacccag ccgctgatgt atcagaaaat caaaaaacat 1440

ccgacgccgc gcaaaatcta cgctgacaag ctggagcagg aaaaagtcgc gacgctggaa 1500

gatgccaccg agatggttaa cctgtaccgc gatgcgctgg atgctggcga ttgcgttgta 1560

gcagagtggc gtccgatgaa catgcactct ttcacctggt cgccgtacct caaccatgaa 1620

tgggacgaag agtacccgaa caaagttgag atgaagcgcc tgcaggaact ggctaaacgc 1680

atcagcacgg tgccggaagc ggttgaaatg cagtctcgcg ttgccaagat ttatggcgat 1740

cgccaggcga tggcagccgg tgagaaactg ttcgactggg gcggcgcgga aaacctcgct 1800

tacgccacgt tggttgacga aggcattccg gttcgcctgt cgggtgaaga ctccggtcgc 1860

ggtaccttct tccaccgcca cgcggtgatc cacaaccagt ctaacggttc cacttacacg 1920

ccgctgcaac atatccataa cggccagggc gcgttccgcg tctgggactc tgtactgtct 1980

gaagaagccg tactggcgtt tgaatacggt tatgccaccg cagaaccacg caccctgacc 2040

atctgggaag cacagttcgg tgacttcgcc aacggtgcac aggtggttat cgaccagttc 2100

atctcctctg gcgaacagaa atggggccgg atgtgtggcc tggtgatgtt gctgccgcac 2160

ggttacgaag ggcaggggcc ggagcactcc tccgcgcgtc tggaacgtta tctgcaactt 2220

tgcgctgagc aaaacatgca ggtttgcgta ccttctaccc cggcacaggt ttaccacatg 2280

ctgcgtcgtc aggcgttgcg cgggatgcgt cgtccactgg tcgtgatgtc gccgaaatcc 2340

ctgctgcgtc atccgctggc ggtatccagc ctcgaagaac tggcgaacgg caccttcctg 2400

ccagccatcg gtgaaatcga cgagcttgat ccgaagggcg tgaagcgcgt agtgatgtgt 2460

tctggtaagg tttattacga cctgctggaa caacgtcgta agaacaatca acacgatgtc 2520

gccattgtgc gtatcgagca actctacccg ttcccgcata aagcgatgca ggaagtgttg 2580

cagcagtttg ctcacgtcaa ggattttgtc tggtgccagg aagagccgct caaccagggc 2640

gcatggtact gcagccagca tcatttccgt gaagtgattc cgtttggggc ttctctgcgt 2700

tatgcaggcc gcccagcctc cgcctctccg gcggtagggt atatgtccgt tcaccagaaa 2760

cagcaacaag atctggttaa tgacgcgctg aacgtcgaat aa 2802

<210> 21
<211> 490
<212> PRT
<213> Corynebacterium glutamicum

<400> 21
Met Thr Ile Asn Val Ser Glu Leu Leu Ala Lys Val Pro Thr Gly Leu
1               5                   10                  15

Leu Ile Gly Asp Ser Trp Val Glu Ala Ser Asp Gly Gly Thr Phe Asp
            20                  25                  30

Val Glu Asn Pro Ala Thr Gly Glu Thr Ile Ala Thr Leu Ala Ser Ala
        35                  40                  45

Thr Ser Glu Asp Ala Leu Ala Ala Leu Asp Ala Ala Cys Ala Val Gln
    50                  55                  60

Ala Glu Trp Ala Arg Met Pro Ala Arg Glu Arg Ser Asn Ile Leu Arg
65                  70                  75                  80

Arg Gly Phe Glu Leu Val Ala Glu Arg Ala Glu Glu Phe Ala Thr Leu
                85                  90                  95

Met Thr Leu Glu Met Gly Lys Pro Leu Ala Glu Ala Arg Gly Glu Val
            100                 105                 110

Thr Tyr Gly Asn Glu Phe Leu Arg Trp Phe Ser Glu Glu Ala Val Arg
        115                 120                 125

Leu Tyr Gly Arg Tyr Gly Thr Thr Pro Glu Gly Asn Leu Arg Met Leu
    130                 135                 140

Thr Ala Leu Lys Pro Val Gly Pro Cys Leu Leu Ile Thr Pro Trp Asn
145                 150                 155                 160

Phe Pro Leu Ala Met Ala Thr Arg Lys Val Ala Pro Ala Ile Ala Ala
                165                 170                 175

Gly Cys Val Met Val Leu Lys Pro Ala Arg Leu Thr Pro Leu Thr Ser

180                 185                 190

Gln Tyr Phe Ala Gln Thr Met Leu Asp Ala Gly Leu Pro Ala Gly Val
        195                 200                 205

Leu Asn Val Val Ser Gly Ala Ser Ala Ser Ala Ile Ser Asn Pro Ile
    210                 215                 220

Met Glu Asp Asp Arg Leu Arg Lys Val Ser Phe Thr Gly Ser Thr Pro
225                 230                 235                 240

Val Gly Gln Gln Leu Leu Lys Lys Ala Ala Asp Lys Val Leu Arg Thr
                245                 250                 255

Ser Met Glu Leu Gly Gly Asn Ala Pro Phe Ile Val Phe Glu Asp Ala
            260                 265                 270

Asp Leu Asp Leu Ala Ile Glu Gly Ala Met Gly Ala Lys Met Arg Asn
        275                 280                 285

Ile Gly Glu Ala Cys Thr Ala Ala Asn Arg Phe Leu Val His Glu Ser
    290                 295                 300

Val Ala Asp Glu Phe Gly Arg Arg Phe Ala Ala Arg Leu Glu Glu Gln
305                 310                 315                 320

Val Leu Gly Asn Gly Leu Asp Glu Gly Val Thr Val Gly Pro Leu Val
                325                 330                 335

Glu Glu Lys Ala Arg Asp Ser Val Ala Ser Leu Val Asp Ala Ala Val
            340                 345                 350

Ala Glu Gly Ala Thr Val Leu Thr Gly Gly Lys Ala Gly Thr Gly Ala
        355                 360                 365

Gly Tyr Phe Tyr Glu Pro Thr Val Leu Thr Gly Val Ser Thr Asp Ala
    370                 375                 380

Ala Ile Leu Asn Glu Glu Ile Phe Gly Pro Val Ala Pro Ile Val Thr
385                 390                 395                 400

Phe Gln Thr Glu Glu Glu Ala Leu Arg Leu Ala Asn Ser Thr Glu Tyr
                405                 410                 415

Gly Leu Ala Ser Tyr Val Phe Thr Gln Asp Thr Ser Arg Ile Phe Arg
            420                 425                 430

Val Ser Asp Gly Leu Glu Phe Gly Leu Val Gly Val Asn Ser Gly Val
        435                 440                 445

Ile Ser Asn Ala Ala Ala Pro Phe Gly Gly Val Lys Gln Ser Gly Met
    450                 455                 460

Gly Arg Glu Gly Gly Leu Glu Gly Ile Glu Glu Tyr Thr Ser Val Gln
465                 470                 475                 480

Tyr Ile Gly Ile Arg Asp Pro Tyr Ala Gly
                485                 490


<210>    22
<211>    453
<212>    PRT

<213>    Corynebacterium glutamicum

<400>    22
Met Ser Leu Thr Phe Pro Val Ile Asn Pro Ser Asp Gly Ser Thr Ile
1               5                   10                  15

Thr Glu Leu Glu Asn His Asp Ser Thr Gln Trp Met Ser Ala Leu Ser
            20                  25                  30

Asp Ala Val Ala Ala Gly Pro Ser Trp Ala Ala Lys Thr Pro Arg Glu
        35                  40                  45

Arg Ser Val Val Leu Thr Ala Ile Phe Glu Ala Leu Thr Glu Arg Ala
    50                  55                  60

Gln Glu Leu Ala Glu Ile Ile His Leu Glu Ala Gly Lys Ser Val Ala
65                  70                  75                  80

Glu Ala Leu Gly Glu Val Ala Tyr Gly Ala Glu Tyr Phe Arg Trp Phe
                85                  90                  95

Ala Glu Glu Ala Val Arg Leu Pro Gly Arg Tyr Gly Gln Ser Pro Ser
            100                 105                 110

Gly Ile Gly His Ile Ala Val Thr Arg Ala Pro Val Gly Pro Val Leu
        115                 120                 125

Ala Ile Thr Pro Trp Asn Phe Pro Ile Ala Met Ala Thr Arg Lys Ile
    130                 135                 140

Ala Pro Ala Leu Ala Ala Gly Cys Pro Val Leu Val Lys Pro Ala Ser
145                 150                 155                 160

Glu Thr Pro Leu Thr Met Val Lys Val Gly Glu Ile Ile Ala Ser Val
                165                 170                 175

Phe Asp Thr Phe Asn Ile Pro Gln Gly Leu Val Ser Ile Ile Thr Thr
            180                 185                 190

Thr Arg Asp Ala Glu Leu Ser Ala Glu Leu Met Ala Asp Pro Arg Leu
        195                 200                 205

Ala Lys Val Thr Phe Thr Gly Ser Thr Asn Val Gly Arg Ile Leu Val
    210                 215                 220

Arg Gln Ser Ala Asp Arg Leu Leu Arg Thr Ser Met Glu Leu Gly Gly
225                 230                 235                 240

Asn Ala Ala Phe Val Ile Asp Glu Ala Ala Asp Leu Asp Glu Ala Val
                245                 250                 255

Ser Gly Ala Ile Ala Ala Lys Leu Arg Asn Ala Gly Gln Val Cys Ile
            260                 265                 270

Ala Ala Asn Arg Phe Leu Val His Glu Ser Arg Ala Ala Glu Phe Thr
        275                 280                 285

Ser Lys Leu Ala Thr Ala Met Gln Asn Thr Pro Ile Gly Pro Val Ile
    290                 295                 300

Ser Ala Arg Gln Arg Asp Arg Ile Ala Ala Leu Val Asp Glu Ala Ile
305                 310                 315                 320

Thr Asp Gly Ala Arg Leu Ile Ile Gly Gly Glu Val Pro Asp Gly Ser
                325                 330                 335

Gly Phe Phe Tyr Pro Ala Thr Ile Leu Ala Asp Val Pro Ala Gln Ser
            340                 345                 350

Arg Ile Val His Glu Glu Ile Phe Gly Pro Val Ala Thr Ile Ala Thr
        355                 360                 365

Phe Thr Asp Leu Ala Glu Gly Val Ala Gln Ala Asn Ser Thr Glu Phe
    370                 375                 380

Gly Leu Ala Ala Tyr Gly Phe Ser Asn Asn Val Lys Ala Thr Gln Tyr
385                 390                 395                 400

Met Ala Glu His Leu Glu Ala Gly Met Val Gly Ile Asn Arg Gly Ala
                405                 410                 415

Ile Ser Asp Pro Ala Ala Pro Phe Gly Gly Ile Gly Gln Ser Gly Phe
            420                 425                 430

Gly Arg Glu Gly Gly Thr Glu Gly Ile Glu Glu Tyr Leu Ser Val Arg
        435                 440                 445

Tyr Leu Ala Leu Pro
    450


<210>    23
<211>    521
<212>    PRT
<213>    Corynebacterium glutamicum

<400>    23
Met Ile Lys Arg Leu Pro Leu Gly Pro Leu Pro Lys Glu Leu His Gln
 1               5                  10                  15

Thr Leu Leu Asp Leu Thr Ala Asn Ala Gln Asp Ala Ala Lys Val Glu
            20                  25                  30

Val Ile Ala Pro Phe Thr Gly Glu Thr Leu Gly Phe Val Phe Asp Gly
        35                  40                  45

Asp Glu Gln Asp Val Glu His Ala Phe Ala Leu Ser Arg Ala Ala Gln
    50                  55                  60

Lys Lys Trp Val His Thr Thr Ala Val Glu Arg Lys Lys Ile Phe Leu
65                  70                  75                  80

Lys Phe His Asp Leu Val Leu Lys Asn Arg Glu Leu Leu Met Asp Ile
                85                  90                  95

Val Gln Leu Glu Thr Gly Lys Asn Arg Ala Ser Ala Ala Asp Glu Val
            100                 105                 110

Leu Asp Val Ala Ile Thr Thr Arg Phe Tyr Ala Asn Asn Ala Gly Lys
        115                 120                 125

Phe Leu Asn Asp Lys Lys Arg Pro Gly Ala Leu Pro Ile Ile Thr Lys
    130                 135                 140

Asn Thr Gln Gln Tyr Val Pro Lys Gly Val Val Gly Gln Ile Thr Pro
145                 150                 155                 160

Trp Asn Tyr Pro Leu Thr Leu Gly Val Ser Asp Ala Val Pro Ala Leu
                165                 170                 175

Leu Ala Gly Asn Ala Val Val Ala Lys Pro Asp Leu Ala Thr Pro Phe
            180                 185                 190

Ser Cys Leu Ile Met Val His Leu Leu Ile Glu Ala Gly Leu Pro Arg
        195                 200                 205

Asp Leu Met Gln Val Val Thr Gly Pro Gly Asp Ile Val Gly Gly Ala
    210                 215                 220

Ile Ala Ala Gln Cys Asp Phe Leu Met Phe Thr Gly Ser Thr Ala Thr
225                 230                 235                 240

Gly Arg Ile Leu Gly Arg Thr Met Gly Glu Arg Leu Val Gly Phe Ser
                245                 250                 255

Ala Glu Leu Gly Gly Lys Asn Pro Leu Ile Val Ala Lys Asp Ala Asp
            260                 265                 270

Leu Asp Lys Val Glu Ala Glu Leu Pro Gln Ala Cys Phe Ser Asn Ser
        275                 280                 285

Gly Gln Leu Cys Val Ser Thr Glu Arg Ile Tyr Val Glu Glu Asp Val
    290                 295                 300

Tyr Glu Glu Val Ile Ala Arg Phe Ser Lys Ala Ala Lys Ala Met Ser
305                 310                 315                 320

Ile Gly Ala Gly Phe Glu Trp Lys Tyr Glu Met Gly Ser Leu Ile Asn
                325                 330                 335

Gln Ala Gln Leu Asp Arg Val Ser Thr Phe Val Asp Gln Ala Lys Ala
            340                 345                 350

Ala Gly Ala Thr Val Leu Cys Gly Gly Lys Ser Arg Pro Asp Ile Gly
        355                 360                 365

Pro Phe Phe Tyr Glu Pro Thr Val Leu Ala Asp Val Pro Glu Gly Thr
    370                 375                 380

Pro Leu Leu Thr Glu Glu Val Phe Gly Pro Val Val Phe Ile Glu Lys
385                 390                 395                 400

Val Ala Thr Leu Glu Glu Ala Val Asp Lys Ala Asn Gly Thr Pro Tyr
                405                 410                 415

Gly Leu Asn Ala Ser Val Phe Gly Ser Ser Glu Thr Gly Asn Leu Val
            420                 425                 430

Ala Gly Gln Leu Glu Ala Gly Gly Ile Gly Ile Asn Asp Gly Tyr Ala
        435                 440                 445

Ala Thr Trp Ala Ser Val Ser Thr Pro Leu Gly Gly Met Lys Gln Ser
    450                 455                 460

Gly Leu Gly His Arg His Gly Ala Glu Gly Ile Thr Lys Tyr Ala Glu
465                 470                 475                 480

Ile Arg Asn Ile Ala Glu Gln Arg Trp Met Ser Met Arg Gly Pro Ala
                485                 490                 495

Lys Met Pro Arg Lys Val Tyr Ser Asp Thr Val Ala Thr Ala Leu Lys
            500                 505                 510

Leu Gly Lys Ile Phe Lys Val Leu Pro
        515                 520

<210>    24
<211>    1473
<212>    DNA
<213>    Corynebacterium glutamicum

<400>    24
atgactatta atgtctccga actacttgcc aaagtcccca cgggtctact gattggtgat      60

tcctgggtgg aagcatccga cggcggtact ttcgatgtgg aaaacccagc gacgggtgaa     120

acaatcgcaa cgctcgcgtc tgctacttcc gaggatgcac tggctgctct tgatgctgca     180

tgcgctgttc aggccgagtg ggctaggatg ccagcgcgcg agcgttctaa tattttacgc     240

cgcggttttg agctcgtagc agaacgtgca gaagagttcg ccaccctcat gaccttggaa     300

atgggcaagc ctttggctga agctcgcggc gaagtcacct acggcaacga attcctgcgc     360

tggttctctg aggaagcagt tcgtctgtat ggccgttacg gaaccacacc agaaggcaac     420

ttgcggatgc tgaccgccct caagccagtt ggcccgtgcc tcctgatcac cccatggaac     480

ttcccactag caatggctac ccgcaaggtc gcacctgcga tcgctgcagg ttgtgtcatg     540

gtgctcaagc cagctcgact taccccgctg acctcccagt attttgctca gaccatgctt     600

gatgccggtc ttccagcagg tgtcctcaat gtggtctccg gtgcttccgc ctctgcgatt     660

tccaacccga ttatggaaga cgatcgcctt cgtaaagtct ccttcaccgg ctccacccca     720

gttggccagc agctgctcaa aaaggctgcc gataaagttc tgcgcacctc catggaactt     780

ggtggcaacg cacctttcat tgtcttcgag gacgccgacc tagatctcgc gatcgaaggt     840

gccatgggtg ccaaaatgcg caacatcggc gaagcttgca ccgcagccaa ccgtttctta     900

gtccacgaat ccgtcgccga tgaattcggc cgtcgcttcg ctgcccgcct tgaagagcaa     960

gtcctaggca acggcctcga cgaaggcgtc accgtgggcc ccctggttga ggaaaaagca    1020

cgagacagcg ttgcatcgct tgtcgacgcc gccgtcgccg aaggtgccac cgtcctcacc    1080

ggcggcaagg ccggcacagg tgcaggctac ttctacgaac caacggtgct cacgggagtt    1140

tcaacagatg cggctatcct gaacgaagag atcttcggtc ccgtcgcacc gatcgtcacc    1200

ttccaaaccg aggaagaagc cctgcgtcta gccaactcca ccgaatacgg actggcctcc    1260

tatgtgttca cccaggacac ctcacgtatt ttccgcgtct ccgatggtct cgagttcggc    1320

ctagtgggcg tcaattccgg tgtcatctct aacgctgctg caccttttgg tggcgtaaaa    1380

caatccggaa tgggccgcga aggtggtctc gaaggaatcg aggagtacac ctccgtgcag    1440

tacatcggta tccgggatcc ttacgccggc tag                                 1473

<210>	25
<211>	1362
<212>	DNA
<213>	Corynebacterium glutamicum

<400>	25
gtgtctttga ccttcccagt aatcaacccc agcgatggct ccaccatcac cgagctagaa	60

aaccacgatt ccacccagtg gatgtccgcg ctctctgatg cagttgcagc tggtccttca	120

tgggctgcga aaactccccg cgaaagatcc gtggtactca ccgcaatctt cgaagcactg	180

accgaacgcg cccaagaact tgcagagatc atccacctgg aagctggaaa atccgttgca	240

gaagctcttg gtgaagtcgc ttatggtgca gaatacttcc gttggtttgc ggaagaagca	300

gtgcgcctgc ccggccgcta cggacagtca ccttccggaa tcggtcacat cgccgtcacc	360

cgcgcacccg tgggaccagt gctggcgatc accccatgga atttccccat cgccatggcc	420

acccgcaaaa tcgccccagc cctggccgct ggttgccccg tgttggtgaa acctgcttcc	480

gaaaccccac tgaccatggt caaagtgggg gagatcatcg cctccgtctt tgataccttt	540

aatatcccgc agggcttggt ctcaatcatc accaccactc gagatgcaga gctatcggca	600

gaactcatgg ctgatcctcg cttggctaaa gtcaccttca ctggatcaac caacgtggga	660

cgcatcctgg tccgccaatc cgcggaccga ctgctgcgca cctccatgga actcggcgga	720

aatgcagctt ttgttatcga cgaagccgca gacctcgacg aagccgtatc cggtgccatc	780

gccgcaaaac tccgcaacgc cggccaagta tgcatcgcag ctaaccgttt cttggttcat	840

gaatcccgcg ctgccgaatt cacctcaaag ctggcgacag ccatgcagaa cactcccatt	900

gggccggtga tttctgcccg ccaacgcgac cggatcgcag cactagtgga tgaagccatc	960

accgacggcg cccgcctcat catcggtggg gaggtccccg acggctccgg cttcttctat	1020

ccagccacca tcttggccga tgtccctgca cagtcacgga ttgtgcatga ggaaatcttc	1080

ggacctgtgg ccaccattgc cactttcacc gacttggccg aaggcgttgc acaagcaaat	1140

tccaccgaat tcggcctcgc agcctacgga ttcagcaaca atgtgaaagc aacacagtac	1200

atggcggaac acttggaagc cggaatggtc ggaatcaaca gaggcgccat ctctgaccca	1260

gcagcacctt ttggcggcat cggacaatcc ggcttcggca gagaaggcgg aaccgaagga	1320

atcgaagaat atctctccgt gcgttacctc gctttgccgt ga	1362

<210>	26
<211>	1566
<212>	DNA
<213>	Corynebacterium glutamicum

<400>	26
atgatcaaac gtcttccttt aggtccgctg cctaaagaac ttcatcagac tctgcttgat	60

ctgaccgcaa atgcccaaga tgcggcgaaa gtggaggtta tagcgccatt tactggcgag 120

accctcggat ttgtttttga tggtgatgag caagacgtcg agcatgcttt tgcactttca 180

agggcagccc agaaaaagtg ggtgcacacc acggcagtgg aacggaagaa gatcttcctg 240

aagtttcatg atctggtatt gaaaaaccgt gagctgctca tggacatcgt gcagttggaa 300

acaggcaaaa atcgagcatc ggctgccgat gaggtgttgg acgttgcgat caccacccgc 360

ttctacgcaa acaatgcagg aaagttttta aatgacaaga aacgccccgg cgcgcttccg 420

atcatcacga aaaacacaca acagtatgtg cccaagggag tggtcggcca gatcacgccg 480

tggaattacc ctttaacttt gggagtatct gatgctgttc cggcgctgct ggcaggaaac 540

gcagtggtgg ctaaacctga cctcgcgaca cctttctcct gcttgatcat ggtgcacctg 600

ctcattgaag ccggtctgcc gcgtgatttg atgcaggttg tcaccggccc tggcgatatt 660

gttggcggtg cgattgcagc tcagtgtgat ttcctcatgt tcactggatc cacggccacg 720

ggccggatct tgggtcggac aatgggtgag cgtttggtgg gtttctctgc ggaattaggc 780

ggaaagaacc ctcttattgt ggccaaggat gcagatctgg acaaggtgga agctgagctt 840

ccgcaggcgt gtttttccaa ctcggggcaa ttgtgtgtct ccactgaacg tatttatgtc 900

gaggaagacg tgtacgagga ggtgattgca cggtttagca aggcggcgaa agccatgtcc 960

attggtgccg gatttgagtg gaaatatgag atgggttcgt tgatcaatca ggcgcagctg 1020

gatcgggtga gcacctttgt tgatcaggct aaagctgcgg gcgccacggt gctgtgcggt 1080

ggcaagtcac gccctgatat tggtcccttc ttctatgagc ccacggtatt ggcggatgtc 1140

ccagagggca ccccactgct cacggaggaa gtcttcgggc cggtggtgtt catcgaaaag 1200

gtagccacac tggaagaagc cgtcgataag gcaaatggca cgccctacgg cctgaatgcg 1260

tccgtctttg ggtcgtcgga aaccggcaat cttgttgcag gccagctgga agctggcggt 1320

atcggtatta atgatggcta cgccgcgacg tgggcgagcg tgtccacgcc tctgggtggc 1380

atgaagcagt cggggctggg gcaccgccat ggtgcggagg gaattacaaa atatgcggag 1440

atccgaaaca tcgcggagca gcgctggatg tctatgcgtg ggccggccaa aatgccgcga 1500

aaggtgtact cagacaccgt ggccacagcg ctaaagctgg gcaaaatctt taaagttttg 1560

ccgtag 1566

<210> 27
<211> 314
<212> PRT
<213> Corynebacterium glutamicum

<400> 27
Met Lys Glu Thr Val Gly Asn Lys Ile Val Leu Ile Gly Ala Gly Asp
1               5                   10                  15

Val Gly Val Ala Tyr Ala Tyr Ala Leu Ile Asn Gln Gly Met Ala Asp

20                  25                  30

His Leu Ala Ile Ile Asp Ile Asp Glu Lys Lys Leu Glu Gly Asn Val
        35                  40                  45

Met Asp Leu Asn His Gly Val Val Trp Ala Asp Ser Arg Thr Arg Val
    50                  55                  60

Thr Lys Gly Thr Tyr Ala Asp Cys Glu Asp Ala Ala Met Val Val Ile
65                  70                  75                  80

Cys Ala Gly Ala Ala Gln Lys Pro Gly Glu Thr Arg Leu Gln Leu Val
                85                  90                  95

Asp Lys Asn Val Lys Ile Met Lys Ser Ile Val Gly Asp Val Met Asp
            100                 105                 110

Ser Gly Phe Asp Gly Ile Phe Leu Val Ala Ser Asn Pro Val Asp Ile
        115                 120                 125

Leu Thr Tyr Ala Val Trp Lys Phe Ser Gly Leu Glu Trp Asn Arg Val
    130                 135                 140

Ile Gly Ser Gly Thr Val Leu Asp Ser Ala Arg Phe Arg Tyr Met Leu
145                 150                 155                 160

Gly Glu Leu Tyr Glu Val Ala Pro Ser Ser Val His Ala Tyr Ile Ile
                165                 170                 175

Gly Glu His Gly Asp Thr Glu Leu Pro Val Leu Ser Ser Ala Thr Ile
            180                 185                 190

Ala Gly Val Ser Leu Ser Arg Met Leu Asp Lys Asp Pro Glu Leu Glu
        195                 200                 205

Gly Arg Leu Glu Lys Ile Phe Glu Asp Thr Arg Asp Ala Ala Tyr His
    210                 215                 220

Ile Ile Asp Ala Lys Gly Ser Thr Ser Tyr Gly Ile Gly Met Gly Leu
225                 230                 235                 240

Ala Arg Ile Thr Arg Ala Ile Leu Gln Asn Gln Asp Val Ala Val Pro
                245                 250                 255

Val Ser Ala Leu Leu His Gly Glu Tyr Gly Glu Glu Asp Ile Tyr Ile
            260                 265                 270

Gly Thr Pro Ala Val Val Asn Arg Arg Gly Ile Arg Arg Val Val Glu
        275                 280                 285

Leu Glu Ile Thr Asp His Glu Met Glu Arg Phe Lys His Ser Ala Asn
    290                 295                 300

Thr Leu Arg Glu Ile Gln Lys Gln Phe Phe
305                 310

<210>   28
<211>   43
<212>   DNA
<213>   Artificial Sequence

<220>

<223>     ldhA_5'_HindIII

<400>     28
catgattacg ccaagcttga gagcccacca cattgcgatt tcc                         43

<210>     29
<211>     42
<212>     DNA
<213>     Artificial Sequence

<220>
<223>     ldhA_up_3'_XhoI

<400>     29
tcgaaactcg agtttcgatc ccacttcctg atttccctaa cc                          42

<210>     30
<211>     39
<212>     DNA
<213>     Artificial Sequence

<220>
<223>     ldhA_dn_5'_XhoI

<400>     30
tcgaaactcg agtaaatctt tggcgcctag ttggcgacg                              39

<210>     31
<211>     46
<212>     DNA
<213>     Artificial Sequence

<220>
<223>     ldhA_3'_EcoRI

<400>     31
acgacggcca gtgaattcga cgacatctga gggtggataa agtggg                      46

<210>     32
<211>     20
<212>     DNA
<213>     Artificial Sequence

<220>
<223>     ldhA up

<400>     32
atcgggcata attaaaggtg                                                   20

<210>     33
<211>     22
<212>     DNA
<213>     Artificial Sequence

<220>
<223>    ldhA down

<400>    33
gtcacctcat caagttctag aa                                                22

<210>    34
<211>    36
<212>    DNA
<213>    Artificial Sequence

<220>
<223>    0049-1 for

<400>    34
gcaggcatgc aagcttaaag tccccacggg tctact                                 36

<210>    35
<211>    35
<212>    DNA
<213>    Artificial Sequence

<220>
<223>    0049-1 rev

<400>    35
gagctcagtc agtcacggag accacattga ggaca                                  35

<210>    36
<211>    35
<212>    DNA
<213>    Artificial Sequence

<220>
<223>    0049-2 for

<400>    36
tgactgactg agctcccaaa atgcgcaaca tcggc                                  35

<210>    37
<211>    36
<212>    DNA
<213>    Artificial Sequence

<220>
<223>    0049-2 rev

<400>    37
ggccagtgcc aagctttacc gatgtactgc acggag                                 36

<210>    38
<211>    36

<212>    DNA
<213>    Artificial Sequence

<220>
<223>    0463-1 for


<400>    38
gcaggcatgc aagcttgcac cagaaggtgg gctaaa                                36


<210>    39
<211>    35
<212>    DNA
<213>    Artificial Sequence

<220>
<223>    0463-1 rev


<400>    39
gagctcagtc agtcatgccg atagctctgc atctc                                 35


<210>    40
<211>    35
<212>    DNA
<213>    Artificial Sequence

<220>
<223>    0463-2 for


<400>    40
tgactgactg agctcgtatg catcgcagct aaccg                                 35


<210>    41
<211>    36
<212>    DNA
<213>    Artificial Sequence

<220>
<223>    0463-2 rev


<400>    41
ggccagtgcc aagctttacc tgcagcattg tgcgca                                36


<210>    42
<211>    36
<212>    DNA
<213>    Artificial Sequence

<220>
<223>    2619-1 for


<400>    42
gcaggcatgc aagcttgtct tcctttaggt ccgctg                                36

<210>    43
<211>    35
<212>    DNA
<213>    Artificial Sequence

<220>
<223>    2619-1 rev


<400>    43
gagctcagtc agtcaccggt gacaacctgc atcaa                                35


<210>    44
<211>    35
<212>    DNA
<213>    Artificial Sequence

<220>
<223>    2619-2 for


<400>    44
tgactgactg agctcggtgc cggatttgag tggaa                                35


<210>    45
<211>    36
<212>    DNA
<213>    Artificial Sequence

<220>
<223>    2619-2 rev


<400>    45
ggccagtgcc aagcttttgc ccagctttag cgctgt                               36


<210>    46
<211>    20
<212>    DNA
<213>    Artificial Sequence

<220>
<223>    0049for


<400>    46
gtgatgctgg taaacctgtg                                                 20


<210>    47
<211>    19
<212>    DNA
<213>    Artificial Sequence

<220>
<223>    0049rev


<400>    47
ttgcgtttgg gaaatacgg                                                  19

<210>    48
<211>    20
<212>    DNA
<213>    Artificial Sequence

<220>
<223>    0463for


<400>    48
gcaccagaag gtgggctaaa                                                    20


<210>    49
<211>    20
<212>    DNA
<213>    Artificial Sequence

<220>
<223>    0463rev


<400>    49
tacctgcagc attgtgcgca                                                    20


<210>    50
<211>    20
<212>    DNA
<213>    Artificial Sequence

<220>
<223>    2619for


<400>    50
gtcttccttt aggtccgctg                                                    20


<210>    51
<211>    20
<212>    DNA
<213>    Artificial Sequence

<220>
<223>    2619rev


<400>    51
ttgcccagct ttagcgctgt                                                    20


<210>    52
<211>    46
<212>    DNA
<213>    Artificial Sequence

<220>
<223>    MD-616

<400>    52
aaagtgtaaa gcctgggaac aacaagaccc atcatagttt gccccc                    46


<210>    53
<211>    36
<212>    DNA
<213>    Artificial Sequence

<220>
<223>    MD-618


<400>    53
gttcttctaa tcagaattgg ttaattggtt gtaaca                               36


<210>    54
<211>    40
<212>    DNA
<213>    Artificial Sequence

<220>
<223>    MD-615


<400>    54
gcgtaatagc gaagaggggc gtttttccat aggctccgcc                           40


<210>    55
<211>    40
<212>    DNA
<213>    Artificial Sequence

<220>
<223>    MD-617


<400>    55
gttcaatcat aacacccctt gtattactgt ttatgtaagc                           40


<210>    56
<211>    31
<212>    DNA
<213>    Artificial Sequence

<220>
<223>    MD-619


<400>    56
gggtgttatg attgaacaag atggattgca c                                    31


<210>    57
<211>    39
<212>    DNA
<213>    Artificial Sequence

<220>
<223>    MD-620

<400>    57
attctgatta gaagaactcg tcaagaaggc gatagaagg                              39


<210>    58
<211>    17
<212>    DNA
<213>    Artificial Sequence

<220>
<223>    LacZa-NR


<400>    58
cctcttcgct attacgc                                                      17


<210>    59
<211>    21
<212>    DNA
<213>    Artificial Sequence

<220>
<223>    MD-404


<400>    59
cccaggcttt acactttatg c                                                 21


<210>    60
<211>    47
<212>    DNA
<213>    Artificial Sequence

<220>
<223>    MD-627


<400>    60
gccaccgcgg tggagctcat ttagcggatg attctcgttc aacttcg                     47


<210>    61
<211>    32
<212>    DNA
<213>    Artificial Sequence

<220>
<223>    MD-628


<400>    61
ttttatttgc aaaaacggcc gaaaccatcc ct                                     32


<210>    62
<211>    40
<212>    DNA
<213>    Artificial Sequence

<220>
<223>    MD-629


<400>    62
ccgtttttgc aaataaaacg aaaggctcag tcgaaagact                              40


<210>    63
<211>    43
<212>    DNA
<213>    Artificial Sequence

<220>
<223>    MD-630


<400>    63
gaacaaaagc tggagctacc gtatctgtgg ggggatggct tgt                          43


<210>    64
<211>    5342
<212>    DNA
<213>    Artificial Sequence

<220>
<223>    pGST1


<400>    64
ttgagatcct ttttttctgc gcgtaatctg ctgcttgcaa acaaaaaaac caccgctacc        60

agcggtggtt tgtttgccgg atcaagagct accaactctt tttccgaagg taactggctt       120

cagcagagcg cagataccaa atactgttct tctagtgtag ccgtagttag gccaccactt       180

caagaactct gtagcaccgc ctacatacct cgctctgcta atcctgttac cagtggctgc       240

tgccagtggc gataagtcgt gtcttaccgg gttggactca agacgatagt taccggataa       300

ggcgcagcgg tcgggctgaa cggggggttc gtgcacacag cccagcttgg agcgaacgac       360

ctacaccgaa ctgagatacc tacagcgtga gctatgagaa agcgccacgc ttcccgaagg       420

gagaaaggcg gacaggtatc cggtaagcgg cagggtcgga acaggagagc gcacgaggga       480

gcttccaggg ggaaacgcct ggtatcttta tagtcctgtc gggtttcgcc acctctgact       540

tgagcgtcga tttttgtgat gctcgtcagg ggggcggagc ctatggaaaa acgccagcaa       600

tcgctattac gccagctggc gaaaggggga tgtgctgcaa ggcgattaag ttgggtaacg       660

ccagggtttt cccagtcacg acgttgtaaa acgacggcca gtgaattgta atacgactca       720

ctatagggcg aattgggtac cgggcccccc ctcgaggtcg acggtatcga taagcttgat       780

atcgaattcc tgcagcccgg gggatccact agttctagag cggccgccac cgcggtggag       840

ctcatttagc ggatgattct cgttcaactt cggccgaagc cacttcgtct gtcataatga       900

cagggatggt ttcggccgtt tttgcaaata aaacgaaagg ctcagtcgaa agactgggcc       960

tttcgtttta tctgttgttt gtcggtgaac gctctcctga gtaggacaaa tccgccggga 1020

gcggatttga acgttgcgaa gcaacggccc ggagggtggc gggcaggacg cccgccataa 1080

actgccaggc atcaaattaa gcagaaggcc atcctgacgg atggcctttt tgcgtttcta 1140

caaactcttc ctgtcgtcat atctacaagc catcccccca cagatacggt agctccagct 1200

tttgttccct ttagtgaggg ttaatttcga gcttggcgta atcatggtca tagctgtttc 1260

ctgtgtgaaa ttgttatccg ctcacaattc cacacaacat acgagccgga agcataaagt 1320

gtaaagcctg ggaacaacaa gacccatcat agtttgcccc cgcgacattg accataaatt 1380

catcgcacaa aatatcgaac ggggtttatg ccgcttttag tgggtgcgaa gaatagtctg 1440

ctcattaccc gcgaacaccg ccgcattcag atcacgctta gtagcgtccc catgagtagg 1500

cagaaccgcg tccaagtcca catcatccat aacgatcatg cacggggtgg aatccacacc 1560

cagacttgcc agcacctcat tagcgacacg ttgcgcagcg gccacgtcct tagccttatc 1620

cacgcaatct aggacgtact gcctaaccgc gaaatcagac tgaatcagtt tccaatcatc 1680

gggcttcacc aaagcaacag caacgcgggt tgattcgacc cgttccggtg cttccagacc 1740

ggcgagcttg tacagttctt cttccatttc acgacgtaca tcagcgtcta tgtaatcaat 1800

gcccaaagca cgcttagccc cacgtgacca ggacgaacgc aggtttttag aaccaacctc 1860

atactcacgc caccgagcca ccaaaacagc gtccatatcc tcgccggcgt cgctttgatc 1920

ggccaacata tccaacatct gaaacggcgt gtacgacccc ttagacgcgg ttttagtagc 1980

ggagccagtc agttcctgag acatgccctt agcgaggtag gttgccattt tcgcagcgtc 2040

tccaccccag gtagacacct gatcaagttt gaccccgtgc tcacgcagtg gcgcgtccat 2100

accggcctta accacaccag cagaccagcg ggaaaacatg gaatcctcaa acgccttgag 2160

ttcatcgtca gacagtggac gatccaagaa caacagcatg ttgcggtgca agtgccaacc 2220

gttcgcccaa gagtctgtga cctcatagtc actataggtg tgctccaccc cgtaccgtgc 2280

acgttctttc ttccactgag atgttttcac catcgaagag tacgcagtct taatacccgc 2340

ttcaacctgc gcaaatgact gtgagcggtt gtgtcgaaca gtgcccacaa acatcatgag 2400

cgcgccaccc gccgccaagt gattcttagt agcaatagcc agctcaatgc ggcgttcgcc 2460

catgacttcc aattcagcca gaggtgaccc ccagcgagag tgagagtttt gcagaccctc 2520

aaactgcgaa gcaccgttag acgaccagga caccgcaaca gcttcgtccc tgcgccacct 2580

atggcacccc gccagagcct tactattggt gatcttgtac atgacgtttt gcctacgcca 2640

cgccctagcg cgagtgacct tagaaccctc attgacctgc ggttccttag aggtgttcac 2700

ttctatttca gtgttaccta gacccgatgt tgtgcggggt tgcgcagtgc gagtttgtgc 2760

gggtgttgtg cccgttgtct tagctagtgc tatggttgtc aattgaaacc ccttcgggtt 2820

atgtggcccc cgtgcatatg agttggtagc tcgcacgggg gtttgtcttg tctaggaact 2880

attaattttt agtggtgttt ggtggccgcc tagcttggct atgcgtgcca gcttacccgt 2940

actcaatgtt aaagatttgc atcgacatgg gagggttacg tgtccgatac ctaggggggg 3000

tatccgcgac taggtgcccc ggtgctcact gtctgtaccg gcggggcaag ccccacaccc 3060

cgcatggaca gggtggctcc gccccctgca cccccagcaa tctgcatgta catgttttac 3120

acattagcac gacatgactg catgtgcatg cactgcatgc agactaggta aatatgagta 3180

tgtacgacta gtaacaggag cactgcacat aatgaatgag ttgcaggaca atgtttgcta 3240

cgcatgcgca tgacatatcg caggaaagct actagagtct taaagcatgg caaccaaggc 3300

acagctagaa cagcaactac aagaagctca acaggcacta caggcgcagc aagcgcaggc 3360

acaagccacc atcgaagcac tagaagcgca ggcaaaggct aagcccgtcg tggtcaccgc 3420

acgcgttcct ttggcactac gtgaggacat gaagcgcgca ggcatgcaga acggtgaaaa 3480

cctccaagag ttcatgatcg ccgcgtttac cgagcggcta gaaaagctca ccaccaccga 3540

caacgaggaa aacaatgtct aacccactag ttctctttgc ccaccgtgac ccggtaaatg 3600

acgtgacgtt cgagtgcatt gagcacgcca cctacgacac actttcacac gctaaagacc 3660

agatcaccgc ccaaatgcaa gccctagacg aagaagccgc cctactgccc taatgggtgt 3720

ttcatgggtg tttccctagt gtttcatggt gttttcacct aagctaggga attgcgcgag 3780

aagtctcgca aaaatcagca acccccggaa ccacacagtt cacgggggtt cttctatgcc 3840

agaaatcaga aaggggaacc agtgaacgac cccgaatatt ggatcacagc gcagcaggtc 3900

gccgcccgcg tagctctcac cccggccacc attaaaaagt gggcaaacga gggaaaaatc 3960

accgcataca agatcggcaa gtccgtccga ttcaaagcat cagacgtaga caagctaggg 4020

gggggggggc gctgaggtct gcctcgtgaa gaaggtgttg ctgactcata ccaggcctga 4080

atcgccccat catccagcca gaaagtgagg gagccacggt tgatgagagc tttgttgtag 4140

gtggaccagt tggtgatttt gaacttttgc tttgccacgg aacggtctgc gttgtcggga 4200

agatgcgtga tctgatcctt caactcagca aaagttcgat ttattcaaca aagccgccgt 4260

cccgtcaagt cagcgtaatg ctctgccagt gttacaacca attaaccaat tctgattaga 4320

agaactcgtc aagaaggcga tagaaggcga tgcgctgcga atcgggagcg gcgataccgt 4380

aaagcacgag gaagcggtca gcccattcgc cgccaagctc ttcagcaata tcacgggtag 4440

ccaacgctat gtcctgatag cggtccgcca cacccagccg gccacagtcg atgaatccag 4500

aaaagcggcc attttccacc atgatattcg gcaagcaggc atcgccatgg gtcacgacga 4560

gatcctcgcc gtcgggcatg ctcgccttga gcctggcgaa cagttcggct ggcgcgagcc 4620

cctgatgctc ttcgtccaga tcatcctgat cgacaagacc ggcttccatc cgagtacgtg 4680

ctcgctcgat gcgatgtttc gcttggtggt cgaatgggca ggtagccgga tcaagcgtat 4740

gcagccgccg cattgcatca gccatgatgg atactttctc ggcaggagca aggtgagatg 4800

acaggagatc ctgccccggc acttcgccca atagcagcca gtcccttccc gcttcagtga 4860

caacgtcgag cacagctgcg caaggaacgc ccgtcgtggc cagccacgat agccgcgctg 4920

cctcgtcttg cagttcattc agggcaccgg acaggtcggt cttgacaaaa agaaccgggc 4980

gcccctgcgc tgacagccgg aacacggcgg catcagagca gccgattgtc tgttgtgccc 5040

agtcatagcc gaatagcctc tccacccaag cggccggaga acctgcgtgc aatccatctt 5100

gttcaatcat aacacccctt gtattactgt ttatgtaagc agacagtttt attgttcatg 5160

atgatatatt tttatcttgt gcaatgtaac atcagagatt ttgagacaca acgtggcttt 5220

cccccccccc ccaaaaggat ctaggtgaag atcctttttg ataatctcat gaccaaaatc 5280

ccttaacgtg agttttcgtt ccactgagcg tcagaccccg tagaaaagat caaaggatct 5340

tc 5342

<210> 65
<211> 5693
<212> DNA
<213> Artificial Sequence

<220>
<223> gapA promoter-cat1-sucD-4hbD-cat2 cassette

<400> 65
gtcgacatga ttttgcatct gctgcgaaat ctttgtttcc ccgctaaagt tgaggacagg 60

ttgacacgga gttgactcga cgaattatcc aatgtgagta ggtttggtgc gtgagttgga 120

aaaattcgcc atactcgccc ttgggttctg tcagctcaag aattcttgag tgaccgatgc 180

tctgattgac ctaactgctt gacacattgc atttcctaca atctttagag gagacacaac 240

atgtctaaag gaatcaagaa tagccaattg aaaaaaaaga acgtcaaggc cagtaacgtt 300

gctgagaaga tcgaagagaa ggtggaaaag accgacaagg tcgttgagaa ggctgctgag 360

gtgaccgaaa agcgaattcg aaacttaaag ctccaggaaa aagttgtgac cgcagatgtc 420

gcagctgaca tgatcgagaa tggcatgatc gtcgcaatta gcggcttcac gccatccggg 480

tatccaaagg aggttccaaa agcccttact aagaaggtta atgcgctgga ggaggagttc 540

aaggtgacgc tgtataccgg ttctagcaca ggcgctgata ttgacggaga atgggcgaag 600

gcaggaataa tcgaacggcg tatcccatac cagaccaact ctgacatgag gaaaaaaata 660

aacgatggtt caatcaagta cgcagatatg cacctgagcc atatggctca atacattaac 720

tattctgtga ttcctaaggt tgacattgcc atcatcgagg cggtggccat taccgaggaa 780

ggggatatta ttcctagtac tggaatcggc aacacagcta cgtttgtcga gaatgcggat 840

aaggtaattg tggaaataaa cgaggctcag ccgcttgagt tggaaggcat ggcagatatc 900

tataccctga agaaccctcc acgtcgcgag cccatcccga tagtcaacgc aggcaaccgc 960
atagggacca cttacgtcac ctgtggctct gaaaaaatct gcgcgatcgt catgaccaac 1020
acccaagaca aaacccgccc actcaccgaa gtttctcctg tcagtcaggc aatctccgat 1080
aacctgattg gcttcctgaa caaagaagta gaggagggta aactcccaaa aaacctgctc 1140
cccatacagt caggtgtcgg ttcggttgct aacgccgtgc tagccggact ctgcgaatca 1200
aacttcaaaa atttgagctg ctacacagaa gtgatccagg attcgatgtt gaagcttatc 1260
aaatgtggaa aggcagatgt ggtgtccggc acctcgatct cgccatcacc ggaaatgctg 1320
cccgagttca taaaggacat aaattttttt cgcgagaaga tagtactgcg cccccaggaa 1380
atatctaata atccggaaat agctcgtcgt ataggagtga tctccataaa cactgctttg 1440
gaagtagaca tctacggtaa tgtgaactcc acgcatgtca tgggctccaa gatgatgaac 1500
ggcatcggcg gcagcggcga ctttgcccgc aacgcatacc tcaccatatt cactacggag 1560
tccatcgcga agaaggcgca catttcctct atcgttccta tggtttccca cgtggaccac 1620
accgagcatg acgtaatggt catcgttacc gaacaggggg ttgcggatct ccgcggtctt 1680
tcccctcggg aaaaggccgt ggcgataatt gagaattgcg tccacccgga ttacaaggat 1740
atgctcatgg agtacttcga ggaggcttgt aagtcctcag gtggcaacac cccacacaac 1800
cttgaaaaag ccctatcctg gcacactaag ttcataaaaa ctggctcgat gaagtaatta 1860
gaggagacac aacatggaga ttaaagagat ggtcagtctt gcgcgcaaag ctcagaagga 1920
gtatcaggcc acccataacc aagaagctgt ggacaacatc tgccgagctg cagcgaaggt 1980
tatttacgaa aatgcagcaa ttctggcccg cgaggcagtg gacgaaaccg gcatgggtgt 2040
ttacgagcac aaggtggcca agaatcaagg caagtccaaa ggtgtttggt acaacctgca 2100
taacaagaag tcgattggca tcctcaatat cgatgagcgt accggcatga tcgagatcgc 2160
aaaacctatc ggggttgtag gcgccgttac gccaaccacc aaccctatcg ttactccgat 2220
gagcaacatc atctttgctc ttaagacctg caacgccatc attatcgccc cacacccgcg 2280
ctccaaaaag tgctctgccc acgcagttcg gctgatcaaa gaggctatcg ctccgttcaa 2340
cgtgcccgaa ggtatggttc agatcatcga ggagcctagc atcgagaaga cgcaggaatt 2400
gatgggcgcc gtagacgtgg tcgttgctac cgggggcatg ggcatggtca agtctgccta 2460
ctcctcaggg aagccttctt tcggtgtcgg agccggcaat gttcaggtga tagtggacag 2520
caacatcgat ttcgaagcgg ctgcagaaaa gatcatcacc ggacgtgcct tcgacaacgg 2580
tatcatctgc tcaggcgaac agtccatcat ctacaacgag gctgacaagg aagcagtttt 2640
cacagcattc cgcaaccacg gtgcgtactt ttgcgacgag gccgagggag atcgggctcg 2700
tgcagcgatc ttcgaaaatg gagccatcgc gaaagatgtt gtgggccagt ccgttgcctt 2760
tattgccaag aaggcgaaca ttaatatccc cgagggtact cgtattctcg tggtcgaagc 2820

tcgcggagta ggcgccgaag atgtcatctg taaagaaaag atgtgtccag tcatgtgcgc 2880

cctctcctac aagcacttcg aagagggggt agagatcgca aggacgaacc tcgcaaacga 2940

aggcaatggc catacctgtg ctatccactc caacaaccaa gcacacatca tcttggcagg 3000

ctcggagctg accgtgtctc gcatcgtggt caacgcgcca agtgctacca cagcaggcgg 3060

tcacatccag aacggtcttg ccgtcaccaa tactctaggc tgcggctctt ggggtaacaa 3120

ctcgatctcc gaaaacttca cttataaaca cctgctcaac atttcacgca tcgccccgtt 3180

gaactccagc attcatatcc cagatgataa ggaaatctgg gaactctaat tagaggagac 3240

acaacatgca gcttttcaag ctcaagagcg tcacacatca ctttgatact tttgcagagt 3300

ttgccaagga attctgtctc ggtgaacgcg acttggtaat taccaacgag ttcatctacg 3360

aaccgtatat gaaggcatgc cagctgcctt gtcattttgt gatgcaggag aaatacggcc 3420

aaggcgagcc ttctgacgag atgatgaaca acatcctagc agatatccgt aatatccagt 3480

tcgaccgcgt gatcgggatc ggaggtggta cggttattga catctcaaaa ctctttgttc 3540

tgaagggatt aaatgatgtt ctcgacgcgt tcgatcgcaa gattcccctt atcaaagaga 3600

aagaactgat cattgtgccc accacctgcg gaaccggctc ggaggtgacg aacatttcca 3660

tcgccgagat caagtcccgg cacaccaaga tgggtttggc tgacgatgca attgttgctg 3720

accacgccat aatcatccct gaacttctga agagcttgcc cttccacttc tatgcatgct 3780

ccgcaatcga tgctcttatt catgccatcg agtcatacgt ttctccaaaa gcgtctccat 3840

actcccgtct gttcagtgag gcggcgtggg acattatcct ggaagttttc aagaaaatcg 3900

ccgaacacgg cccagagtac cgcttcgaga agctggggga aatgatcatg gccagcaact 3960

atgccggtat cgctttcggc aacgcaggcg ttggcgccgt ccacgctcta tcctacccgt 4020

tgggcggcaa ctatcacgtg ccgcatggag aagcaaacta tcagttcttc accgaggtct 4080

ttaaagtata ccaaaagaag aatccgttcg gctatattgt cgaactcaac tggaagctct 4140

ccaagattct gaactgccag ccagagtacg tgtacccgaa gctggatgaa ctgctcggtt 4200

gccttcttac caagaaacct ttgcacgaat acggcatgaa ggacgaagag gttcgtggct 4260

tcgcggaatc ggtcctgaag acccagcaac gcttgctcgc caacaactac gtcgaactta 4320

ctgtcgatga gatcgaaggt atctaccgac gtctctacta attagaggag acacaacatg 4380

aaggatgtac tggcggaata cgcctcccgc attgtttcgg cggaggaggc cgttaagcac 4440

atcaaaaacg gtgaacgggt agctttgtca cacgctgccg gcgtgcctca gagttgcgtt 4500

gacgcactgg tgcagcaggc cgaccttttc cagaatgtgg aaatctatca catgctgtgc 4560

ctcggtgagg gtaagtatat ggcgcctgag atggcccctc acttccgcca catcaccaac 4620

tttgtcggtg gtaactcccg taaggcggtc gaagaaaacc gggccgattt cattccggta 4680

ttcttttacg aggtgccaag catgattcgc aaagacatcc tccacattga tgtcgccatc 4740

gttcagcttt caatgcctga cgaaaatggt tactgttcct ttggagtatc ttgcgattac 4800

tccaagccgg cagcagagag cgctcacctg gttatcggag aaatcaaccg tcaaatgcca 4860

tacgtacacg gcgacaactt gattcatatc tccaagttgg attacatcgt gatggcagac 4920

taccccatct actctcttgc aaagcccaag atcggggaag tcgaggaagc tatcgggagg 4980

aattgtgccg agcttattga agatggtgcc actctccagc tgggaatcgg cgcgattcct 5040

gatgcggccc tgttatttct caaggacaaa aaggatctgg gcatccatac cgaaatgttc 5100

tccgatggtg ttgtcgaatt ggttcgctcc ggcgttatca caggcaagaa aaagactctt 5160

caccccggaa agatggtcgc aaccttcctg atgggaagcg aggacgtgta tcatttcatc 5220

gataaaaacc ccgatgtaga actgtatcca gtagattacg tgaatgaccc gcgtgtgatc 5280

gcccaaaacg acaatatggt ctcgattaac agctgcatcg aaatcgacct tatgggacag 5340

gtcgtgtccg agtgcatcgg ctcaaagcaa ttcagcggca ccggcggcca agttgactac 5400

gtgcgtggcg cagcatggtc taaaaacggc aaatcgatca tggcaatccc gtccactgca 5460

aaaaacggta cggcatctcg aattgtacct atcatcgcgg agggcgctgc tgtcaccacc 5520

ctgcgcaacg aggtcgatta cgttgtaacc gagtacggta tcgctcagct caagggcaag 5580

agcctgcgcc agcgcgcaga ggctttgatc gcgatagccc accccgactt ccgtgaggaa 5640

ctaacgaaac atctccgcaa gcgattcgga taagcggccg cagatctgga tcc 5693

<210> 66
<211> 44
<212> DNA
<213> Artificial Sequence

<220>
<223> 3G up

<400> 66
gatatcgaat tcctgcagat gattttgcat ctgctgcgaa atct 44

<210> 67
<211> 47
<212> DNA
<213> Artificial Sequence

<220>
<223> 3G down

<400> 67
gcggtggcgg ccgctctaga ttagtagaga cgtcggtaga taccttc 47

<210> 68
<211> 48

<212>    DNA
<213>    Artificial Sequence

<220>
<223>    ncgl0049_up F


<400>    68
catgattacg ccaagcttca atgcctttga tttgaactaa gcacagga                    48


<210>    69
<211>    43
<212>    DNA
<213>    Artificial Sequence

<220>
<223>    ncgl0049_up R


<400>    69
tgcaaaatca ttagagctca aaaccgcggc gtaaaatatt aga                         43


<210>    70
<211>    31
<212>    DNA
<213>    Artificial Sequence

<220>
<223>    ncgl 0049_down F


<400>    70
tacggtgcta gcatctgccc ctttacaaat c                                      31


<210>    71
<211>    48
<212>    DNA
<213>    Artificial Sequence

<220>
<223>    ncgl0049_down R


<400>    71
acgacggcca gtgaattcgg atcagcccta aatacaacta tgagacag                    48


<210>    72
<211>    42
<212>    DNA
<213>    Artificial Sequence

<220>
<223>    G3G F


<400>    72
tgagctctaa tgattttgca tctgctgcga aatctttgtt tc                          42

<210>    73
<211>    32
<212>    DNA
<213>    Artificial Sequence

<220>
<223>    G3G R


<400>    73
cagatgctag caccgtatct gtggggggat gg                                    32


<210>    74
<211>    24
<212>    DNA
<213>    Artificial Sequence

<220>
<223>    cat1-seq1F


<400>    74
agcccttact aagaaggtta atgc                                             24


<210>    75
<211>    22
<212>    DNA
<213>    Artificial Sequence

<220>
<223>    cat1-seq2R


<400>    75
cgtacttgat tgaaccatcg tt                                               22


<210>    76
<211>    22
<212>    DNA
<213>    Artificial Sequence

<220>
<223>    sucD-seq1F


<400>    76
caatgttcag gtgatagtgg ac                                               22


<210>    77
<211>    20
<212>    DNA
<213>    Artificial Sequence

<220>
<223>    sucD-seq2R


<400>    77
cacaacatct ttcgcgatgg                                                  20

<210>    78
<211>    21
<212>    DNA
<213>    Artificial Sequence

<220>
<223>    4hbD-seq1F


<400>    78
ctgaccacgc cataatcatc c                                                21


<210>    79
<211>    20
<212>    DNA
<213>    Artificial Sequence

<220>
<223>    4hbD-seq2R


<400>    79
cgataccggc atagttgctg                                                  20


<210>    80
<211>    5567
<212>    DNA
<213>    Artificial Sequence

<220>
<223>    pGSX1


<400>    80
ttgagatcct ttttttctgc gcgtaatctg ctgcttgcaa acaaaaaaac caccgctacc      60

agcggtggtt tgtttgccgg atcaagagct accaactctt tttccgaagg taactggctt     120

cagcagagcg cagataccaa atactgttct tctagtgtag ccgtagttag gccaccactt     180

caagaactct gtagcaccgc ctacatacct cgctctgcta atcctgttac cagtggctgc     240

tgccagtggc gataagtcgt gtcttaccgg gttggactca agacgatagt taccggataa     300

ggcgcagcgg tcgggctgaa cggggggttc gtgcacacag cccagcttgg agcgaacgac     360

ctacaccgaa ctgagatacc tacagcgtga gctatgagaa agcgccacgc ttcccgaagg     420

gagaaaggcg gacaggtatc cggtaagcgg cagggtcgga acaggagagc gcacgaggga     480

gcttccaggg ggaaacgcct ggtatcttta tagtcctgtc gggtttcgcc acctctgact     540

tgagcgtcga tttttgtgat gctcgtcagg ggggcggagc ctatggaaaa acgccagcaa     600

tcgctattac gccagctggc gaaaggggga tgtgctgcaa ggcgattaag ttgggtaacg     660

ccagggtttt cccagtcacg acgttgtaaa acgacggcca gtgaattgta atacgactca     720

ctatagggcg aattgggtac catgattttg catctgctgc gaaatctttg tttccccgct     780

aaagttgagg acaggttgac acggagttga ctcgacgaat tatccaatgt gagtaggttt 840

ggtgcgtgag ttggaaaaat tcgccatact cgcccttggg ttctgtcagc tcaagaattc 900

ttgagtgacc gatgctctga ttgacctaac tgcttgacac attgcatttc ctacaatctt 960

tagaggagac acaacctcga ggtcgacggt atcgataagc ttgatatcga attcctgcag 1020

cccgggggat ccactagttc tagagcggcc gccaccgcgg tggagctcat ttagcggatg 1080

attctcgttc aacttcggcc gaagccactt cgtctgtcat aatgacaggg atggtttcgg 1140

ccgtttttgc aaataaaacg aaaggctcag tcgaaagact gggcctttcg ttttatctgt 1200

tgtttgtcgg tgaacgctct cctgagtagg acaaatccgc cgggagcgga tttgaacgtt 1260

gcgaagcaac ggcccggagg gtggcgggca ggacgcccgc cataaactgc caggcatcaa 1320

attaagcaga aggccatcct gacggatggc ctttttgcgt ttctacaaac tcttcctgtc 1380

gtcatatcta caagccatcc ccccacagat acggtagctc cagcttttgt tccctttagt 1440

gagggttaat ttcgagcttg gcgtaatcat ggtcatagct gtttcctgtg tgaaattgtt 1500

atccgctcac aattccacac aacatacgag ccggaagcat aaagtgtaaa gcctgggaac 1560

aacaagaccc atcatagttt gcccccgcga cattgaccat aaattcatcg cacaaaatat 1620

cgaacggggt ttatgccgct tttagtgggt gcgaagaata gtctgctcat tacccgcgaa 1680

caccgccgca ttcagatcac gcttagtagc gtccccatga gtaggcagaa ccgcgtccaa 1740

gtccacatca tccataacga tcatgcacgg ggtggaatcc acacccagac ttgccagcac 1800

ctcattagcg acacgttgcg cagcggccac gtccttagcc ttatccacgc aatctaggac 1860

gtactgccta accgcgaaat cagactgaat cagtttccaa tcatcgggct tcaccaaagc 1920

aacagcaacg cgggttgatt cgacccgttc cggtgcttcc agaccggcga gcttgtacag 1980

ttcttcttcc atttcacgac gtacatcagc gtctatgtaa tcaatgccca aagcacgctt 2040

agccccacgt gaccaggacg aacgcaggtt tttagaacca acctcatact cacgccaccg 2100

agccaccaaa acagcgtcca tatcctcgcc ggcgtcgctt tgatcggcca acatatccaa 2160

catctgaaac ggcgtgtacg accccttaga cgcggtttta gtagcggagc cagtcagttc 2220

ctgagacatg cccttagcga ggtaggttgc cattttcgca gcgtctccac cccaggtaga 2280

cacctgatca agtttgaccc cgtgctcacg cagtggcgcg tccataccgg ccttaaccac 2340

accagcagac cagcgggaaa acatggaatc ctcaaacgcc ttgagttcat cgtcagacag 2400

tggacgatcc aagaacaaca gcatgttgcg gtgcaagtgc caaccgttcg cccaagagtc 2460

tgtgacctca tagtcactat aggtgtgctc caccccgtac cgtgcacgtt ctttcttcca 2520

ctgagatgtt ttcaccatcg aagagtacgc agtcttaata cccgcttcaa cctgcgcaaa 2580

tgactgtgag cggttgtgtc gaacagtgcc cacaaacatc atgagcgcgc cacccgccgc 2640

caagtgattc ttagtagcaa tagccagctc aatgcggcgt tcgcccatga cttccaattc 2700

agccagaggt gacccccagc gagagtgaga gttttgcaga ccctcaaact gcgaagcacc 2760

gttagacgac caggacaccg caacagcttc gtccctgcgc cacctatggc accccgccag 2820

agccttacta ttggtgatct tgtacatgac gttttgccta cgccacgccc tagcgcgagt 2880

gaccttagaa ccctcattga cctgcggttc cttagaggtg ttcacttcta tttcagtgtt 2940

acctagaccc gatgttgtgc ggggttgcgc agtgcgagtt tgtgcgggtg ttgtgcccgt 3000

tgtcttagct agtgctatgg ttgtcaattg aaaccccttc gggttatgtg gcccccgtgc 3060

atatgagttg gtagctcgca cgggggtttg tcttgtctag gaactattaa tttttagtgg 3120

tgtttggtgg ccgcctagct tggctatgcg tgccagctta cccgtactca atgttaaaga 3180

tttgcatcga catgggaggg ttacgtgtcc gatacctagg gggggtatcc gcgactaggt 3240

gccccggtgc tcactgtctg taccggcggg gcaagcccca caccccgcat ggacagggtg 3300

gctccgcccc ctgcaccccc agcaatctgc atgtacatgt tttacacatt agcacgacat 3360

gactgcatgt gcatgcactg catgcagact aggtaaatat gagtatgtac gactagtaac 3420

aggagcactg cacataatga atgagttgca ggacaatgtt tgctacgcat gcgcatgaca 3480

tatcgcagga aagctactag agtcttaaag catggcaacc aaggcacagc tagaacagca 3540

actacaagaa gctcaacagg cactacaggc gcagcaagcg caggcacaag ccaccatcga 3600

agcactagaa gcgcaggcaa aggctaagcc cgtcgtggtc accgcacgcg ttcctttggc 3660

actacgtgag gacatgaagc gcgcaggcat gcagaacggt gaaaacctcc aagagttcat 3720

gatcgccgcg tttaccgagc ggctagaaaa gctcaccacc accgacaacg aggaaaacaa 3780

tgtctaaccc actagttctc tttgcccacc gtgacccggt aaatgacgtg acgttcgagt 3840

gcattgagca cgccacctac gacacacttt cacacgctaa agaccagatc accgcccaaa 3900

tgcaagccct agacgaagaa gccgccctac tgccctaatg ggtgtttcat gggtgtttcc 3960

ctagtgtttc atggtgtttt cacctaagct agggaattgc gcgagaagtc tcgcaaaaat 4020

cagcaacccc cggaaccaca cagttcacgg gggttcttct atgccagaaa tcagaaaggg 4080

gaaccagtga acgaccccga atattggatc acagcgcagc aggtcgccgc ccgcgtagct 4140

ctcaccccgg ccaccattaa aaagtgggca aacgagggaa aaatcaccgc atacaagatc 4200

ggcaagtccg tccgattcaa agcatcagac gtagacaagc tagggggggg ggggcgctga 4260

ggtctgcctc gtgaagaagg tgttgctgac tcataccagg cctgaatcgc cccatcatcc 4320

agccagaaag tgagggagcc acggttgatg agagctttgt tgtaggtgga ccagttggtg 4380

attttgaact tttgctttgc cacggaacgg tctgcgttgt cgggaagatg cgtgatctga 4440

tccttcaact cagcaaaagt tcgatttatt caacaaagcc gccgtcccgt caagtcagcg 4500

taatgctctg ccagtgttac aaccaattaa ccaattctga ttagaagaac tcgtcaagaa 4560

ggcgatagaa ggcgatgcgc tgcgaatcgg gagcggcgat accgtaaagc acgaggaagc 4620

ggtcagccca ttcgccgcca agctcttcag caatatcacg ggtagccaac gctatgtcct 4680

gatagcggtc cgccacaccc agccggccac agtcgatgaa tccagaaaag cggccatttt 4740

ccaccatgat attcggcaag caggcatcgc catgggtcac gacgagatcc tcgccgtcgg 4800

gcatgctcgc cttgagcctg gcgaacagtt cggctggcgc gagcccctga tgctcttcgt 4860

ccagatcatc ctgatcgaca agaccggctt ccatccgagt acgtgctcgc tcgatgcgat 4920

gtttcgcttg gtggtcgaat gggcaggtag ccggatcaag cgtatgcagc cgccgcattg 4980

catcagccat gatggatact ttctcggcag gagcaaggtg agatgacagg agatcctgcc 5040

ccggcacttc gcccaatagc agccagtccc ttcccgcttc agtgacaacg tcgagcacag 5100

ctgcgcaagg aacgcccgtc gtggccagcc acgatagccg cgctgcctcg tcttgcagtt 5160

cattcagggc accggacagg tcggtcttga caaaaagaac cgggcgcccc tgcgctgaca 5220

gccggaacac ggcggcatca gagcagccga ttgtctgttg tgcccagtca tagccgaata 5280

gcctctccac ccaagcggcc ggagaacctg cgtgcaatcc atcttgttca atcataacac 5340

cccttgtatt actgtttatg taagcagaca gttttattgt tcatgatgat atatttttat 5400

cttgtgcaat gtaacatcag agattttgag acacaacgtg gctttccccc cccccccaaa 5460

aggatctagg tgaagatcct ttttgataat ctcatgacca aaatccctta acgtgagttt 5520

tcgttccact gagcgtcaga ccccgtagaa aagatcaaag gatcttc 5567

<210> 81
<211> 48
<212> DNA
<213> Artificial Sequence

<220>
<223> Primer MD-625

<400> 81
tagggcgaat tgggtaccat gattttgcat ctgctgcgaa atctttgt 48

<210> 82
<211> 55
<212> DNA
<213> Artificial Sequence

<220>
<223> Primer MD-626

<400> 82
gataccgtcg acctcgaggt tgtgtctcct ctaaagattg taggaaatgc aatgt 55

<210> 83

<211>    34
<212>    DNA
<213>    Artificial Sequence

<220>
<223>    Primer 1502-up


<400>    83
agaggagaca caaccatgac tgaagtagca accg                                    34


<210>    84
<211>    34
<212>    DNA
<213>    Artificial Sequence

<220>
<223>    Primer 1502-dn


<400>    84
tggcggccgc tctagttaca tgccaagaac tgcg                                    34


<210>    85
<211>    34
<212>    DNA
<213>    Artificial Sequence

<220>
<223>    Primer 1503-up


<400>    85
agaggagaca caacccggat catgacttcg gcaa                                    34


<210>    86
<211>    33
<212>    DNA
<213>    Artificial Sequence

<220>
<223>    Primer 1503-dn


<400>    86
tggcggccgc tctagttagc ccaccgatcc ttc                                     33


<210>    87
<211>    35
<212>    DNA
<213>    Artificial Sequence

<220>
<223>    Primer 0776-up


<400>    87
agaggagaca caaccatggt gaaaaaccga ttcaa                                   35

<210>    88
<211>    33
<212>    DNA
<213>    Artificial Sequence

<220>
<223>    Primer 0776-dn


<400>    88
tggcggccgc tctagctact gagctgcctt ctc                                    33


<210>    89
<211>    945
<212>    DNA
<213>    Corynebacterum glutamicum

<400>    89
atgaaagaaa ccgtcggtaa caagattgtc ctcattggcg caggagatgt tggagttgca      60

tacgcatacg cactgatcaa ccagggcatg gcagatcacc ttgcgatcat cgacatcgat     120

gaaaagaaac tcgaaggcaa cgtcatggac ttaaaccatg gtgttgtgtg ggccgattcc     180

cgcacccgcg tcaccaaggg cacctacgct gactgcgaag acgcagccat ggttgtcatt     240

tgtgccggcg cagcccaaaa gccaggcgag acccgcctcc agctggtgga caaaaacgtc     300

aagattatga aatccatcgt cggcgatgtc atggacagcg gattcgacgg catcttcctc     360

gtggcgtcca acccagtgga tatcctgacc tacgcagtgt ggaaattctc cggcttggaa     420

tggaaccgcg tgatcggctc cggaactgtc ctggactccg ctcgattccg ctacatgctg     480

ggcgaactct acgaagtggc accaagctcc gtccacgcct acatcatcgg cgaacacggc     540

gacactgaac ttccagtcct gtcctccgcg accatcgcag gcgtatcgct tagccgaatg     600

ctggacaaag acccagagct tgagggccgt ctagagaaaa ttttcgaaga cacccgcgac     660

gctgcctatc acattatcga cgccaagggc tccacttcct acggcatcgg catgggtctt     720

gctcgcatca cccgcgcaat cctgcagaac caagacgttg cagtcccagt ctctgcactg     780

ctccacggtg aatacggtga ggaagacatc tacatcggca ccccagctgt ggtgaaccgc     840

cgaggcatcc gccgcgttgt cgaactagaa atcaccgacc acgagatgga acgcttcaag     900

cattccgcaa ataccctgcg cgaaattcag aagcagttct tctaa                     945


<210>    90
<211>    4990
<212>    DNA
<213>    Artificial Sequence

<220>
<223>    pGSK+


<400>    90

ttgagatcct ttttttctgc gcgtaatctg ctgcttgcaa acaaaaaaac caccgctacc 60

agcggtggtt tgtttgccgg atcaagagct accaactctt tttccgaagg taactggctt 120

cagcagagcg cagataccaa atactgttct tctagtgtag ccgtagttag gccaccactt 180

caagaactct gtagcaccgc ctacatacct cgctctgcta atcctgttac cagtggctgc 240

tgccagtggc gataagtcgt gtcttaccgg gttggactca agacgatagt taccggataa 300

ggcgcagcgg tcgggctgaa cggggggttc gtgcacacag cccagcttgg agcgaacgac 360

ctacaccgaa ctgagatacc tacagcgtga gctatgagaa agcgccacgc ttcccgaagg 420

gagaaaggcg gacaggtatc cggtaagcgg cagggtcgga acaggagagc gcacgaggga 480

gcttccaggg ggaaacgcct ggtatcttta tagtcctgtc gggtttcgcc acctctgact 540

tgagcgtcga tttttgtgat gctcgtcagg ggggcggagc ctatggaaaa acgcccctct 600

tcgctattac gccagctggc gaaaggggga tgtgctgcaa ggcgattaag ttgggtaacg 660

ccagggtttt cccagtcacg acgttgtaaa acgacggcca gtgaattgta atacgactca 720

ctatagggcg aattgggtac cgggcccccc ctcgaggtcg acggtatcga taagcttgat 780

atcgaattcc tgcagcccgg gggatccact agttctagag cggccgccac cgcggtggag 840

ctccagcttt tgttcccttt agtgagggtt aatttcgagc ttggcgtaat catggtcata 900

gctgtttcct gtgtgaaatt gttatccgct cacaattcca cacaacatac gagccggaag 960

cataaagtgt aaagcctggg aacaacaaga cccatcatag tttgcccccg cgacattgac 1020

cataaattca tcgcacaaaa tatcgaacgg ggtttatgcc gcttttagtg ggtgcgaaga 1080

atagtctgct cattacccgc gaacaccgcc gcattcagat cacgcttagt agcgtcccca 1140

tgagtaggca gaaccgcgtc caagtccaca tcatccataa cgatcatgca cggggtggaa 1200

tccacaccca gacttgccag cacctcatta gcgacacgtt gcgcagcggc cacgtcctta 1260

gccttatcca cgcaatctag gacgtactgc ctaaccgcga aatcagactg aatcagtttc 1320

caatcatcgg gcttcaccaa agcaacagca acgcgggttg attcgacccg ttccggtgct 1380

tccagaccgg cgagcttgta cagttcttct tccatttcac gacgtacatc agcgtctatg 1440

taatcaatgc ccaaagcacg cttagcccca cgtgaccagg acgaacgcag gtttttagaa 1500

ccaacctcat actcacgcca ccgagccacc aaaacagcgt ccatatcctc gccggcgtcg 1560

ctttgatcgg ccaacatatc caacatctga aacggcgtgt acgacccctt agacgcggtt 1620

ttagtagcgg agccagtcag ttcctgagac atgcccttag cgaggtaggt tgccattttc 1680

gcagcgtctc caccccaggt agacacctga tcaagtttga ccccgtgctc acgcagtggc 1740

gcgtccatac cggccttaac cacaccagca gaccagcggg aaaacatgga atcctcaaac 1800

gccttgagtt catcgtcaga cagtggacga tccaagaaca acagcatgtt gcggtgcaag 1860

tgccaaccgt tcgcccaaga gtctgtgacc tcatagtcac tataggtgtg ctccaccccg 1920

taccgtgcac gttctttctt ccactgagat gttttcacca tcgaagagta cgcagtctta 1980

atacccgctt caacctgcgc aaatgactgt gagcggttgt gtcgaacagt gcccacaaac 2040

atcatgagcg cgccacccgc cgccaagtga ttcttagtag caatagccag ctcaatgcgg 2100

cgttcgccca tgacttccaa ttcagccaga ggtgaccccc agcgagagtg agagttttgc 2160

agaccctcaa actgcgaagc accgttagac gaccaggaca ccgcaacagc ttcgtccctg 2220

cgccacctat ggcaccccgc cagagcctta ctattggtga tcttgtacat gacgttttgc 2280

ctacgccacg ccctagcgcg agtgacctta gaaccctcat tgacctgcgg ttccttagag 2340

gtgttcactt ctatttcagt gttacctaga cccgatgttg tgcggggttg cgcagtgcga 2400

gtttgtgcgg gtgttgtgcc cgttgtctta gctagtgcta tggttgtcaa ttgaaacccc 2460

ttcgggttat gtggcccccg tgcatatgag ttggtagctc gcacgggggt ttgtcttgtc 2520

taggaactat taatttttag tggtgtttgg tggccgccta gcttggctat gcgtgccagc 2580

ttacccgtac tcaatgttaa agatttgcat cgacatggga gggttacgtg tccgatacct 2640

agggggggta tccgcgacta ggtgccccgg tgctcactgt ctgtaccggc ggggcaagcc 2700

ccacaccccg catggacagg gtggctccgc cccctgcacc cccagcaatc tgcatgtaca 2760

tgttttacac attagcacga catgactgca tgtgcatgca ctgcatgcag actaggtaaa 2820

tatgagtatg tacgactagt aacaggagca ctgcacataa tgaatgagtt gcaggacaat 2880

gtttgctacg catgcgcatg acatatcgca ggaaagctac tagagtctta aagcatggca 2940

accaaggcac agctagaaca gcaactacaa gaagctcaac aggcactaca ggcgcagcaa 3000

gcgcaggcac aagccaccat cgaagcacta gaagcgcagg caaaggctaa gcccgtcgtg 3060

gtcaccgcac gcgttccttt ggcactacgt gaggacatga agcgcgcagg catgcagaac 3120

ggtgaaaacc tccaagagtt catgatcgcc gcgtttaccg agcggctaga aaagctcacc 3180

accaccgaca acgaggaaaa caatgtctaa cccactagtt ctctttgccc accgtgaccc 3240

ggtaaatgac gtgacgttcg agtgcattga gcacgccacc tacgacacac tttcacacgc 3300

taaagaccag atcaccgccc aaatgcaagc cctagacgaa gaagccgccc tactgcccta 3360

atgggtgttt catgggtgtt tccctagtgt ttcatggtgt tttcacctaa gctagggaat 3420

tgcgcgagaa gtctcgcaaa aatcagcaac ccccggaacc acacagttca cgggggttct 3480

tctatgccag aaatcagaaa ggggaaccag tgaacgaccc cgaatattgg atcacagcgc 3540

agcaggtcgc cgcccgcgta gctctcaccc cggccaccat taaaaagtgg gcaaacgagg 3600

gaaaaatcac cgcatacaag atcggcaagt ccgtccgatt caaagcatca gacgtagaca 3660

agctaggggg gggggggcgc tgaggtctgc ctcgtgaaga aggtgttgct gactcatacc 3720

aggcctgaat cgccccatca tccagccaga aagtgaggga gccacggttg atgagagctt 3780

tgttgtaggt ggaccagttg gtgattttga acttttgctt tgccacggaa cggtctgcgt 3840

tgtcgggaag atgcgtgatc tgatccttca actcagcaaa agttcgattt attcaacaaa 3900

gccgccgtcc cgtcaagtca gcgtaatgct ctgccagtgt tacaaccaat taaccaattc 3960

tgattagaag aactcgtcaa gaaggcgata gaaggcgatg cgctgcgaat cgggagcggc 4020

gataccgtaa agcacgagga agcggtcagc ccattcgccg ccaagctctt cagcaatatc 4080

acgggtagcc aacgctatgt cctgatagcg gtccgccaca cccagccggc cacagtcgat 4140

gaatccagaa aagcggccat tttccaccat gatattcggc aagcaggcat cgccatgggt 4200

cacgacgaga tcctcgccgt cgggcatgct cgccttgagc ctggcgaaca gttcggctgg 4260

cgcgagcccc tgatgctctt cgtccagatc atcctgatcg acaagaccgg cttccatccg 4320

agtacgtgct cgctcgatgc gatgtttcgc ttggtggtcg aatgggcagg tagccggatc 4380

aagcgtatgc agccgccgca ttgcatcagc catgatggat actttctcgg caggagcaag 4440

gtgagatgac aggagatcct gccccggcac ttcgcccaat agcagccagt cccttcccgc 4500

ttcagtgaca acgtcgagca cagctgcgca aggaacgccc gtcgtggcca gccacgatag 4560

ccgcgctgcc tcgtcttgca gttcattcag ggcaccggac aggtcggtct tgacaaaaag 4620

aaccgggcgc ccctgcgctg acagccggaa cacggcggca tcagagcagc cgattgtctg 4680

ttgtgcccag tcatagccga atagcctctc cacccaagcg gccggagaac ctgcgtgcaa 4740

tccatcttgt tcaatcataa caccccttgt attactgttt atgtaagcag acagttttat 4800

tgttcatgat gatatatttt tatcttgtgc aatgtaacat cagagatttt gagacacaac 4860

gtggctttcc cccccccccc aaaaggatct aggtgaagat cctttttgat aatctcatga 4920

ccaaaatccc ttaacgtgag ttttcgttcc actgagcgtc agaccccgta gaaaagatca 4980

aaggatcttc 4990

## Description

### BACKGROUND

### 1. Field

The present disclosure relates to microorganisms having increased iron-regulated ABC transporter activity and methods of producing hydroxycarboxylic acid by using the microorganisms.

### 2. Description of the Related Art

Hydroxycarboxylic acid has a chemical structure including two functional groups, namely, a hydroxy group and a carboxy group, and is capable of being used in various chemical conversions. The hydroxy group and the carboxy group may each independently undergo a chemical reaction or may be transformed into a dimer, an oligomer, or a polymer material through a reaction between them. In the case of a β-hydroxycarboxylic acid, an α or β-unsaturated carboxylic acid may be obtained from a dehydration reaction between the hydroxy group and a nearby hydrogen atom.

The hydroxycarboxylic acid, such as a 4-hydroxybutyric acid or a 3-hydroxypropionic acid, is a useful material that may be used as an intermediate or monomer in the synthesis of a polymer or a pharmaceutical. The 4-hydroxybutyric acid may be used as a biologically important precursor for a C4 compound, such as 1,4-butanediol or γ-butyrolactone, and may also be used as a monomer of polyhydroxy butyrate.

It has been reported that the hydroxycarboxylic acid may be produced by a petrochemical process or a biological method. However, considering the increase in the manufacturing costs due to the increase in the oil price, a method of producing a highly concentrated hydroxycarboxylic acid is needed as a biological method to supplement and supplant a chemical process.

### SUMMARY

Provided is a recombinant microorganism having increased iron-regulated ABC transporter activity and increased hydroxycarboxylic acid production as compared to a parent cell of the microorganism. In one embodiment, the recombinant microorganism has increased expression of a polynucleotide (gene) encoding an iron-regulated ABC transporter, such as a recombinant microorganism having an exogenous gene encoding an iron regulated ABC transporter.

Also provided is a method of producing a hydroxycarboxylic acid using the microorganisms. The method comprises culturing the recombinant microorganism with a carbon source, and recovering the hydroxycarboxylic acid from the culture.

Also provided is a method of providing a recombinant microorganism with improved hydroxycarboxylic acid production, or improving the hydroxycarboxylic acid production of a microorganism. The method comprises introducing an exogenous gene encoding an iron-regulated ABC transporter into a hydroxycarboxylic acid producing microorganism, to provide a recombinant microorganism with improved hydroxycarboxylic acid production.

Additional aspects will be set forth in part in the description which follows and, in part, will be apparent from the description, or may be learned by practice of the presented embodiments. The invention will be described in more detail by way of examples hereinafter using advantageous embodiments and with reference to the accompanying drawings. The described embodiments are only possible configurations in which individual features may, however, as described above, be implemented independently of each other or may be omitted.

### BRIEF DESCRIPTION OF THE DRAWINGS

These and/or other aspects will become apparent and more readily appreciated from the following description of the embodiments, taken in conjunction with the accompanying drawings in which:
FIG. 1 is a cleavage map of a pGSK+ vector;
FIG. 2 is a cleavage map of a pGST1 vector;
FIG. 3 is a cleavage map of a pG3G vector;
FIG. 4 is a cleavage map of a pGS-EX1 vector;
FIG. 5 is a cleavage map of a pEX1-1502 vector;
FIG. 6 is a cleavage map of a pEX1-1503 vector; and
FIG. 7 is a cleavage map of a pEX1-0776 vector.

### DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

As used herein, the term "increase in activity" or "increased activity" may refer to a detectable increase in the activity of a cell, a protein, or an enzyme. In other words, the term "increase in activity" or "increased activity" of a cell, a protein, or an enzyme may refer to a higher activity level of a modified (i.e., genetically engineered) cell, protein, or enzyme than that of a comparable cell of the same type, a protein, or an enzyme without the given genetic modification (e.g., an original or wide-type cell, protein, or enzyme). For example, the activity of the modified or genetically engineered cell, protein, or enzyme may be increased by about 5% or more, about 10% or more, about 15% or more, about 20% or more, about 30% or more, about 50% or more, about 60% or more, about 70% or more, or about 100% or more than the activity of an originally unengineered cell, protein, or enzyme, such as a wild-type cell, protein, or an enzyme. As used herein, the term "unengineered" refers to a cell, protein or enzyme, or gene, etc. that does not have a given specific modification, encompassing but not necessarily limited to an absolutely unmodified (e.g., "wild-type) cell, protein, enzyme, gene, etc. as well as a cell protein, enzyme, gene, etc. that is engineered or recombinant (e.g., not naturally occurring) missing a particular referenced modification. Thus, as compared to a cell comprising a particular modification, an unengineered cell does not contain that particular modification, but may contain other modifications. The activity of particular protein or enzyme in cells may be about 5% or more, about 10% or more, about 15% or more, about 20% or more, about 30% or more, about 50% or more, about 60% or more, about 70% or more, or about 100% or more increased than the activity of a parent cell, e.g., the same protein or enzyme in the originally unengineered cells. The cells with increased activity of a protein or an enzyme may be identified by using methods known in the art, and these cells may have a genetic modification that increases the activity of at least one enzyme or polypeptide as compared with the activity of the cell without having the given genetic modification.

Meanwhile, as used herein, the term "decreased activity" or "decrease in activity" of a cell may refer to an activity level of an enzyme or a polypeptide in a cell that is lower than that measured in a parent cell (e.g., a genetically unengineered cell). In other words, the term "decreased activity" or "decrease in activity" of an enzyme or a polypeptide may refer to an activity level of an isolated enzyme or polypeptide that is lower than that measured in the unengineered (e.g., original or wild-type) enzyme or polypeptide. The "decreased activity" or "decrease in activity" of a cell may also refer to an activity level at which an enzyme or a polypeptide shows no activity. For example, the activity of a modified (i.e., genetically engineered) cell or enzyme in terms of enzymatic conversion of a product from a substrate may be decreased by about 20% or more, about 30% or more, about 40% or more, about 50% or more, about 55% or more, about 60% or more, about 70% or more, about 75% or more, about 80% or more, about 85% or more, about 90% or more, about 95% or more, or about 100% as compared to the activity of an unengineered cell or enzyme, such as a parent cell or a "wild-type" cell or enzyme, in terms of enzymatic conversion. The decreased activity of an enzyme or a cell may be identified by using methods known in the art. The decreased activity includes the case in which the enzyme is inactive or has reduced activity even when the enzyme is expressed as compared with a cell having an unengineered gene, i.e., a parent cell or a wild-type cell, or the case in which the gene encoding the enzyme is not expressed or has reduced expression as compared with the genetically unengineered gene even when the enzyme is expressed. The cells with decreased activity may have a genetic modification that decreases the activity of at least one enzyme or polypeptide as compared with the activity of the cell without having the given genetic modification.

An unengineered cell includes a parent cell. As used herein, the term "parent cell" denotes a cell in a state immediately prior to a particular genetic modification, for example, a cell that serves as a starting material for producing a cell having a genetic modification that increases the activity of a given protein. A parent cell, thus, is a cell without a particular referenced genetic modification, but with the other traits of the genetically modified cell and of the same cell type as the genetically modified cell. Although the "parent cell" does not have the specific referenced genetic modification, the parent cell may be engineered in other respects and, thus, might not be a "wild-type" cell.

As used herein, the term "genetic modification" may refer to introduction of a polynucleotide encoding a polypeptide (i.e., an increase in a copy number of the gene), or substitution, addition, insertion, or deletion of at least one nucleotide with a genetic material of a parent cell, or chemical mutation of a genetic material of a parent cell. In other words, genetic modification may include cases associated with a coding region of a polypeptide or a functional fragment thereof of a polypeptide that is heterologous, homologous, or both heterologous and homologous with a referenced species. Genetic modification may also refer to modification in non-coding regulatory regions that are capable of modifying expression of a gene or an operon, wherein the non-coding regulatory regions include a 5'-non coding sequence and/or a 3'-non coding sequence.

As used herein, the term "disruption" of a gene refers to a genetic modification that decreases expression of the referenced gene. The disruption of the gene may include a case where the referenced gene shows no activity in gene expression thereof (hereinafter, the case is referred to as "inactivation" of the gene), or a case where the referenced gene has a reduced expression level, even when the gene is expressed (hereinafter, the case is referred to as "attenuation" of the gene). In this regard, the inactivation of the gene may refer to not only a case where a functional product of the gene is not expressed, but also a case where the gene is expressed without expressing a functional product, and the attenuation of the gene may refer to a case where expression of the gene is reduced or the activity level of the gene product is reduced. The functional product of the gene may be the gene product of a parent cell or a wild-type cell including a gene with biochemical or physiological function (i.e., enzymatic activity). Thus, the disruption of the gene includes functional disruption of the gene.

The disruption of the gene may be achieved by genetic manipulation including homologous recombination, directed mutagenesis, or molecular evolution. When a cell includes multiple copies of the same gene or two or more paralogs of the gene, at least one of the genes may be disrupted. For example, such genetic modification may be performed by transforming a cell with a vector including a partial sequence of the gene, culturing the transformed cell, disrupting the gene by homologous recombination between the sequence and the endogenous gene, and then by screening the cells of the homologous recombination using a selectable marker.

As used herein, the term "gene" may refer to a nucleic acid fragment expressing a specific protein. The gene may include a regulatory sequence, such as a 5'-non-coding sequence and/or a 3'-non-coding sequence.

As used herein, the term "sequence identity" of nucleic acid or polypeptide may refer to the extent of identity between bases or amino acid residues of sequences after aligning the sequences such that they maximally match in certain comparative regions. The sequence identity is a value calculated by optimally aligning two sequences at certain comparative regions, wherein portions of the sequences at the certain comparative regions may be added or deleted compared to reference sequences. A percentage of sequence identity may be calculated by, for example, comparing two optimally aligned sequences in the entire comparative region, determining the number of locations in which the same amino acids or nucleic acids appear to obtain the number of matched locations, dividing the number of the matched locations by the total number of locations in the comparative region (that is, the size of the range), and multiplying 100 thereto to calculate the percentage of the sequence identity. The percentage of the sequence identity may be calculated by using a known sequence comparison program, and examples of such program include BLASTN (NCBI), CLC Main Workbench (CLC bio), and MegAlignTM (DNASTAR Inc).

Various levels of sequence identity may be used to identify various types of polypeptides or polynucleotides having the same or similar functions. For example, a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100% may be used as a standard.

As used herein, the term "exogenous" gene may refer to a gene or nucleic acid (e.g., encoding a polypeptide or enzyme) that is introduced into a host cell. The exogenous gene may be introduced into a genetic material of the host cell, e.g., a chromosome, or may be introduced as a non-chromosomal genetic material, e.g., a plasmid. In regard to the expression of the encoding nucleic acid, the term "exogenous" indicates a practice of introducing the encoding nucleic acid into an individual in an expressible form. In regard to biosynthetic activity, the term "exogenous" indicates the activity introduced into a host, parent cell. A source of the exogenous gene may be, for example, a homologous or heterologous source. The term "endogenous" denotes a gene (or gene product or biological activity) that is derived from a source that that is of the same genetic origin (e.g., same species) as the host cell. The term "heterologous" denotes a gene (or gene product or activity) that is derived from a different organism, for example, derived from a different cell type or a different species from the recipient. Thus, an exogenous gene or nucleic acid (or gene product or activity) may be heterologous or homologous to a host cell.

In addition, as used herein, the term "genetic engineering" or "genetically engineered" may refer to a practice of performing genetic modification with respect to one or more cells, or a cell resulted from genetic modification. The term "genetically engineered" may be used interchangeably with the term "recombinant".

According to an aspect of the present invention, provided is a recombinant microorganism having increased iron-regulated ABC transporter activity and increased hydroxycarboxylic acid production as compared to a parent cell of the recombinant microorganism.

The iron-regulated ABC transporter may be a member of ATP-binding cassette transporter ("ABC transporter") family. For example, the iron-regulated ABC transporter may be a membrane protein for transporting materials through a membrane, or the transporter may be a catalyst for the reaction:

ATP + Fe³⁺ (in extracellular space) + H₂O -> ADP + Fe³⁺ (in cytosol) + phosphate.

The transporter may include SufB (e.g., Ncgl1502), SufD (e.g., DNc11503), a member of the ABC-type cobalamin/Fe3+-siderophore transport system, or a combination thereof. Members of the ABC-type cobalamin/Fe3+-siderophore transport system may include ABC-type cobalamin/Fe3+-siderophore transport system, permease (e.g., Ncgl0777 and Ncgl0778), ATPase (Ncgl0779), and periplasmic component (e.g., Ncgl0776). For example, the ABC-type cobalamin/Fe3+-siderophore transport system may include ABC-type cobalamin/Fe3+-siderophore transport system, periplasmic component (e.g., Ncgl0776).

Genes for the iron-regulated ABC transporters, sufB and sufD, may form a part of a suf operon, and participate in an iron metabolism, an assembly of a Fe-S cluster, or oxidative stress response.

The iron-regulated ABC transporter SufB may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to SEQ ID NO: 1. Examples of SufB include NCBI Reference Sequence: NP_600778.1, YP_225849.1, YP_007560762.1, NP_416198.2, YP_489945.1, or YP_006319280.1. The polynucleotide encoding the SufB may have a nucleotide sequence encoding an amino acid sequence having a sequence identity of 95% to the SEQ ID NO: 1 or a nucleotide sequence of SEQ ID NO: 2. The nucleotide sequence coding SufB may be, but is not limited to, Gene ID: 1019532, Gene ID: 62390447, Gene ID: 14794195, Gene ID: 945753, Gene ID: 12931288, or Gene ID: 12955518. The transporter may be derived from a microorganism such as bacteria, yeast, and bacteria, and in greater detail, the microorganism may be *Corynebacterium glutamicum, Escherichia coli, or Escherichia blattae.*

The iron-regulated ABC transporter SufD may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to SEQ ID NO: 3. Examples of SufD include, but are not limited to, NCBI Reference Sequence: NP_600779.1, YP_225848, NP_416196.1, or YP_489943.1. The polynucleotide encoding the SufD may have a nucleotide sequence encoding an amino acid sequence having a sequence identity of 95% to the SEQ ID NO: 3 or a nucleotide sequence of SEQ ID NO: 4. The nucleotide sequence coding SufD may be Gene ID: 1019533, Gene ID: 62390446, Gene ID: 944878, or Gene ID: 12931286. The transporter may be derived from a microorganism such as bacteria, yeast, and bacteria, and in greater detail, the microorganism may be *Corynebacterium glutamicum, Escherichia coli, or Escherichia blattae.*

The ABC-type cobalamin/Fe3+-siderophore transport system, periplasmic component may include an amino acid sequence having a sequence identity of 65% or more, for example, 70% or more, 80% or more, 85% or more, 90% or more, 91 % or more, 92% or more, 93% or more, 94% or more, 95% or more, 96% or more, 97% or more, 98% or more, 99% or more, or 100% to SEQ ID NO: 5. The polynucleotide encoding the transporter may have a nucleotide sequence encoding an amino acid sequence having a sequence identity of 95% or more to the SEQ ID NO: 5 or a nucleotide sequence of SEQ ID NO: 6. The nucleotide sequence may be, but is not limited to, NCgl0776. The transporter may be derived from a microorganism such as bacteria, yeast, and bacteria. More specifically, the microorganism may be *Corynebacterium glutamicum, Escherichia coli, or Escherichia blattae.*

The increased transporter activity in a cell may increase the production of the hydroxycarboxylic acid. The mode by which production is increased is not particularly limited, but may include increased production of the hydroxycarboxylic acid within the cell and/or increased amount or rate of extracellular secretion of the produced hydroxycarboxylic acid.

In the recombinant microorganism, the SufB, the SufD, and the ABC-type cobalamin/Fe3+-siderophore transport system, periplasmic component may have an amino acid sequence having a sequence identity of 95% or more to SEQ ID NOS: 1, 3, and 5, respectively.

The recombinant microorganism may be subjected to genetic modification to increase activity of the transporter as compared with the activity of the parent cell of the microorganism. The recombinant microorganism may be, for example, a recombinant microorganism in which expression of a polynucleotide encoding an iron-regulated ABC transporter is increased. In other words, the recombinant microorganism may be a recombinant microorganism that expresses or over-expresses the polynucleotide encoding the iron-regulated ABC transporter producing an increased amount of hydroxycarboxylic acid than the microorganism that does not express or over-express the polynucleotide encoding the iron-regulated ABC transporter cultured under the same condition.

In some embodiments, the recombinant microorganism may be a recombinant microorganism transformed with a polynucleotide encoding an iron-regulated ABC transporter that produces an increased amount of hydroxycarboxylic acid compared to a microorganism that is not transformed with the polynucleotide encoding the iron-regulated ABC transporter when cultured under the same conditions. In other words, the recombinant microorganism may include an exogenous gene encoding the transporter, wherein the exogenous gene may encode an amino acid having a sequence identity of 95% or more to each of SEQ ID NOS: 1, 3, and/or 5, respectively. For example, the exogenous gene may a nucleotide sequence having a sequence identity of 95% or more to each of SEQ ID NOS: 2, 4, and/or 6, respectively.

The recombinant microorganism may be genetically engineered to have increased expression of the gene that encodes one or more of the transporter as compared to a genetically unengineered cell (e.g., a parent cell). When the transporter is already present in an active form in a parent cell, the expression of the transporter may be further increased through genetic manipulation. When the transporter is not present in an active form in a wild-type microorganism, the gene encoding the transporter may be introduced into the parent cell to express or over-express the same through genetic manipulation. The genetically unengineered cell refers to a wild-type or a parent cell from which the recombinant microorganism is derived.

The expression or the over-expression of the transporter may be achieved by various methods known to one of ordinary skill in the art. For example, a copy number of the gene may be increased, or a regulatory material such as an inducer or a repressor may be used to increase the expression. The increased copy number may be due to introduction or amplification of the gene. The increased copy number may be achieved by introducing a vector, or an expression cassette, including the transporter gene operably linked a regulatory element into a host cell.

Alternatively, the increased activity of the transporter may be due to changes to the expression regulatory sequence of the gene. The regulatory sequence may be a promoter sequence for gene expression or a transcription terminator sequence. Also, the regulatory sequence may be a sequence that encodes a motif that may affect the gene expression. The motif may be, for example, a secondary structure-stabilizing motif, an RNA destabilizing motif, a splice-activating motif, a polyadenylation motif, an adenine-rich sequence, or an endonuclease recognition region.

Increased hydroxycarboxylic acid may be produced in a recombinant microorganism that produces an increased amount of the iron-regulated ABC transporter as compared to the microorganism that does not express or over-express the polynucleotide encoding the iron-regulated ABC transporter when cultured under the same condition.

The recombinant microorganism may be selected from bacteria, yeast, fungi, or the like, provided the microorganism has (natively or by way of genetic engineering) a biosynthetic pathway for the production of a hydroxycarboxylic acid. The recombinant microorganism may be any one selected from the group consisting of *Escherichia* sp., rumen bacteria sp., *Brevibacterium* sp., and *Corynebacterium* sp., but it is not limited thereto. In one embodiment, the cell may be *Corynebacterium* sp., such as *Corynebacterium glutamicum* or *Corynebacterium thermoaminogenes.* In other embodiments, the microorganism may be *E.coli, Brevibacterium flavum,* or *Brevibacterium lactofermentum.*

In an embodiment of the present invention, the hydroxycarboxylic acid may have a chemical structure represented by Formula 1 below:

[Formula 1] HO-R'-COOH

In Formula 1, R¹ may be a straight or branched C₁-C₂₀ alkyl group, optionally substituted with a phenyl group, and in some embodiments, R¹ may be a straight unsubstituted C₁-C₉ alkyl group. The hydroxycarboxylic acid may be 3-hydroxypropionic acid, 4-hydroxybutyric acid, 3-hydroxy-2-methylpropionic acid, 3-hydroxybutanoic acid, 3-hydroxy-2-methylbutanoic acid, 3-hydroxy-2-methylpentanoic acid, 3-hydroxy-3-methylbutanoic acid, 2,3-dimethyl-3-hydroxybutanoic acid, 3-hydroxy-3-phenylpropionic acid, or a combination thereof.

The recombinant microorganism may be a type (species) that naturally produces hydroxycarboxylic acid, or may be a microorganism that has been genetically engineered to produce the hydroxycarboxylic acid. For example, when the hydroxycarboxylic acid is 4-hydroxybutyric acid, 4-hydroxybutyric acid may be produced by biological transformation of succinic acid or alpha-ketoglutarate. In the case of using succinic acid as a starting material in biosynthesis of 4-hydroxybutyric acid, 4-hydroxybutyric acid may be synthesized by transforming succinic acid into succinyl-CoA, succinyl-CoA into succinic semialdehyde, and succinic semialdehyde into 4-hydroxybutyric acid. Alternatively, in the case of using a-ketoglutarate as a starting material in biosynthesis of 4-hydroxybutyric acid, 4-hydroxybutyric acid may be synthesized by transforming a-ketoglutarate into succinic semialdehyde, and succinic semialdehyde into 4-hydroxybutyric acid.

The recombinant microorganism may include at least one of an exogenous gene encoding an enzyme that catalyzes conversion of succinic acid to succinyl-CoA, an exogenous gene encoding an enzyme that catalyzes conversion of succinyl-CoA to succinic semialdehyde, and an exogenous gene encoding an enzyme that catalyzes conversion of succinic semialdehyde to 4-hydroxybutyric acid.

The enzyme catalyzing conversion of succinic acid to succinyl-CoA may be categorized as EC.2.8.3.- or EC.2.8.3.8. The enzyme may be derived from *Clostridium kluyveri, Corynebacterium kroppenstedtii, Corynebacterium glutamicum, Pseudomonas protegens,* or the like. The enzyme may include an amino acid sequence having a sequence identity of 65% or more, for example, 70% or more, 80% or more, 85% or more, 90% or more, 9% or more, 92% or more, 93% or more, 94% or more, 95% or more, 96% or more, 97% or more, 98% or more, 99% or more, or 100% to SEQ ID NO: 7, 9, or 11. The enzyme having an amino acid sequence of SEQ ID NO: 7 may be succinyl-CoA:coenzyme A transferase (Cat1) derived from *Clostridium kluyveri.* The enzymes each having an amino acid sequence of SEQ ID NOS: 9 and 11 may be acetyl-CoA hydrolase (ActA or ActB) derived from *Corynebacterium glutamicum,* and these enzymes may include an amino acid sequence of GenBank No: AAA92346.1 (J. Bacteriol. 178:871-880(1996)), GenBank NO: ACR17185.1 (J. Biotechnol. 136 (1-2), 22-30 (2008)), NCBI YP_007997424.1, or the like. The exogenous gene encoding the enzyme that catalyzes conversion of succinic acid to succinyl-CoA may include a nucleotide sequence having a sequence identity of 95% or more to SEQ ID NO: 8, 10, and 12, respectively. The recombinant microorganism may include two or more exogenous genes encoding the enzyme that catalyzes conversion of succinic acid to succinyl-CoA.

The enzyme catalyzing conversion of succinyl-CoA to succinic semialdehyde may be categorized as EC.1.2.1.16. The enzyme may be CoA-dependent succinate semialdehyde dehydrogenase (hereinafter, referred to as "CoA-dependent SSADH"). The enzyme may include an amino acid sequence having a sequence identity of 65% or more, for example, 70% or more, 80% or more, 85% or more, 90% or more, 91 % or more, 92% or more, 93% or more, 94% or more, 95% or more, 96% or more, 97% or more, 98% or more, 99% or more, or 100% to SEQ ID NO: 13. Alternatively, the enzyme may include an amino acid sequence of Accession No: P38947.1 (GI NO: 730847), NP_904963.1 (GI NO: 34540484), or the like. The CoA-dependent SSADH may be derived from *Clostridium kluyveri, Porphyromonas gingivalis,* or the like. The polynucleotide encoding the CoA-dependent SSADH may include a nucleotide sequence encoding an amino acid having a sequence identity of 95% or more to SEQ ID NO: 13. The polynucleotide may have a nucleotide sequence with a sequence identity of 95% or more to SEQ ID NO: 14 (sucD).

The enzyme catalyzing conversion of succinic semialdehyde to 4-hydroxybutyric acid may be categorized as EC.1.1.1.1. The enzyme may be an alcohol dehydrogenase or 4-hydroxybutyric acid dehydrogenase (4hbd). The enzyme may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to SEQ ID NO: 15 (Porphyromonas gingivalis). Alternatively, the enzyme may include an amino acid sequence of NCBI Reference Sequence: NP_904964.1, YP_726053.1, EDK35022.1, or Q94B07. The 4-hydroxybutyric acid dehydrogenase may be derived from *Porphyromonas gingivalis, Ralstonia eutropha, Clostridium kluyveri,* or *Arabidopsis thaliana.* The polynucleotide encoding the 4-hydroxybutyric acid dehydrogenase may include a nucleotide sequence encoding an amino acid sequence having a sequence identity of 95% or more to SEQ ID NO: 15. The polynucleotide may have a nucleotide sequence with a sequence identity of 95% or more to SEQ ID NO: 16 (4hbd). Also, a nucleotide sequence encoding the 4-hydroxybutyric acid dehydrogenase may be a nucleotide sequence of Gene ID No: 2552693, 113867564, 146348486, or 75249805.

The recombinant microorganism may include at least one of an exogenous gene encoding an enzyme that catalyzes conversion of a-ketoglutarate into succinic semialdehyde and an exogenous gene encoding an enzyme that catalyzes conversion of succinic semialdehyde into 4-hydroxybutyric acid.

The enzyme catalyzing conversion of a-ketoglutarate into succinic semialdehyde may be categorized as EC.4.1.1.71. The enzyme may be a-ketoglutarate decarboxylase, and the a-ketoglutarate decarboxylase may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to SEQ ID NO: 17 or 18. A gene encoding the a-ketoglutarate decarboxylase may be, for example, a nucleotide sequence encoding an amino acid sequence having a sequence identity of 95% or more to each of SEQ ID NO: 17 or 18, or a nucleotide sequence of SEQ ID NO: 19 or 20. The a-ketoglutarate decarboxylase may be derived from, for example, *Corynebacterium* sp., *Rhodococcus* sp., *Mycobacterium* sp., or *Escherichia* sp. A gene encoding the a-ketoglutarate decarboxylase may be, for example, *Corynebacterium glutamicum, Corynebacterium callunae, Corynebacterium efficiens, Corynebacterium ulcerans, Corynebacterium halotolerans, Corynebacterium pseudotuberculosis, Corynebacterium durum, Corynebacterium striatum, Rhodococcus pyridinivorans, Rhodococcus ruber, Mycobacterium abscessus, Mycobacterium smegmatis, Escherichia coli, Escherichia fergusonii,* or a combination thereof.

The exogenous gene encoding the enzyme that catalyzes conversion of succinic semialdehyde into 4-hydroxybutyric acid may be defined the same as described above.

The exogenous genes may be introduced into the recombinant microorganism in an expressible form, and accordingly, may be over-expressed compared to gene expression in an unengineered microorganism or a parent cell.

The hydroxycarboxylic acid produced from the recombinant microorganism may be converted into various useful materials. In the case of the 4-hydroxybutyric acid, the hydroxycarboxylic acid may be converted into gamma-butyrolactone (GBL), 1,4-butanediol, poly-4-hydroxybutyric acid, tetrahydrofuran (THF), or N-methylpyrrolidone (NMP).

In addition, the recombinant microorganism may have a genetic element that inhibits a pathway reducing the production of the hydroxycarboxylic acid. In this regard, the recombinant microorganism may have disrupted activity in converting succinic semialdehyde into succinic acid. That is, the recombinant microorganism may have a gene encoding succinic semialdehyde dehydrogenase (SSADH) removed or disrupted. The succinic semialdehyde dehydrogenase (SSADH) may catalyze a reaction that converts succinic semialdehyde into succinic acid. The SSADH may be an enzyme categorized as EC.1.2.1.16 or EC.1.2.1.39. The SSADH may be NAD+ or NADP+ dependent. The SSADH may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to SEQ ID NO: 21, SEQ ID NO: 22, and SEQ ID NO: 23, respectively. The gene that encodes the SSADH may include a polynucleotide that encodes an amino acid sequence having a sequence identity of 95% or more to SEQ ID NO: 21, SEQ ID NO: 22, and SEQ ID NO: 23, respectively, or the polynucleotide may have a nucleotide sequence a sequence identity of 95% or more to SEQ ID NO: 24, SEQ ID NO: 25, or SEQ ID NO: 26. The microorganism may have at least one of a polynucleotide having a nucleotide sequence of SEQ ID NO: 24, a polynucleotide having a nucleotide sequence of SEQ ID NO: 25, and a polynucleotide having a nucleotide sequence of SEQ ID NO: 26 disrupted or removed. The microorganism may have a polynucleotide having a nucleotide sequence of SEQ ID NO: 24 or a polynucleotide having a nucleotide sequence of SEQ ID NO: 25 disrupted or removed. The microorganism may have a polynucleotide having a nucleotide sequence of SEQ ID NO: 24 and a polynucleotide having a nucleotide sequence of SEQ ID NO: 25 disrupted or removed. The microorganism may have a polynucleotide having a nucleotide sequence of SEQ ID NO: 24, a polynucleotide having a nucleotide sequence of SEQ ID NO: 25, and a polynucleotide having a nucleotide sequence of SEQ ID NO: 26 disrupted or removed.

The recombinant microorganism may have a pathway for converting pyruvate into lactate disrupted. That is, the recombinant microorganism may have a gene encoding an enzyme that catalyzes the conversion of pyruvate into lactate disrupted. The enzyme catalyzing the conversion of pyruvate into lactate may be lactate dehydrogenase (LDH) categorized as EC.1.1.1.27 or EC.1.1.1.28. The LDH may be NAD(P)H dependent, and in addition, may function in D-lactate and/or L-lactate. The LDH may include an amino acid sequence having a sequence identity of 65% or more, for example, 70% or more, 80% or more, 85% or more, 90% or more, 91% or more, 92% or more, 93% or more, 94% or more, 95% or more, 96% or more, 97% or more, 98% or more, 99% or more, or 100% to an amino acid sequence of SEQ ID NO: 27. The recombinant microorganism may have a gene encoding the LDH that is disrupted or removed. Here, the gene encoding the LDH may have a nucleotide sequence encoding an amino acid sequence having a sequence identity of 95% or more to the SEQ ID NO: 27, or a nucleotide sequence with a sequence identity of 95% or more to SEQ ID NO: 89 (Ncgl2810).

The recombinant microorganism may be, for example, a recombinant microorganism that produces 4-hydroxybutyric acid, including at least one of a polynucleotide encoding the iron-regulated ABC transporter, a polynucleotide encoding the succinyl-CoA:coenzyme A transferase, and a polynucleotide encoding the CoA-dependent SSADH. In some embodiments, the recombinant microorganism may further include a polynucleotide encoding the 4hbd. The recombinant microorganism may have at least one of genes encoding the LDH and the SSADH that are disrupted.

Another aspect of the present invention provides a method of producing hydroxycarboxylic acid comprising culturing the microorganism that produces the hydroxycarboxylic acid as described above; and recovering the hydroxycarboxylic acid from the culture.

The culturing may be performed in a suitable medium under suitable culturing conditions known in the art. One of ordinary skill in the art may suitably change a culture medium and culturing conditions according to the microorganism selected. A culturing method may be batch culturing, continuous culturing, fed-batch culturing, or a combination thereof.

The culture medium may include various carbon sources, nitrogen sources, and trace elements.

The carbon source may be, for example, carbohydrate such as glucose, sucrose, lactose, fructose, maltose, starch, or cellulose; fat such as soybean oil, sunflower oil, castor oil, or coconut oil; fatty acid such as palmitic acid, stearic acid, linoleic acid; alcohol such as glycerol or ethanol; organic acid such as acetic acid, or a combination thereof. The culturing may be performed by having glucose as the carbon source. The nitrogen source may be an organic nitrogen source such as peptone, yeast extract, beef stock, malt extract, corn steep liquor (CSL), or soybean flour, or an inorganic nitrogen source such as urea, ammonium sulfate, ammonium chloride, ammonium phosphate, ammonium carbonate, and ammonium nitrate, or a combination thereof. The culture medium is a supply source of phosphorus and may include, for example, potassium dihydrogen phosphate, dipotassium phosphate, and corresponding sodium-containing salt thereof, and a metal salt such as magnesium sulfate or iron sulfate. Also, amino acid, vitamin, a suitable precursor, or the like may be included in the culture medium. The culture medium or individual component may be added to a culture medium solution in a batch, fed-batch, or continuous manner.

Also, pH of the culture medium solution may not be adjusted or may be adjusted by adding a compound such as ammonium hydroxide, potassium hydroxide, ammonia, phosphoric acid, and sulfuric acid to the culture medium solution by using a suitable method during the culturing process. Also, an antifoaming agent such as fatty acid polyglycol ester may be used during the culturing process to inhibit the generation of bubbles.

The culturing process may be performed in a suitable oxygen condition for the production of the hydroxycarboxylic acid. The cells may be cultured in an aerobic condition for the cell proliferation. Afterwards, in order to produce the hydroxycarboxylic acid, the cells may be cultured in a microaerobic or anaerobic condition. The term "anaerobic condition" as used herein refers to a condition without oxygen. The term "microaerobic condition" as used herein in terms of cell culture or growth conditions refers to a condition in which a concentration of dissolved oxygen in a medium is maintained greater than 0% and below about 10% of the concentration of dissolved oxygen in an oxygen-saturated liquid medium of the same type. The microaerobic condition may also refer to a condition in which cells are grown or present in a resting state within a sealed chamber held in an atmosphere with an oxygen content of less than 1%. The concentration of oxygen may be maintained by, for example, sparging a culture with a N₂/CO₂ mixture or other suitable non-oxygen gas. Oxygen conditions may also include a concentration of dissolved oxygen is maintained in a range of about 0% to about 10%, for example, about 0% to about 8%, about 0% to about 6%, about 0% to about 4%, about 0% to about 2%.

The hydroxycarboxylic acid produced by the recombinant microorganism may be secreted from the cell and then recovered from the culture medium, and may additionally be separated from the culture medium. The separation of the hydroxycarboxylic acid from the culture medium solution may be performed by a separation and purification method known in the art. The recovery may be performed by centrifugation, chromatography, extraction, filtration, precipitation, or a combination thereof.

The hydroxycarboxylic acid produced by the method described above may be chemically converted to a compound that is structurally related thereto. For example, 4-hydroxybutyric acid may be reacted in the presence of a strong acid at a temperature of about 100°C to about 200 °C and then distilled to obtain gamma-butyrolactone (GBL). The obtained GBL may be converted into N-methylpyrrolidone (NMP) by amination using an aminating agent, for example, methylamine. Also, the GBL may be selectively converted into tetrahydrofuran (THF), 1,4-butanediol, or butanol by hydrogenation reaction using a metal-containing catalyst such as a catalyst including copper (Cu), ruthenium (Ru), and palladium (Pd). Also, the 4-hydroxybutyric acid may be biologically converted into poly-4-hydroxybutyric acid. The biological conversion may be performed by polyhydroxyalkanoate synthase, 4HB-coenzyme A:coenzyme A transferase, or a combination thereof.

According to an aspect of the present invention, provided is a method of improving the hydroxycarboxylic acid production of a microorganism, the method comprising introducing an exogenous gene encoding an iron-regulated ABC transporter into a hydroxycarboxylic acid producing microorganism, thereby improving hydroxycarboxylic acid production in the microorganism.

In some embodiments, the microorganism having increased activity of the iron-regulated ABC transporters may be used for the production of the hydroxycarboxylic acid.

In some embodiments, the hydroxycarboxylic acid may be produced in an effective manner by using the method described above.

Hereinafter, the present invention is described in greater detail with reference to embodiments. However, the embodiments are for illustrative purposes only and do not limit the scope of the present invention.

### Example 1: Preparing a strain having a L-lactate dehydrogenase (Idh) gene removed therefrom

### (1) Manufacturing a replacement vector

L-lactate dehydrogenase (ldh) gene of *Corynebacterium glutamicum* (CGL) ATCC 13032 was inactivated by homologous recombination using a pK19 mobsacB (ATCC 87098) vector.

Two homologous regions for removing the ldh gene were obtained by PCR amplification using a genomic DNA of *Corynebacterium glutamicum* ATCC 13032 as a template. The homologous regions were regions upstream and downstream of the ldh gene, which were obtained by PCR amplification using a primer set of ldhA_5'_HindIII (SEQ ID NO: 28) and ldhA_up_3'_XhoI (SEQ ID NO: 29) and a primer set of ldhA_dn_5'_XhoI (SEQ ID NO: 30) and ldhA-3'_EcoRI (SEQ ID NO: 31). The PCR amplification was performed by repeating the processes of denaturing at a temperature of 95 °C for 30 seconds, annealing at a temperature of 55 °C for 30 seconds, and elongation at a temperature of 72 °C for 30 seconds. All PCR amplifications were performed in the same manner.

The amplification product obtained therefrom was cloned at HindIII and EcoRI restriction sites of the pK19 mobsacB vector to manufacture a pK19_Δldh vector.

### (2) Preparing a CGL (Δldh) strain

*Corynebacterium glutamicum* ATCC 13032 was electroporated to introduce the pK19_Δldh vector therein. The introduced strain was smeared on 25 µg/ml of a kanamycin-containing LBHIS culture medium and then cultured at a temperature of 30°C. LBHIS culture medium includes 18.5 g/L of brain-heart infusion broth, 0.5 M sorbitol, 5 g/L of bacto-tryptone, 2.5 g/L of bacto-yeast extract, 5 g/L of NaCl, and 18 g/L of bacto-agar. Hereinafter, the composition of the LBHIS culture medium is as described above. The colony formed therefrom was smeared on a LB-sucrose culture medium and then cultured at a temperature of 30 °C, followed by selection of colonies in which double cross-over occurred. Genomic DNA was separated from the selected colonies, and a primer set of ldhA up (SEQ ID NO: 32) and ldhA down (SEQ ID NO: 33) was used to confirm deletion of the ldh gene through PCR. As a result, a CGL (Δldh) strain was obtained.

### Example 2: Deletion of CoA-dependent succinic semialdehyde dehydrogenase gene

### (1) Manufacturing a replacement vector

A CoA-dependent succinic semialdehyde dehydrogenase (ssadh) gene of *Corynebacterium glutamicum* ATCC 13032, which encodes an enzyme catalyzing the conversion of succinic semialdehyde into succinic acid, was inactivated by homologous recombination using a pK19 mobsacB vector.

Homologous regions for removing NCgl0049 (SEQ ID NO: 24), NCgl0463 (SEQ ID NO: 25), and NCgl2619 (SEQ ID NO: 26), which are CoA-dependent ssadh genes, were obtained by PCR amplification using genomic DNA of *Corynebacterium glutamicum* ATCC 13032 as a template.

Two homologous regions for removing the NCgl0049 gene (regions upstream and downstream of the gene) were obtained by PCR amplification using a primer set of 0049-1 for (SEQ ID NO: 34) and 0049-1 rev (SEQ ID NO: 35), and a primer set of 0049-2 for (SEQ ID NO: 36) and 0049-2 rev (SEQ ID NO: 37). The amplification product obtained therefrom was cloned at HindIII and Pstl restriction sites of the pK19 mobsacB vector to manufacture a pK19_Δn0049 vector.

Two homologous regions for removing the NCgl0463 gene (regions upstream and downstream of the gene) were obtained by PCR amplification using a primer set of 0463-1 for (SEQ ID NO: 38) and 0463-1 rev (SEQ ID NO: 39) and a primer set of 0463-2 for (SEQ ID NO: 40) and 0463-2 rev (SEQ ID NO: 41). The amplification product obtained therefrom was cloned at HindIII and Pstl restriction sites of the pK19 mobsacB vector to manufacture a pK19_Δn0463 vector.

Two homologous regions for removing the NCgl2619 gene (regions upstream and downstream of the gene) were obtained by PCR amplification using a primer set of 2619-1 for (SEQ ID NO: 42) and 2619-1 rev (SEQ ID NO: 43) and a primer set of 2619-2 for (SEQ ID NO: 44) and 2619-2 rev (SEQ ID NO: 45). The amplification product obtained therefrom was cloned at HindIII and Pstl restriction sites of the pK19 mobsacB vector to manufacture a pK19_Δn2619 vector.

### (2) Preparing a CGL (Δldh ΔgD³) strain

pK19_Δn0049, pK19_Δn0463, and pK19_Δn2619 vectors were sequentially introduced into the CGL (Δldh) strain prepared in Example 1 by electroporation. The introduced strain was smeared on 25 µg/ml of kanamycin-containing LBHIS culture medium and then cultured at a temperature of 30°C. The colony formed therefrom was smeared on a LB-sucrose culture medium and then cultured at a temperature of 30°C, followed by selection of the colonies in which double-crossing occurred. Genomic DNA was separated from the selected colonies, and a primer set of 0049for (SEQ ID NO: 46) and 0049rev (SEQ ID NO: 47), a primer set of 0463for (SEQ ID NO: 48) and 0463rev (SEQ ID NO: 49), or a primer set of 2619for (SEQ ID NO: 50) and 2619rev (SEQ ID NO: 51) was used to confirm the deletion of NCgl0049, NCgl0463 and NCgl2619 by using PCR. As a result, a CGL (Δldh ΔgD³) strain was obtained. Table 1 below shows strain names and gene types prepared in the present invention, wherein D003 is CGL (Δldh, ΔgD³, G3G).

**[Table 1]**

| Strain name | Gene type |
|---|---|
| CGL (Δldh ΔgD³) | C. glutamicum ATCC 13032 (Δldh ΔNCgl0049 ΔNCgl0463 ΔNCgl2619) |
| D003 | C.glutamicum ATCC13032 (Δldh, ΔNCgl0049, ΔNCgl0463,ΔNCgl2619, Δ gapA::cat1,sucD,4hbD) |
| D003:pEX1-1502 | C.glutamicum ATCC13032 (Δldh, ΔNCgl0049, ΔNCgl0463, ΔNCgl2619, gapA::cat1, sucD, 4hbD pEX1-1502) |
| D003:pEX1-1503 | C.glutamicum ATCC13032 (Δldh, ΔNCgl0049, ΔNCgl0463, ΔNCgl2619, gapA::cat1, sucD, 4hbD pEX1-1503) |

### Example 3: Introduction of cat1, sucD, and 4hbD genes

### (1) Manufacturing a pGST1 vector

To express cat1, sucD, and 4hbD genes from a vector, a cat1, sucD, and 4hbD gene expression vector, pG3G, was manufactured based on a pGST1 vector. The pGST1 vector was manufactured according to the method described below.

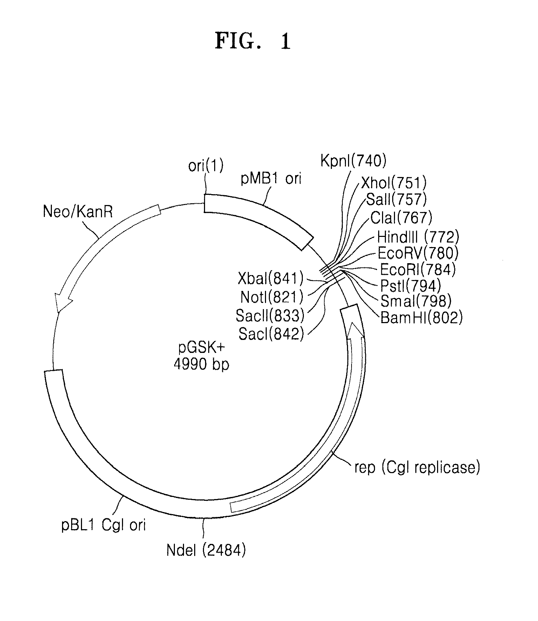

Phusion High-Fidelity DNA Polymerase (cat.# M0530, a product of New England Biolabs) was used to obtain four PCR products. PCR (Cgl replication origin including Cgl replicase gene) was performed by using (i) a *C.glutamicum* promoter screening vector, pET2 (GenBank accession number: AJ885178.1, 7513 bp), as a template and a primer sequence of MD-616 (SEQ ID NO: 52) and MD-618 (SEQ ID NO: 53); (ii) by using the *C.glutamicum* promoter screening vector, pET2, as a template and a primer sequence of MD-615 (SEQ ID NO: 54) and MD-617 (SEQ ID NO: 55) (E.coli replication origin); (iii) by using a mammalian fluorescence protein expression vector, pEGFP-C1 (a product of Clontech), as a template and a primer sequence of MD-619 (SEQ ID NO: 56) and MD-620 (SEQ ID NO: 57) (Kanamycin resistant neo gene (Nptll)); and (iv) by using an *E.coli* cloning vector, pBluescriptII SK+, as a template and a primer sequence of LacZa-NR (SEQ ID NO: 58) and a primer sequence of MD-404 (SEQ ID NO: 59) (Multiple cloning site: MCS). All four PCR products, i.e., 3010 bp, 854 bp, 809 bp, and 385 bp, were ligated to a linearized pUC19 vector available from In-Fusion EcoDry PCR Cloning Kit (cat.# 639690, available from Clontech), to obtain a circular plasmid. It was confirmed that the 4 genes were ligated to the linearized pUC19 vector. The vector was named pGSK+ (SEQ ID NO: 90). FIG. 1 is a cleavage map of the pGSK+ vector.

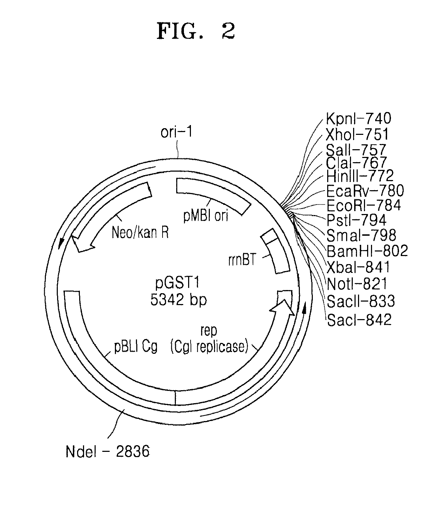

To manufacture a *C.glutamicum* shuttle vector including a transcription terminator and a 3' untranslated region (UTR) of the pSGK+ vector, a 3' UTR of *C.glutamicum* gltA (NCgl0795) and a rho-independent terminator of rrnB of *E.coli* rrnB were inserted into the pGSK+ vector. PCR was performed by using a *C.glutamicum* (ATCC13032) genomic DNA as a template and using a primer sequence of MD-627 (SEQ ID NO: 60) and MD-628 (SEQ ID NO: 61) to obtain a 108 bp PCR fragment of gltA 3' UTR. Genomic DNA *of E.coli* (MG1655) was used as a template and a primer sequence of MD-629(SEQ ID NO: 62) and MD-630(SEQ ID NO: 63) was used to obtain a 292bp PCR product of rrnB transcription terminator. Two amplified fragments were inserted into pGSK+ cleaved by SacI using In-Fusion EcoDry PCR Cloning Kit (*cat*.# *639690,* a product of Clontech). The cloned vector was introduced into One Shot TOP10 Chemically Competent Cell (cat.# C4040-06, a product of Invitrogen) and then cultured in 25 ug/ml of kanamycin-containing LB culture medium, followed by selection of growth colonies therefrom. A vector was recovered from the selected colonies for whole sequence analysis. The vector was named pGST1 (SEQ ID NO: 64). FIG. 2 is a cleavage map of the pGST1 vector. In FIG. 2, "rrnBT" denotes a gltA 3'-UTR and a transcription terminator of rrn.

### (2) Manufacturing a pG3G vector

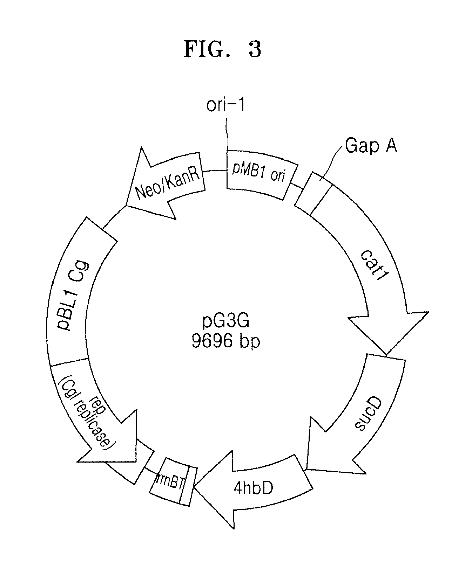

As a template for PCR amplification of the three types of genes described above, a gene cassette (SEQ ID NO: 65) sequentially including gapA promoter, and cat1, sucD, 4hbD, and cat2 genes was synthesized. In the cassette, the gapA promoter, cat1, sucD, and 4hbD gene sequences were obtained by PCR amplification using a primer set of 3G up (SEQ ID NO: 66) and 3G down (SEQ ID NO: 67). The amplification product obtained therefrom was cloned at Pstl and Xbal restriction sites of pGST1 vector using an In-fusion HD cloning kit (#PT5162-1, a product of Clontech) to manufacture an expression vector pG3G. FIG. 3 is a cleavage map of the pG3G vector. The gapA promoter was a promoter of gapA gene (NCgl1526) derived from *Corynebacterium glutamicum.*

### (3) Manufacturing a pK19 gapA::3G vector

To insert the three types of genes into a genome, a vector, pK19 gapA::3G, for inserting cat1, sucD and 4hbD genes was manufactured based on pK19 mobsacB.

Two homologous regions for homologous recombination were obtained by PCR amplification using a genomic DNA of *Corynebacterium glutamicum* ATCC 13032 as a template, and using each of a primer set of ncgl0049_up F (SEQ ID NO: 68) and ncgl0049_up R (SEQ ID NO: 69), and a primer set of ncgl0049_down F (SEQ ID NO: 70) and ncgl0049_down R (SEQ ID NO: 71).

A fragment including gapA promoter and the cat1, sucD, and 4hbD genes was obtained by PCR amplification using a pG3G vector as a template and using a primer set of pG3G F (SEQ ID NO: 72) and pG3G R (SEQ ID NO: 73).

In the HindIII and EcoRI restriction sites of the pK19 mobsacB vector, the amplified product was cloned to locate the fragment including the gapA promoter and cat1, sucD, and 4hbD genes between the two homologous regions by using an In-fusion HD cloning kit (#PT5162-1, a product of Clontech), to thereby manufacture a pK19 gapA::3G vector.

### (4) Preparing a D003 strain

The pK19 gapA::3G vector was introduced into the CGL (Δldh ΔgD³) strain prepared in Example 1 by electroporation. The introduced strain was smeared on 25 µg/ml of a kanamycin-containing LBHIS culture medium and then cultured at a temperature of 30°C. The colony was smeared on a LB-sucrose culture medium and then cultured at a temperature of 30°C, followed by selection of colonies in which double cross-over occurred. A genomic DNA was separated from the selected colonies, and introduction of the cat1, sucD and 4hbD genes was confirmed by PCR amplification using a primer set of cat1-seq1 F (SEQ ID NO: 74) and cat1-seq2R (SEQ ID NO: 75), a primer set of sucD-seq1F (SEQ ID NO: 76) and sucD-seq2R (SEQ ID NO: 77), or a primer set of 4hbD-seq1 F (SEQ ID NO: 78) and 4hbD-seq2R (SEQ ID NO: 79), respectively. As a result, a D003 strain, namely, CGL (Δldh, ΔgD³, G3G) was obtained.

### Example 4: Introduction of sufD, sufB, and Ncgl0776

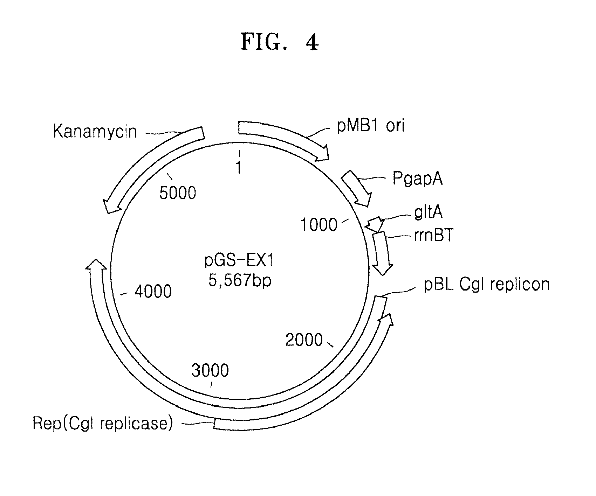

As a plasmid vector for expressing sufD, sufB, and NCgl0776 genes, a pGS-Ex1 (SEQ ID NO: 80) vector including a gapA promoter was manufactured as follows. A *Corynebacterium glutamicum-derived* promoter of a gapA gene (NCgl1526) was obtained by PCR amplification using a genomic DNA of *Corynebacterium glutamicum* ATCC 13032 as a template, and a primer set of MD-625 (SEQ ID NO: 81) and MD-626 (SEQ ID NO: 82). The amplified product was cloned such that two DNA fragments of 270 bp each obtained from the PCR reaction were located at Kpnl and Xhol restriction sites of the pGST1 vector manufactured in Example 3 to provide the pGS-Ex1 vector. Cloning was performed using an In-fusion HD cloning kit (#PT5162-1, a product of Clontech). FIG. 4 is a cleavage map of the pGS-EX1 vector.

### (1) Manufacturing pEX1-1502, pEX1-1503, and pEX1-0776 vectors

To express NCgI 1502 (sufD), NCgl 1503 (sufB), and Ncgl 0776 genes from a vector, NCgl 1502 (sufD), NCgl 1503 (sufB), and Ncgl 0776 gene expression vectors of pEX1-1502, pEX1-1503, and pEX1-0776 were each manufactured based on the pGS-Ex1 vector (SEQ ID NO: 80).

For PCR amplification of the NCgl 1502, NCgl 1503, and NCgl 0776 genes, genomic DNA of *Corynebacterium glutamicum* ATCC 13032 was used as a template with a primer sets as follows:
NCgl 1502: 1502-up (SEQ ID NO: 83) and 1502-dn (SEQ ID NO: 84)
NCgl 1503: 1503-up (SEQ ID NO: 85) and 1503-dn (SEQ ID NO: 86)
NCgl 0776: 0776-up (SEQ ID NO: 87) and 0776-dn (SEQ ID NO: 88).

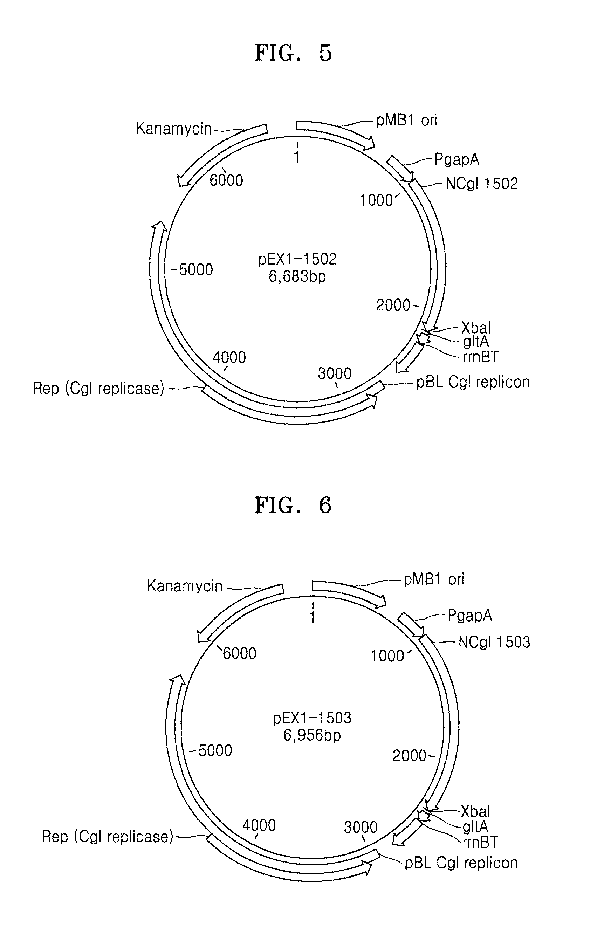

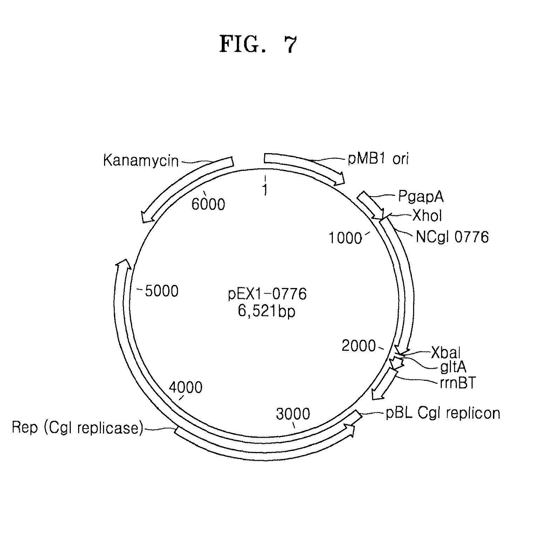

The amplification products obtained therefrom were cloned at Xhol and Xbal restriction sites of a pGS-Ex1 vector using the In-fusion HD cloning kit (#PT5162-1, a product of Clontech) to manufacture expression vectors of pEX1-1502, pEX1-1503, and pEX1-0776 for NCgl 1502, NCgl 1503, and NCgl 0776 genes, respectively. FIG. 5 is a cleavage map of the pEX1-1502 vector, FIG. 6 is a cleavage map of the pEX1-1503 vector, and FIG. 7 is a cleavage map of the pEX1-0776 vector.

pEX1-1502, pEX1-1503, and pEX1-0776 vectors were introduced into the D003 strain manufactured above by electroporation. Thereafter, the product was cultured in a LB culture medium, frozen, and stored.

### Example 5: Evaluation of productivity of 4-hydroxybutyate

The control group strain D0003 (CGL (Δldh, ΔgD³, G3G) manufactured in Example 3) and the D003(+pEX1-1502), D003(+pEX1-1503), and D003(+pEX1-0776) strains manufactured in Example 4 were seed-cultured in 25 mL of brain heart infusion (BHI) culture medium (a product of Becton, Dickinson and Company) contained in a 125 mL flask at a temperature of 30°C, stirring at a rate of 230 rpm for 8 hours. The obtained culture was inoculated in 37.5 mL of a CgXII culture medium (Keilhauer et al. J. Bacteriol. 1993, 175:5595) contained in a 125 mL flask at a concentration of 1/50, and then cultured at a temperature of 30°C, stirring at a rate of 230rpm for 16 hours. The flask was configured with a vented cap to allow an aerobic culture. Thereafter, cap and neck portions of the flask were sealed to create an anaerobic or microaerobic condition and the inoculated culture medium was cultured at a temperature of 30°C, stirring at a rate of 230 rpm for 24 hours. The final culture medium obtained therefrom was centrifuged, the supernatant obtained therefrom was filtered by using a syringe filter having a diameter of 0.45 um, and 4-hydroxybutyric acid (4HB) content was analyzed by using UPLC. The analysis was performed by using a conventional method as disclosed in J. Chromatogra. B, 885-886, 2012, 37-42. As an apparatus for the analysis, UPLC (a product of Waters) mounted with Aminex HPX-87H column (300 mm x 7.8 mm, a product of Bio-Rad) was used. As a mobile phase, 2.5 mM sulfuric acid was used at a flow rate of 0.6 ml/min, and the column was maintained at a temperature of 60°C. Measurement of a refractive index and detection of a diode array were performed by using a detector.

Table 2 shows measurement results of the amount of 4HB produced by culturing the D003 (CGL (Δldh, ΔgD³,G3G)), D003(pEX1-1502), D003(pEX1-1503), and D003(pEX1-0776).

**[Table 2]**

| **Strain** | **Amount of 4HB produced (mg/L)** |
|---|---|
| D003 | 1110 |
| D003 (pEX1-1502) | 1373 |
| D003 (pEX1-1503) | 1297 |
| D003 (pEX1-0776) | 1367 |

As shown in Table 2, three strains in which the iron-regulated ABC transporter was over-expressed had increased 4HB production compared to the control group (strain D003) by 24%, 17%, and 23%, respectively.

Also, the method of producing hydroxycarboxylic acid has high efficiency.

It should be understood that the exemplary embodiments described therein should be considered in a descriptive sense only and not for purposes of limitation. Descriptions of features or aspects within each embodiment should typically be considered as available for other similar features or aspects in other embodiments.

## Claims

1. A recombinant microorganism having an increased iron-regulated ABC transporter activity in comparison with a parent cell of the recombinant microorganism, and producing an increased amount of hydroxycarboxylic acid as compared to a parent cell of the recombinant microorganism.

2. The recombinant microorganism of claim 1, wherein the transporter is (a) SufB, (b) SufD, (c) ABC-type cobalamin/Fe³⁺-siderophore transport system, periplasmic component, or (d) a combination thereof.

3. The recombinant microorganism of claim 2, wherein
the SufB comprises an amino acid sequence having a sequence identity of 95% or more to SEQ ID NO: 1,
the SufD comprises an amino acid sequence having a sequence identity of 95% or more to SEQ ID NO: 3, and
the ABC-type cobalamin/Fe³⁺-siderophore transport system, periplasmic component comprises an amino acid sequence having a sequence identity of 95% or more to SEQ ID NO: 5.

4. The recombinant microorganism of any one of claims 1 to 3, wherein the recombinant microorganism has a genetic modification that increases the activity of the transporter as compared to an activity of the parent cell of the recombinant microorganism.

5. The recombinant microorganism of any one of claims 1 to 4, wherein the recombinant microorganism comprises an exogenous gene that encodes the transporter, wherein the exogenous gene preferably encodes an amino acid sequence having a sequence identity of 95% or more to SEQ ID NOS: 1, 3, and/or 5.

6. The recombinant microorganism of any one of claims 1 to 5, wherein the hydroxycarboxylic acid
(a) has a chemical structure represented by Formula 1 below:
[Formula 1] HO-R¹-COOH
wherein, R¹ is a straight or branched C₁-C₂₀ alkyl group, wherein, R¹ preferably is a straight unsubstituted C₁-C₉ alkyl group, or
(b) is 3-hydroxypropionic acid, 4-hydroxybutyric acid, 3-hydroxy-2-methylpropionic acid, 3-hydroxybutanoic acid, 3-hydroxy-2-methylbutanoic acid, 3-hydroxy-2-methylpentanoic acid, 3-hydroxy-3-methylbutanoic acid, 2,3-dimethyl-3-hydroxybutanoic acid, 3-hydroxy-3-phenylpropionic acid, or a combination thereof.

7. The recombinant microorganism of any one of claims 1 to 6, wherein the recombinant microorganism belong to *Escherichia* sp, Rumen bacteria sp, *Corynebacterium* sp, or *Brevibacterium* sp.

8. The recombinant microorganism of any one of claims 1 to 7, wherein the recombinant microorganism further comprises at least one of an exogenous gene encoding an enzyme that catalyzes conversion of succinic acid to succinyl-CoA, an exogenous gene encoding an enzyme that catalyzes conversion of succinyl-CoA to succinic semialdehyde (SSA), and an exogenous gene encoding an enzyme that catalyzes conversion of SSA to 4-hydroxybutyric acid.

9. The recombinant microorganism of claim 8, wherein:
- the enzyme catalyzing conversion of succinic acid to succinyl-CoA is categorized as EC.2.8.3.-,
- the enzyme catalyzing conversion of succinyl-CoA to SSA is categorized as EC.1.2.1.16,
- the enzyme catalyzing conversion of SSA to 4-hydroxybutyric acid is categorized as EC.1.1.1.1,-
- the enzyme catalyzing conversion of succinic acid to succinyl-CoA comprises an amino acid sequence having a sequence identity of 95% or more to SEQ ID NO: 7, 9, or 11,
- the enzyme catalyzing conversion of succinyl-CoA to SSA comprises an amino acid sequence having a sequence identity of 95% or more to SEQ ID NO: 13,the enzyme catalyzing conversion of SSA to 4-hydroxybutyric acid comprises an amino acid sequence having a sequence identity of 95% or more to SEQ ID NO: 15,
- the exogenous gene encoding the enzyme that catalyzes conversion of succinic acid to succinyl-CoA comprises a nucleotide sequence having a sequence identity of 95% or more to SEQ ID NO: 8, 10, or 12;
- the exogenous gene encoding the enzyme that catalyzes conversion of succinyl-CoA to SSA comprises a nucleotide sequence having a sequence identity of 95% or more to SEQ ID NO: 14; and/or
- the exogenous gene encoding the enzyme that catalyzes conversion of SSA to 4-hydroxybutyrat comprises a nucleotide sequence having a sequence identity of 95% or more to SEQ ID NO: 16.

10. The recombinant microorganism of any one of claims 1 to 9, wherein the activity of at least one an enzyme catalyzing conversion of pyruvate into lactate or an enzyme catalyzing conversion of SSA into succinic acid is reduced in the recombinant microorganism as compared to a parent cell of the recombinant microorganism.

11. The recombinant microorganism of claim 10, wherein at least one of a gene encoding an enzyme that catalyzes conversion of pyruvate into lactate and a gene encoding an enzyme that catalyzes conversion of SSA into succinic acid is disrupted or deleted in the recombinant microorganism.

12. The recombinant microorganism of claim 10 or 11, wherein:
- the enzyme catalyzing conversion of pyruvate into lactate is categorized as EC.1.1.1.27,
- the enzyme catalyzing conversion of SSA into succinic acid is categorized as EC.1.2.1.16 or EC.1.2.1.39,
- the enzyme catalyzing conversion of pyruvate into lactate comprises an amino acid sequence having a sequence identity of 95% or more to SEQ ID NO: 27,
- the enzyme catalyzing conversion of SSA into succinic acid comprises an amino acid sequence having a sequence identity of 95% or more to SEQ ID NO: 21, 22, or 23,
- the gene encoding the enzyme that catalyzes conversion of pyruvate into lactate comprises a nucleotide sequence having a sequence identity of 95% or more to SEQ ID NO: 89, and/or
- the gene encoding the enzyme that catalyzes conversion of SSA into succinic acid comprises a nucleotide sequence having a sequence identity of 95% or more to SEQ ID NO: 24, 25, or 26.

13. A method of producing hydroxycarboxylic acid, comprising:
culturing the recombinant microorganism of any one of claims 1 to 12 with a carbon source; and
recovering hydroxycarboxylic acid from the culture.

14. The method of claim 13, wherein the culturing is performed in a microaerobic condition.

15. A method of producing a recombinant microorganism with improved hydroxycarboxylic acid production, the method comprising introducing an exogenous gene encoding an iron-regulated ABC transporter into a hydroxycarboxylic acid producing microorganism to provide a recombinant microorganism with improved hydroxycarboxylic acid production.
